# EUROPEAN PATENT APPLICATION

(11) **EP 3 640 334 A1**
(43) Date of publication of application: **22.04.2020**
(21) Application number: 18817082.3
(22) Date of filing: 14.06.2018
(51) Int. Cl.: C12N 15/86, C12N 9/22, C12N 15/10, C12N 15/113, C12N 15/90, A61K 48/00

(54) **GENOME EDITING SYSTEM FOR REPEAT EXPANSION MUTATION**

(30) Priority: 15.06.2017 US 201762520098 P
(71) Applicant: Toolgen Incorporated, Seoul 08501 (KR); College of Medicine Pochon Cha University Industry-Academic Cooperation Foundation, Pocheon-si Gyeonggi-do 11160 (KR)
(72) Inventor: LEE, Jae Young, Seoul 06721 (KR); KIM, Seokjoong, Seoul 06622 (KR); BAE, Heesook, Bucheon-si Gyeonggi-do 14413 (KR); SONG, Jihwan, Seoul 06575 (KR); PARK, Hyun Jung, Anyang-si Gyeonggi-do 14035 (KR)
(74) Representative: dompatent von Kreisler Selting Werner - Partnerschaft von Patent- und Rechtsanwälten mbB
(86) International application number: PCT/KR2018/006731
(87) International publication number: WO 2018/230976

(57) **Abstract**

The present specification relates to an artificial manipulation or modification in an expression regulatory gene of an over-amplified repeated sequence. More specifically, a system for regulating the expression of an over-amplified repeated sequence including an artificially manipulated gene to reduce the expression of the over-amplified repeated sequence. The artificially manipulated gene includes one or more genes selected from the group consisting of an SPT4 gene, SPT5 gene, SUPT4H gene, and SUPT5H gene. The artificially manipulated gene includes an artificial mutation in a genomic sequence of the gene. The artificially manipulated gene includes a gene whose function is impaired or has reduced expression.

## Description

### [Technical Field]

The present specification relates to an artificial manipulation or modification in an expression regulatory gene of an over-amplified repeated sequence. More specifically, the present specification relates to a composition for gene manipulation to artificially manipulate an expression regulatory gene of an over-amplified repeated sequence.

### [Background Art]

Expression of a specific nucleotide sequence with a repeated sequence may cause a disease depending on the degree of amplification of the repeated sequence. In particular, when the degree of amplification of the repeated sequence is high, a serious genetic disease may be caused in a subject. For example, common characteristics of a disease such as Huntington's Disease (HD), Dentatorubropallidoluysian atrophy (DRPLA), Spinal and bulbar muscular atrophy (SBMA), Spinocerebellar ataxia (SCA), Fragile X syndrome (FXS), Fragile X-associated tremor/ataxia syndrome (FXTAS), Fuchs corneal dystrophy, Friedreich's ataxia (FRDA), myotonic dystrophy, and amyotrophic lateral sclerosis (C9orf72mutation) are genetic diseases caused by a specific gene including an over-amplified repeated sequence. Since the aforementioned diseases have life-threatening effects, there is a need for developing therapeutic agents.

Under the situation in which there is a need for the emergence of a therapeutic agent for a genetic disease caused by a specific gene including an over-amplified repeated sequence, the present inventors confirmed that the expression of a specific gene including an over-amplified repeated sequence could be regulated by regulating the expression of an SPT4 gene and/or SPT5 gene; or an SUPT4H gene/or SUPT5H gene, which are/is gene(s) involved in the regulation of expression of a specific gene including an over-amplified repeated sequence using target-specific gene scissors. Thus, the present inventors confirmed a genetic therapeutic agent for alleviating or treating a disease caused by an over-amplified repeated sequence and a method for treating the disease, thereby completing the present application.

### [Disclosure]

### [Technical Problem]

An object to be achieved by the content disclosed by the present application is to provide a system for regulating the expression of an over-amplified repeated sequence.

Another object to be achieved by the content disclosed by the present application is to provide a composition for gene manipulation to reduce the expression of an over-amplified repeated sequence.

Still another object to be achieved by the content disclosed by the present application is to provide a method for treating a repeat expansion disorder.

### [Technical Solution]

To achieve the above-described object, provided is a system for regulating the expression of an over-amplified repeated sequence. The system for regulating the expression of the over-amplified repeated sequence is a system for regulating the expression of an over-amplified repeated sequence including an artificially manipulated gene to reduce the expression of the over-amplified repeated sequence, in which the artificially manipulated gene includes one or more genes selected from the group consisting of an SPT4 gene, SPT5 gene, SUPT4H gene, and SUPT5H gene, the artificially manipulated gene includes an artificial mutation in a genomic sequence of the gene, and the artificially manipulated gene includes a gene whose function is impaired or has reduced expression.

To achieve the above-described another object, a composition for gene manipulation is provided by the present application. According to an aspect disclosed by the present application, provided is a composition for gene manipulation to reduce the expression of an over-amplified repeated sequence, the composition including any one of clustered regularly interspaced short palindromic repeats (CRISPR)-CRISPR associated protein(Cas) system, a zinc finger nuclease (ZFN), a transcription activator-like effector nuclease(TALEN), FokI, and an endonuclease, in which the gene is one or more genes selected from the group consisting of the SPT4 gene, SPT5 gene, SUPT4H gene, and SUPT5H gene. According to another aspect disclosed by the present application, the composition for gene manipulation is a composition for gene manipulation which includes guide nucleic acids for a target sequence of one or more genes selected from the group consisting of the SPT4 gene, SPT5 gene, SUPT4H gene, and SUPT5H gene, in which the target sequence complementarily binds to the guide nucleic acid; or the target sequence is complementary to a sequence complementarily binding with the guide nucleic acid.

To achieve the above-described still another object, provided is a method for treating a repeat expansion disorder by the present application. The treatment method is a method for treating a repeat expansion disorder, the method including: administering a composition including the aforementioned composition as an active ingredient to a subject to be treated.

### [Description of Drawings]

FIG. 1 illustrates the results of screening SpCas9 and CjCas9 gene scissors targeting the hSUPT4H1 gene in a human HEK293T cell line.
FIG. 2 illustrates the results of screening SpCas9 and CjCas9 gene scissors targeting the Supt4a gene in a mouse NIH3T3 cell line.

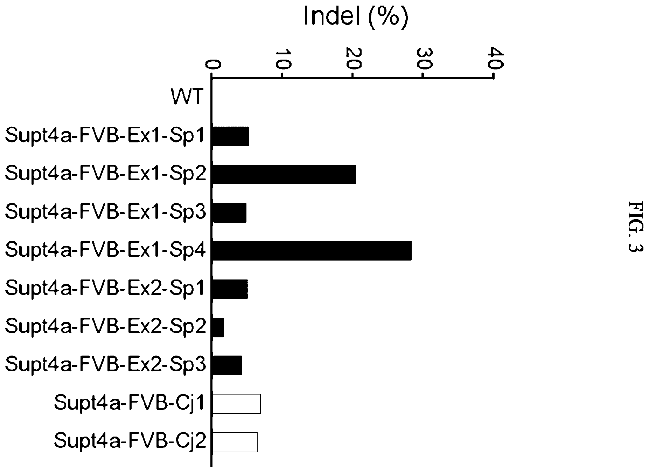

FIG. 3 illustrates the results of screening SpCas9 and CjCas9 gene scissors targeting the Supt4a gene in primary neural stem cells of mouse FVB/NJ embryos.

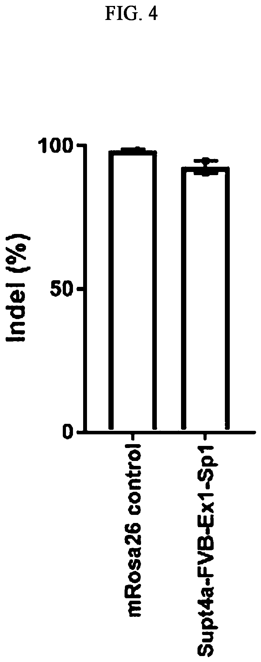

FIG. 4 illustrates the results of confirming an Indel (%) efficiency by gene manipulation or editing using SpCas9 gene scissors targeting the mRosa26 gene (control) or Supt4a gene in primary neural stem cells of a Huntington's disease mouse model (YAC128) in a mouse FVB/NJ.

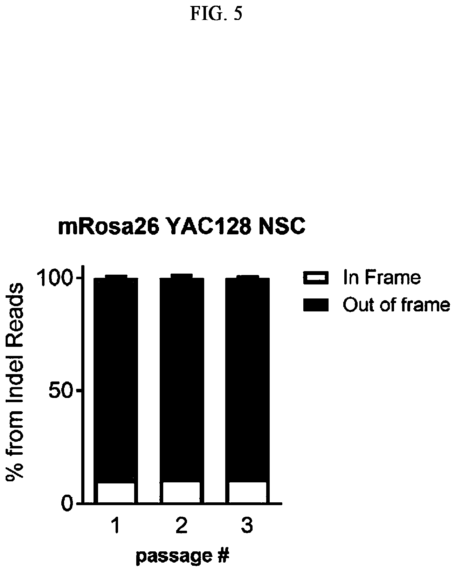

FIG. 5 illustrates the results of confirming whether an alteration in a reading frame of mRosa26 has a selective effect on survival during three passages after an Indel is caused using SpCas9 gene scissors targeting the mRosa26 gene (control) in primary neural stem cells of a Huntington's disease mouse model (YAC128) in a mouse FVB/NJ.

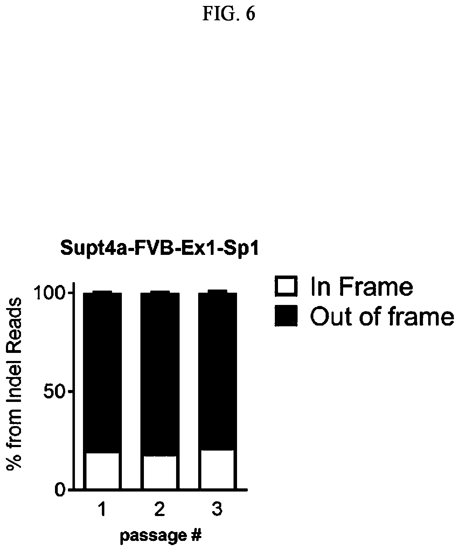

FIG. 6 illustrates the results of confirming whether a change in a reading frame of Supt4a has a selective effect on survival during three passages after an Indel is caused using SpCas9 gene scissors targeting the SpCas9 gene in primary neural stem cells of a Huntington's disease mouse model (YAC128) in a mouse FVB/NJ.

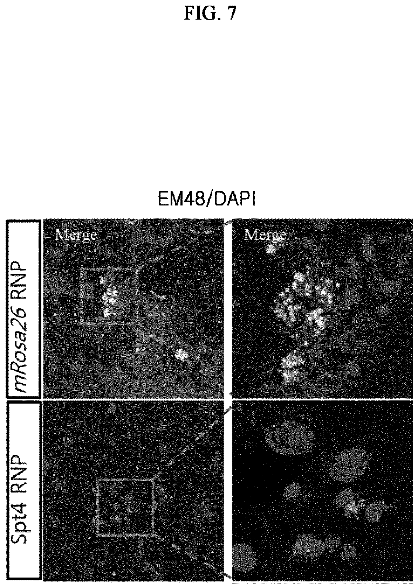

FIG. 7 illustrates the results of confirming the expression level of the Huntington's gene through anti-mutant HTT (EM48) detection as a result of a gene manipulation using SpCas9 gene scissors targeting the mRosa26 gene (control) or Supt4a gene in primary neural stem cells of a Huntington's disease mouse model (YAC128) in a mouse FVB/NJ.

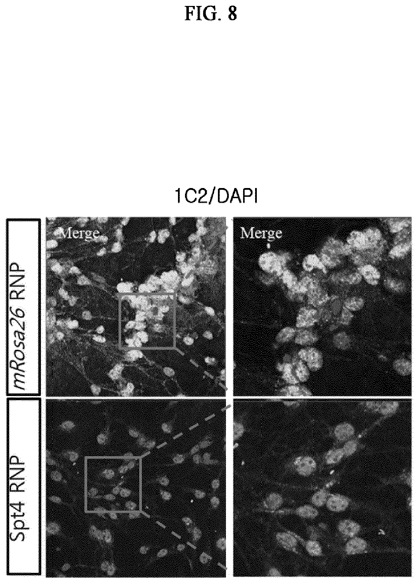

FIG. 8 illustrates the results of confirming the expression level of polyglutamine through 1C₂(anti-PolyQ) detection as a result of gene manipulation using SpCas9 gene scissors targeting the mRosa26 gene (control) or Supt4a gene in primary neural stem cells of a Huntington's disease mouse model (YAC128) in a mouse FVB/NJ.

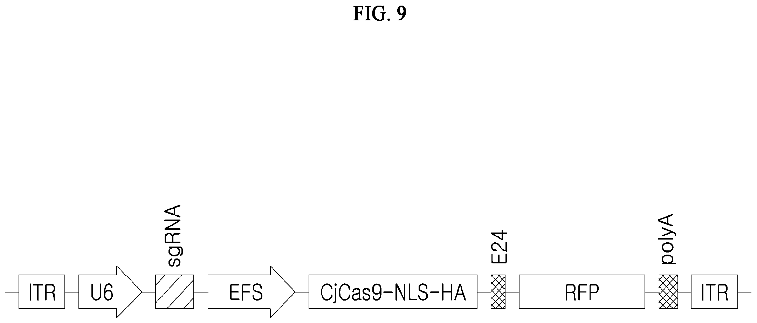

FIG. 9 illustrates the structure of a vector including guide nucleic acids and the CjCas9 gene, which are components of a composition for gene manipulation used for alleviating or treating a genetic disease caused by the expression of a specific gene including an over-amplified repeated sequence.

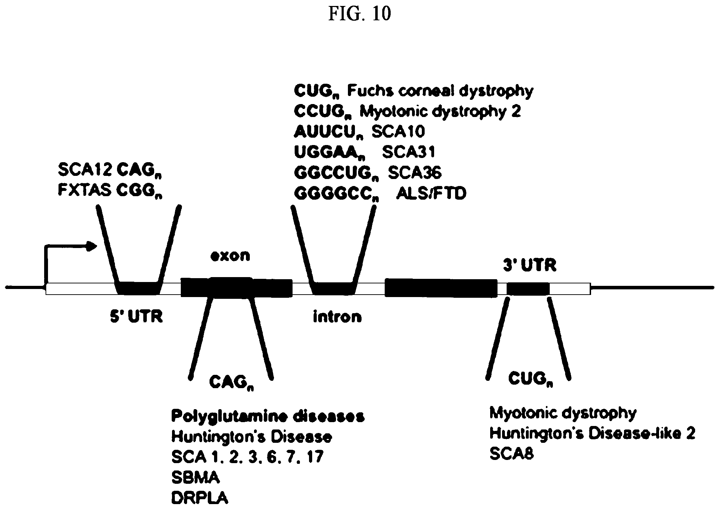

FIG. 10 is a schematic view of the types of repeated sequences and genetic disease caused by over-amplified repeated sequences.

### [Modes of the Invention]

Unless otherwise defined, all technical and scientific terms used herein have the same meaning as commonly understood by one of ordinary skill in the art to which the content disclosed herein belongs. Although methods and materials similar or equivalent to those described herein can be used in the practice or testing of the content disclosed herein, suitable methods and materials are described below. All publications, patent applications, patents, and other references mentioned herein are incorporated by reference in their entity. In addition, the materials, methods, and examples are illustrative only and not intended to be limiting.

Hereinafter, the regulation of expression of an over-amplified repeated sequence disclosed by the present application will be described in detail.

According to an aspect disclosed by the present specification, a system for regulating the expression of an over-amplified repeated sequence may be provided.

The system for regulating the expression of the over-amplified repeated sequence is a system for regulating an expression of an over-amplified repeated sequence including an artificially manipulated gene to reduce the expression of an over-amplified repeated sequence,
in which the artificially manipulated gene includes one or more genes selected from the group consisting of an SPT4 gene, SPT5 gene, SUPT4H gene, and SUPT5H gene,
in which the artificially manipulated gene includes an artificial mutation in a genome sequence of the gene,
in which the artificially manipulated gene includes the gene which is dysfunctional or is reduced on expression,
in which the reduction in expression of the gene is characterized in that an expression level of an expression product thereof is reduced or suppressed compared to that of a gene which is not artificially manipulated.

An embodiment disclosed in the present specification relates to a system for regulating the expression of an expression regulatory gene of an over-amplified repeated sequence.

The "an expression regulatory gene of an over-amplified repeated sequence" may be a gene for regulating the expression of a specific gene including an over-amplified repeated sequence.

The "over-amplified repeated sequence" may be a specific nucleotide sequence including duplication and over-duplication of the repeated sequence. The over-amplified repeated sequence may include a first repeated part including duplication of the repeated sequence; and a second repeated part including over-duplication of the repeated sequence.

The "duplication of the repeated sequence" may be a repeat degree in which the repeat degree of the repeated sequence appears in a gene of a normal subject. The duplication of the repeated sequence may be a repeat degree appearing even in a gene of a diseased subject.

The "over-duplication of the repeated sequence" may be a repeat degree which the repeat degree of the repeated sequence does not appear in a gene of a normal subject. The over-duplication of the repeated sequence may be a repeat degree appearing in a gene of a diseased subject.

The first repeated part may be a region included in a diseased subject and a normal subject. For example, a first repeated part in a HTT gene related to Huntington's disease may be a sequence corresponding to 35 to 40 repetitions of a CAG-nucleotide repeat unit. In another example, a first repeated part in an ATXN1 gene related to Spinocerebellar ataxia Type 1 (SCA1) may be a sequence corresponding to 35 to 40 repetitions of the CAG-nucleotide repeat unit. In still another example, a first repeated part in a DRPLA gene related to Dentatorubropallidoluysian atrophy (DRPLA) may be a sequence corresponding to 45 to 50 repetitions of the CAG-nucleotide repeat unit.

The second repeated part may be a region which is included in a gene of a diseased subject, but not included in a gene of a normal subject. For example, a second repeated part in the HTT gene related to Huntington's disease may be a corresponding sequence after 35 to 40 repetitions of the CAG-nucleotide repeat unit. In another example, a second repeated part in the ATXN1 gene related to Spinocerebellar ataxia Type 1 (SCA1) may be a corresponding sequence after 35 to 40 repetitions of the CAG-nucleotide repeat unit. In still another example, a second repeated part in the DRPLA gene related to Dentatorubropallidoluysian atrophy (DRPLA) may be a corresponding sequence after 45 to 50 repetitions of the CAG-nucleotide repeat unit.

The "regulation of the expression of an over-amplified repeated sequence" may be a reduction in expression of a specific gene including the over-amplified repeated sequence.

The regulation of the expression of a specific gene including the over-amplified repeated sequence may be a reduction in expression of the over-amplified repeated sequence in a specific gene including the over-amplified repeated sequence.

The regulation of the expression of a specific gene including the over-amplified repeated sequence may be a reduction in expression of a second repeated part in an over-amplified repeated sequence of a specific gene including the over-amplified repeated sequence. A reduction in expression of the second repeated part may include a reduction in transcription of the second repeated part.

The "expression regulatory gene of an over-amplified repeated sequence" refers to all genes which directly participate in or indirectly affect the functions related to the expression regulation of a specific gene including an over-amplified repeated sequence.

The expression regulatory gene of the over-amplified repeated sequence disclosed in the present specification includes all genes which directly participate in or indirectly affect the regulation of any one or more of the entire gene expression process including the transcription, post-transcriptional modification, translation, and post-translational modification of a specific gene including an over-amplified repeated sequence.

The expression regulatory gene of the over-amplified repeated sequence may be, for example, the SPT4 gene and/or SPT gene.

A product expressed from the SPT4 gene and/or SPT5 gene may be an SPT4, SPT5 or SPT4/5 protein.

The SPT4, SPT5 or SPT4/5 protein may function as a transcription regulator in the RNA polymerization process of an RNA polymerase.

The RNA polymerase may be RNA polymerase I, RNA polymerase II or RNA polymerase III. The RNA polymerase may be one that transcribes the sequence below the Class II promoter, as RNA polymerase II. The RNA polymerase may be one that transcribes a nucleotide sequence having a repeated sequence below the Class II promoter, as RNA polymerase II. The RNA polymerase may be one that transcribes a nucleotide sequence having an over-amplified repeated sequence below the Class II promoter, as RNA polymerase II. The RNA polymerase may be one that transcribes a first repeated part and/or a second repeated part of a nucleotide sequence having an over-amplified repeated sequence below the Class II promoter, as RNA polymerase II. A transcript by RNA polymerase II may be mRNA.

The SPT4, SPT5 or SPT4/5 protein may be involved in the processivity of the RNA polymerase II.

The processivity may be related to the degree to which a product is synthesized without being separated from a template DNA strand during the polymerization process of a polymerase. The polymerase may be an RNA polymerase. The RNA polymerase may be RNA polymerase II.

The processivity of the RNA polymerase II may be related to the degree to which an RNA product is synthesized without being separated from a template DNA strand during the polymerization process of RNA polymerase II. The high processivity of the RNA polymerase II may mean that in the synthesis of RNA from the transcription initiation sequence (+1) of a DNA template strand, the degree of synthesis up to the downstream sequence is high. The high processivity of the RNA polymerase II may mean that in the synthesis of RNA from the transcription initiation sequence (+1) of a DNA template strand, the synthesis proceeds from the transcription initiation sequence up to the downstream sequence, and the degree to which a longer RNA transcript is synthesized is high. The high processivity of the RNA polymerase II may mean that in the synthesis of RNA from the transcription initiation sequence (+1) of a DNA template strand, the degree to which RNA is synthesized from the transcription initiation sequence up to the termination sequence or its peripheral part is high.

The SPT4, SPT5 or SPT4/5 protein may be involved in the progressivity regulation of RNA polymerase II. The SPT4, SPT5 or SPT4/5 protein may maintain or increase the processivity of the RNA polymerase II.

The SPT4, SPT5 or SPT4/5 protein may provide the function of a transcription regulator. The SPT4, SPT5 or SPT4/5 protein may be involved in any one or more processes of the initiation, elongation or termination of the transcription process in order to provide the function of the transcription regulator. The SPT4, SPT5 or SPT4/5 protein may provide the function of a factor which regulates the transcription elongation process.

The SPT4, SPT5 or SPT4/5 protein may provide the function of a transcription elongation factor. The SPT4, SPT5 or SPT4/5 protein acts as a transcription elongation factor, and thus may provide a function capable of obtaining RNA products with higher efficiency from an RNA polymerase.

The SPT4, SPT5 or SPT4/5 protein may be involved in the regulation of a specific gene including an over-amplified repeated sequence.

The "over-amplified repeated sequence" may be a specific nucleotide sequence including duplication and over-duplication of the repeated sequence.

The over-amplified repeated sequence may include a first repeated part including duplication of the repeated sequence; and a second repeated part including over-duplication of the repeated sequence. The first repeated part is a repeated sequence included in a diseased subject and a normal subject. The second repeated part is a repeated sequence which is included in a gene of a diseased subject, but is not included in a gene of a normal subject.

The regulation of the expression of a specific gene including the over-amplified repeated sequence may be the regulation of the expression of the second repeated part. The expression may be any one or more including transcription, post-transcriptional processing, translation, and post-translational modification.

The SPT4, SPT5 or SPT4/5 protein may regulate the expression of the second repeated part in the expression of a specific gene including an over-amplified repeated sequence. The regulation of the expression in which the SPT4, SPT5 or SPT4/5 protein is involved may be the regulation of transcription.

The SPT4, SPT5 or SPT4/5 protein may maintain or increase the expression of the second repeated part in the expression of a specific gene including an over-amplified repeated sequence. The expression may be transcription. The maintenance or increase of expression in which the SPT4, SPT5 or SPT4/5 protein is involved may be the maintenance of or an increase in transcription.

As a result of regulation of the expression of the second repeated part in the expression of a specific gene including the over-amplified repeated sequence by the SPT4, SPT5 or SPT4/5 protein, the transcription of a second repeated part may occur. As a result of transcription of the second repeated part, a polypeptide encoded by the second repeated part may be expressed. The polypeptide encoded by the second repeated part may correspond to a simple repetition of a specific amino acid sequence. The simple repeat number of the specific amino acid sequence may be determined according to the disease.

For example, as a result of regulation of the expression of the second repeated part in the expression of the HTT gene by the SPT4, SPT5 or SPT4/5 protein, the transcription of a CAG-nucleotide which is a second repeated part of the HTT gene may occur. Poly-glutamine may be expressed from a transcript of the second repeated part.

The transcription of a specific gene including the over-amplified repeated sequence may occur by RNA polymerase II. The SPT4, SPT5 or SPT4/5 protein may be involved in the process of transcribing a specific gene including a repeated sequence in which the RNA polymerase II is over-amplified. The SPT4, SPT5 or SPT4/5 protein may provide a function of enhancing the processivity of the RNA polymerase II in the process of transcribing a specific gene including a repeated sequence in which the RNA polymerase II is over-amplified. The SPT4, SPT5 or SPT4/5 protein may provide a function of a transcription elongation factor in the process of transcribing a specific gene including a repeated sequence in which the RNA polymerase II is over-amplified.

The "expression regulatory gene of an over-amplified repeated sequence" may be, for example, an SUPT4H gene and/or SUPT5H gene.

The SUPT4H gene and/or SUPT5H gene may be an SPT4 gene and/or SPT5 gene derived from mammals.

The SUPT4H gene may be referred to as an SUPT4H1, SPT4, SPT4H, SUPT4H, or Supt4a gene. The SUPT5H gene may be referred to as an SPT5, SPT5H, or Tat-CT1 gene.

An expression product from the SUPT4H gene and/or SUPT5H gene may be an SUPT4H, SUPT5H or SUPT4/5H protein.

The SUPT4H, SUPT5H or SUPT4/5H protein which is an expression product from the SUPT4H gene and/or SUPT5H gene may provide a function equal or similar to that of the SPT4, SPT5 or SPT4/5 protein.

The SUPT4H, SUPT5H or SUPT4/5H protein may function as a transcription regulator in the RNA polymerization process of an RNA polymerase.

The SUPT4H, SUPT5H or SUPT4/5H protein may be involved in the processivity of the RNA polymerase II.

The SUPT4H, SUPT5H or SUPT4/5H protein may be involved in the regulation of the processivity of the RNA polymerase II. The SUPT4H, SUPT5H or SUPT4/5H protein may maintain or increase the processivity of the RNA polymerase II.

The SUPT4H, SUPT5H or SUPT4/5H protein may provide a function of a transcription regulator. The SUPT4H, SUPT5H or SUPT4/5H protein may be involved in any one or more processes of the initiation, elongation, or termination of the transcription process in order to provide the function of the transcription regulator. The SUPT4H, SUPT5H or SUPT4/5H protein may provide a function of a factor which regulates the transcription elongation process.

The SUPT4H, SUPT5H or SUPT4/5H protein may provide a function of a transcription elongation factor. The SUPT4H, SUPT5H or SUPT4/5H protein acts as a transcription elongation factor, and thus may provide a function capable of obtaining mRNA products with higher efficiency from an RNA polymerase.

The SUPT4H, SUPT5H or SUPT4/5H protein may regulate the expression of the second repeated part in the expression of a specific gene including an over-amplified repeated sequence. The regulation of the expression in which the SUPT4H, SUPT5H or SUPT4/5H protein is involved may be the regulation of transcription.

The SUPT4H, SUPT5H or SUPT4/5H protein may maintain or increase the expression of the second repeated part in the expression of a specific gene including an over-amplified repeated sequence. The maintenance or increase of expression in which the SUPT4H, SUPT5H or SUPT4/5H protein is involved may be the maintenance of or an increase in transcription.

As a result of regulation of the expression of the second repeated part in the expression of a specific gene including the over-amplified repeated sequence by the SUPT4H, SUPT5H or SUPT4/5H protein, the transcription of a second repeated part may occur. As a result of transcription of the second repeated part, a polypeptide encoded by the second repeated part may be expressed. The polypeptide encoded by the second repeated part may correspond to a simple repetition of a specific amino acid sequence. The simple repeat number of the specific amino acid sequence may be determined according to the disease.

For example, as a result of regulation of the expression of the second repeated part in the expression of the HTT gene by the SUPT4H, SUPT5H or SUPT4/5H protein, the transcription of a CAG-nucleotide which is a second repeated part of the HTT gene may occur. Poly-glutamine may be expressed from a transcript of the second repeated part.

The transcription of a specific gene including the over-amplified repeated sequence may occur by RNA polymerase II. The SUPT4H, SUPT5H or SUPT4/5H protein may be involved in the process of transcribing a specific gene including an over-amplified repeated sequence by the RNA polymerase II. The SUPT4H, SUPT5H or SUPT4/5H protein may provide a function of enhancing the processivity of the RNA polymerase II in the process of transcribing a specific gene including a repeated sequence in which the RNA polymerase II is over-amplified. The SUPT4H, SUPT5H or SUPT4/5H protein may provide a function of a transcription elongation factor in the process of transcribing a specific gene including a repeated sequence in which the RNA polymerase II is over-amplified.

The genes may be derived from mammals including primates such as humans and monkeys, rodents such as rats and mice, and the like.

Information on the genes may be obtained from a publicly-known database such as GenBank of the National Center for Biotechnology Information (NCBI).

The "regulation of the expression of an over-amplified repeated sequence" may include those resulting by regulating the expression of an expression regulatory gene of an over-amplified repeated sequence.

The "regulation of the expression of an expression regulatory gene of an over-amplified repeated sequence" may be a reduction in expression of the expression regulatory gene of the over-amplified repeated sequence.

The regulation of the expression of the expression regulatory gene of the over-amplified repeated sequence may be caused by the manipulation of the expression regulatory gene of the over-amplified repeated sequence.

The manipulation of the gene may be a deletion, substitution, or insertion of one or more nucleotides into a target gene.

The manipulation of the gene may be a change in one element which is involved in transcription, post-transcriptional modification, translation, or post-translational modification of a target gene.

The manipulation of the gene may be an artificial modification of one or more nucleotides in a target gene and/or reduction of its expression product.

The expression product may be an mRNA and/or a protein.

The expression product may be an expression product in which an expression level thereof is reduced or suppressed compared to that of an expression product expressed from a gene which is not artificially manipulated.

The manipulation of the gene may cause a reduction in an expression product from a target gene. The expression product may be an RNA transcribed from the target gene. The expression product may be a polypeptide translated from the target gene. The expression product may be a protein expressed from the target gene. The expression product may be a protein having activity among proteins expressed from the target gene.

The manipulation of the gene may be a knockdown or knockout of a target gene.

In this case, the knockdown may be an effect by artificial manipulation or modification of a target gene.

In this case, the knockout may be an effect by artificial manipulation or modification of a target gene.

The knockdown of the target gene may regulate the expression of the target gene. The knockdown of the target gene may induce RNA interference in the target gene. The knockdown of the target gene may decrease an amount of polypeptide translated from the RNA of the target gene. The knockdown of the target gene may decrease an amount of protein expressed from the target gene.

The knockout of the target gene may regulate the expression of the target gene. The knockout of the target gene may manipulate the DNA of the target gene. The DNA of the target gene may be manipulated by gene scissors. The gene scissors may be a clustered regularly interspaced short palindromic repeats(CRISPR)-CRISPR associated protein (Cas) system, a zinc finger nuclease(ZFN), a transcription activator-like effector nuclease(TALEN), FokI, or an endonuclease, but is not limited thereto. The knockout of the target gene may manipulate the target gene such that an abnormal RNA is transcribed from the DNA of the target gene. The knockout of the target gene may manipulate the target gene such that an RNA is not transcribed from the DNA of the target gene. The knockout of the target gene may manipulate the target gene such that a normal polypeptide is not translated from the DNA of the target gene. The knockout of the target gene may manipulate the target gene such that a protein is not expressed from the DNA of the target gene.

The target gene may be an expression regulatory gene of an over-amplified repeated sequence.

The manipulation of the gene may be a change in one element which is involved in transcription, post-transcriptional modification, translation, or post-translational modification of the expression regulatory gene of the over-amplified repeated sequence.

The manipulation of the gene may induce a decrease in expression product from the expression regulatory gene of the over-amplified repeated sequence. The expression product may be an RNA transcribed from the expression regulatory gene of the over-amplified repeated sequence. The expression product may be a polypeptide translated from the expression regulatory gene of the over-amplified repeated sequence. The expression product may be a protein having activity among proteins expressed from the expression regulatory gene of the over-amplified repeated sequence.

The manipulation of the gene may be a knockout or knockdown of the expression regulatory gene of the over-amplified repeated sequence.

In this case, the knockdown may be an effect by artificial manipulation or modification of the expression regulatory gene of the over-amplified repeated sequence.

In this case, the knockout may be an effect by artificial manipulation or modification of the expression regulatory gene of the over-amplified repeated sequence.

The knockdown of the expression regulatory gene of the over-amplified repeated sequence may regulate the expression of the expression regulatory gene of the over-amplified repeated sequence. The knockdown of the expression regulatory gene of the over-amplified repeated sequence may induce RNA interference in the expression regulatory gene of the over-amplified repeated sequence. The knockdown of the expression regulatory gene of the over-amplified repeated sequence may decrease an amount of polypeptide translated from the RNA of the expression regulatory gene of the over-amplified repeated sequence. The knockdown of the expression regulatory gene of the over-amplified repeated sequence may decrease an amount of protein expressed from the gene of the expression regulatory gene of the over-amplified repeated sequence.

The knockout of the expression regulatory gene of the over-amplified repeated sequence may regulate the expression of the expression regulatory gene of the over-amplified repeated sequence. The knockout of the expression regulatory gene of the over-amplified repeated sequence may manipulate the DNA of the expression regulatory gene of the over-amplified repeated sequence. The DNA of the expression regulatory gene of the over-amplified repeated sequence may be manipulated by gene scissors. The gene scissors may be ZFN, TALEN, or a CRISPR-Cas system. The knockout of the expression regulatory gene of the over-amplified repeated sequence may manipulate the expression regulatory gene of the over-amplified repeated sequence, such that an abnormal RNA is transcribed from the DNA of the expression regulatory gene of the over-amplified repeated sequence. The knockout of the expression regulatory gene of the over-amplified repeated sequence may manipulate the expression regulatory gene of the over-amplified repeated sequence, such that an RNA is not transcribed from the DNA of the expression regulatory gene of the over-amplified repeated sequence. The knockout of the expression regulatory gene of the over-amplified repeated sequence may manipulate the expression regulatory gene of the over-amplified repeated sequence, such that a normal polypeptide is not translated from the DNA of the expression regulatory gene of the over-amplified repeated sequence. The knockout of the expression regulatory gene of the over-amplified repeated sequence may manipulate the expression regulatory gene of the over-amplified repeated sequence, such that a protein is not expressed from the DNA of the expression regulatory gene of the over-amplified repeated sequence.

The expression regulatory gene of the over-amplified repeated sequence may be an SPT4 gene and/or SPT5 gene.

The manipulation of the gene may be a change in one element which is involved in transcription, post-transcriptional modification, translation, or post-translational modification of the SPT4 gene and/or SPT5 gene.

The manipulation of the gene may induce a decrease in expression product from the SPT4 gene and/or SPT5 gene. The expression product may be an RNA transcribed from the SPT4 gene and/or SPT5 gene. The expression product may be a polypeptide translated from the SPT4 gene and/or SPT5 gene. The expression product may be a protein having activity among proteins expressed from the SPT4 gene and/or SPT5 gene.

The manipulation of the gene may be a knockout or knockdown of the SPT4 gene and/or SPT5 gene.

In this case, the knockdown may be an effect by artificial manipulation or modification of the expression regulatory gene of the over-amplified repeated sequence.

In this case, the knockout may be an effect by artificial manipulation or modification of the SPT4 gene and/or SPT5 gene.

The knockdown of the SPT4 gene and/or SPT5 gene may regulate the expression of the SPT4 gene and/or SPT5 gene. The knockdown of the SPT4 gene and/or SPT5 gene may induce RNA interference in the SPT4 gene and/or SPT5 gene. The knockdown of the SPT4 gene and/or SPT5 gene may decrease an amount of polypeptide translated from an RNA of the SPT4 gene and/or SPT5 gene. The knockdown of the SPT4 gene and/or SPT5 gene may decrease an amount of a protein expressed from the SPT4 gene and/or SPT5 gene.

The knockout of the SPT4 gene and/or SPT5 gene may regulate the expression of the SPT4 gene and/or SPT5 gene. The knockout of the SPT4 gene and/or SPT5 gene may manipulate a DNA of the SPT4 gene and/or SPT5 gene. The DNA of the SPT4 gene and/or SPT5 gene may be manipulated by gene scissors. The gene scissors may be ZFN, TALEN, or a CRISPR-Cas system. The knockout of the SPT4 gene and/or SPT5 gene may manipulate the SPT4 gene and/or SPT5 gene, such that an abnormal RNA is transcribed from the DNA of the SPT4 gene and/or SPT5 gene. The knockout of the SPT4 gene and/or SPT5 gene may manipulate the SPT4 gene and/or SPT gene, such that an RNA is not transcribed from the DNA of the SPT4 gene and/or SPT5 gene. The knockout of the SPT4 gene and/or SPT5 gene may manipulate the SPT4 gene and/or SPT5 gene, such that a normal polypeptide is not expressed from the DNA of the SPT4 gene and/or SPT5 gene. The knockout of the SPT4 gene and/or SPT5 gene may manipulate the SPT4 gene and/or SPT5 gene, such that a protein is not expressed from the DNA of the SPT4 gene and/or SPT5 gene.

The expression regulatory gene of the over-amplified repeated sequence may be an SUPT4H gene and/or SUPT5H gene.

The manipulation of the gene may be a change in one element which is involved in transcription, post-transcriptional modification, translation, or post-translational modification of the SUPT4H gene and/or SUPT5H gene.

The manipulation of the gene may induce a decrease in expression product from the SUPT4H gene and/or SUPT5H gene. The expression product may be an RNA transcribed from the SUPT4H gene and/or SUPT5H gene. The expression product may be a polypeptide translated from the SUPT4H gene and/or SUPT5H gene. The expression product may be a protein having activity among proteins expressed from the SUPT4H gene and/or SUPT5H gene.

The manipulation of the gene may be a knockdown or knockout of the SUPT4H gene and/or SUPT5H gene.

In this case, the knockdown may be an effect by artificial manipulation or modification of the SUPT4H gene and/or SUPT5H gene.

In this case, the knockout may be an effect by artificial manipulation or modification of SUPT4H gene and/or SUPT5H gene.

The knockdown of the SUPT4H gene and/or SUPT5H gene may regulate the expression of the SUPT4H gene and/or SUPT5H gene. The knockdown of the SUPT4H gene and/or SUPT5H gene may induce RNA interference in the SUPT4H gene and/or SUPT5H gene. The knockdown of the SUPT4H gene and/or SUPT5H gene may decrease an amount of polypeptide translated from an RNA of the SUPT4H gene and/or SUPT5H gene. The knockdown of the SUPT4H gene and/or SUPT5H gene may decrease an amount of a protein expressed from the SUPT4H gene and/or SUPT5H gene.

The knockout of the SUPT4H gene and/or SUPT5H gene may regulate the expression of the SUPT4H gene and/or SUPT5H gene. The knockout of the SUPT4H gene and/or SUPT5H gene may manipulate the DNA of the SUPT4H gene and/or SUPT5H gene. The DNA of the SUPT4H gene and/or SUPT5H gene may be manipulated by gene scissors. The gene scissors may be ZFN, TALEN, or a CRISPR-Cas system. The knockout of the SUPT4H gene and/or SUPT5H gene may manipulate the SUPT4H gene and/or SUPT5H gene, such that a normal RNA is not transcribed from the DNA of the SUPT4H gene and/or SUPT5H gene. The knockout of the SUPT4H gene and/or SUPT5H gene may manipulate the SUPT4H gene and/or SUPT5H gene, such that an RNA is not transcribed from the DNA of the SUPT4H gene and/or SUPT5H gene. The knockout of the SUPT4H gene and/or SUPT5H gene may manipulate the SUPT4H gene and/or SUPT5H gene, such that a normal polypeptide is not translated from the DNA of the SUPT4H gene and/or SUPT5H gene. The knockout of the SUPT4H gene and/or SUPT5H gene may manipulate the SUPT4H gene and/or SUPT5H gene, such that a protein is not expressed from the DNA of the SUPT4H gene and/or SUPT5H gene.

The knockout may be a deletion of some nucleotides included in the target gene.

The knockout may be a deletion of some nucleotides included in the expression regulatory gene of the over-amplified repeated sequence.

The knockout may be a deletion of some nucleotides included in the SPT4 gene and/or SPT5 gene.

The knockout may be a deletion of some nucleotides included in the SUPT4H gene and/or SUPT5H gene.

The nucleotide to be deleted may be a nucleotide fragment including a 2 bp or more nucleotide.

The nucleotide fragment to be deleted may have a size of 2 bp to 5 bp, 6 bp to 10 bp, 11 bp to 15 bp, 16 bp to 20 bp, 21 bp to 25 bp, 26 bp to 30 bp, 31 bp to 35 bp, 36 bp to 40 bp, 41 bp to 45 bp or 46 bp to 50 bp.

As a result of gene manipulation of the expression regulatory gene of the over-amplified repeated sequence, the expression of the expression regulatory gene of the over-amplified repeated sequence may be regulated.

As a result of regulation of the expression regulatory gene of the over-amplified repeated sequence, the expression of the over-amplified repeated sequence may be regulated.

As a result of regulation of the expression of the expression regulatory gene of the over-amplified repeated sequence, the expression of a second repeated part in the over-amplified repeated sequence may be regulated.

As a result of regulation of the expression of the expression regulatory gene of the over-amplified repeated sequence, the RNA polymerization process of an RNA polymerase may be regulated.

The RNA polymerase may be RNA polymerase II.

As a result of gene manipulation of the expression regulatory gene of the over-amplified repeated sequence, the processivity of the RNA polymerase II may be regulated.

As a result of knockdown of the expression regulatory gene of the over-amplified repeated sequence, the processivity of the RNA polymerase II may be reduced.

As a result of knockout of the expression regulatory gene of the over-amplified repeated sequence, the processivity of the RNA polymerase II may be reduced.

As a result of gene manipulation of the expression regulatory gene of the over-amplified repeated sequence, the expression of a protein acting as a transcription elongation factor in the RNA polymerase II may be regulated.

As a result of knockdown of the expression regulatory gene of the over-amplified repeated sequence, the expression of a protein acting as a transcription elongation factor for RNA polymerase II may be reduced. A transcript by the RNA polymerase II may be reduced due to the decrease in expression of the transcription elongation factor. The transcript may be the over-amplified repeated sequence. The transcript may be the second repeated part in the over-amplified repeated sequence.

As a result of knockout of the expression regulatory gene of the over-amplified repeated sequence, the expression of a protein acting as a transcription elongation factor for RNA polymerase II may be reduced. A transcript by the RNA polymerase II may be reduced due to the decrease in expression of the transcription elongation factor. The transcript may be the over-amplified repeated sequence. The transcript may be the second repeated part in the over-amplified repeated sequence.

As a result of gene manipulation of the expression regulatory gene of the over-amplified repeated sequence, the expression of a specific gene including an over-amplified repeated sequence may be regulated.

As a result of gene manipulation of the expression regulatory gene of the over-amplified repeated sequence, the expression of the second repeated part of the specific gene including the over-amplified repeated sequence may be regulated. Specifically, as a result of gene manipulation of the expression regulatory gene of the over-amplified repeated sequence, mRNA transcription from the second repeated part of the specific gene including the over-amplified repeated sequence may be regulated. The expression of a polypeptide from the second repeated part of the specific gene including the over-amplified repeated sequence may be regulated. As an example, as a result of gene manipulation of the expression regulatory gene of the over-amplified repeated sequence, mRNA transcription is normally performed from a first repeated part of the HTT-gene related to Huntington's disease, but mRNA transcription from a second repeated part may be reduced. As a result of gene manipulation of the expression regulatory gene of the over-amplified repeated sequence, a polypeptide is normally expressed from the first repeated part, but the expression of the polypeptide from the second repeated part may be reduced.

As a result of gene manipulation of the expression regulatory gene of the over-amplified repeated sequence, the mRNA transcribed from a specific gene including the over-amplified repeated sequence may have a shorter length than that prior to the gene manipulation. As a result of gene manipulation of the expression regulatory gene of the over-amplified repeated sequence, the polypeptide expressed from the specific gene including the over-amplified repeated sequence may be expressed in a shorter length than that prior to the gene manipulation. For example, as a result of gene manipulation of the expression regulatory gene of the over-amplified repeated sequence, the mRNA transcribed from the HTT gene may have a shorter length than that prior to the gene manipulation. As a result of gene manipulation of the expression regulatory gene of the over-amplified repeated sequence, the polypeptide expressed from the HTT gene may be expressed in a shorter length than the previous length.

As a result of knockdown of the expression regulatory gene of the over-amplified repeated sequence, the expression of the specific gene including the over-amplified repeated sequence may be reduced.

As a result of knockdown the expression regulatory gene of the over-amplified repeated sequence, the expression of the second repeated part of the specific gene including the over-amplified repeated sequence may be reduced.

As a result of knockout the expression regulatory gene of the over-amplified repeated sequence, the expression of the specific gene including the over-amplified repeated sequence may be reduced.

As a result of knockout of the expression regulatory gene of the over-amplified repeated sequence, the expression of the second repeated part of the specific gene including the over-amplified repeated sequence may be reduced.

As a result of gene manipulation of the expression regulatory gene of the over-amplified repeated sequence, the expression of the specific gene including the over-amplified repeated sequence is regulated, and thus, a repeat expansion disorder may be treated or alleviated.

As a result of knockdown of the expression regulatory gene of the over-amplified repeated sequence, an expression of the specific gene including the over-amplified repeated sequence is reduced, and thus, a repeat expansion disorder may be treated or alleviated.

As a result of knockout of the expression regulatory gene of the over-amplified repeated sequence, an expression of the specific gene including the over-amplified repeated sequence is reduced, and thus, a repeat expansion disorder may be treated or alleviated.

The expression regulatory gene of the over-amplified repeated sequence may be an SPT4 gene and/or SPT5 gene.

As a result of gene manipulation of the SPT4 gene and/or SPT5 gene, the expression of the SPT4 gene and/or SPT5 gene may be regulated.

As a result of regulation of the expression of the SPT4 gene and/or SPT5 gene, the RNA polymerization process of an RNA polymerase may be regulated.

The RNA polymerase may be RNA polymerase II.

As a result of gene manipulation of the SPT4 gene and/or SPT5 gene, the processivity of the RNA polymerase II may be regulated.

As a result of knockdown of the SPT4 gene and/or SPT5 gene, the processivity of the RNA polymerase II may be reduced.

As a result of knockout of the SPT4 gene and/or SPT5 gene, the processivity of the RNA polymerase II may be reduced.

As a result of gene manipulation of the SPT4 gene and/or SPT5 gene, the expression of a protein acting as a transcription elongation factor for RNA polymerase II may be regulated.

As a result of knockdown of the SPT4 gene and/or SPT5 gene, the expression of a protein acting as a transcription elongation factor for RNA polymerase II may be reduced.

As a result of knockout of the SPT4 gene and/or SPT5 gene, the expression of a protein acting as a transcription elongation factor for RNA polymerase II may be reduced.

As a result of gene manipulation of the SPT4 gene and/or SPT5 gene, the expression of a specific gene including an over-amplified repeated sequence may be regulated.

As a result of knockdown of the SPT4 gene and/or SPT5 gene, the expression of the specific gene including the over-amplified repeated sequence may be reduced.

As a result of knockout of the SPT4 gene and/or SPT5 gene, the expression of the repeated sequence including the over-amplified repeated sequence may be reduced.

As a result of gene manipulation of the SPT4 gene and/or SPT5 gene, the transcription of an mRNA from a second repeated part included in an over-amplified repeated sequence may be regulated. As a result of gene manipulation of the SPT4 gene and/or SPT5 gene, the expression of a polypeptide from a second repeated part included in an over-amplified repeated sequence may be regulated.

As a result of knockdown of the SPT4 gene and/or SPT5 gene, the transcription of an mRNA from a second repeated part included in an over-amplified repeated sequence may be reduced. As a result of gene manipulation of the SPT4 gene and/or SPT5 gene, the expression of a polypeptide from a second repeated part included in an over-amplified repeated sequence may be reduced.

As a result of knockout of the SPT4 gene and/or SPT5 gene, the transcription of an mRNA from a second repeated part included in an over-amplified repeated sequence may be reduced. As a result of gene manipulation of the SPT4 gene and/or SPT5 gene, the expression of a polypeptide from a second repeated part included in an over-amplified repeated sequence may be reduced.

As a result of gene manipulation of the SPT4 gene and/or SPT5 gene, the expression of the specific gene including the over-amplified repeated sequence is regulated, and thus, a repeat expansion disorder may be treated or alleviated.

As a result of knockdown of the SPT4 gene and/or SPT5 gene, the expression of the specific gene including the over-amplified repeated sequence is reduced, and thus, a repeat expansion disorder may be treated or alleviated.

As a result of knockout of the SPT4 gene and/or SPT5 gene, the expression of the specific gene including the over-amplified repeated sequence is reduced, and thus, a repeat expansion disorder may be treated or alleviated.

The expression regulatory gene of the over-amplified repeated sequence may be an SUPT4H gene and/or SUPT5H gene.

As a result of gene manipulation of SUPT4H gene and/or SUPT5H gene, the expression of the SUPT4H gene and/or SUPT5H gene may be regulated.

As a result of regulation of the expression of the SUPT4H gene and/or SUPT5H gene, the RNA polymerization process of an RNA polymerase may be regulated.

The RNA polymerase may be RNA polymerase II.

As a result of gene manipulation of the SUPT4H gene and/or SUPT5H gene, the processivity of the RNA polymerase II may be regulated.

As a result of knockdown of the SUPT4H gene and/or SUPT5H gene, the processivity of the RNA polymerase II may be reduced.

As a result of knockout of the SUPT4H gene and/or SUPT5H gene, the processivity of the RNA polymerase II may be reduced.

As a result of gene manipulation of the SUPT4H gene and/or SUPT5H gene, the expression of a protein acting as a transcription elongation factor for RNA polymerase II may be regulated.

As a result of knockdown of the SUPT4H gene and/or SUPT5H gene, the expression of a protein acting as a transcription elongation factor for RNA polymerase II may be reduced.

As a result of knockout of the SUPT4H gene and/or SUPT5H gene, the expression of a protein acting as a transcription elongation factor for RNA polymerase II may be reduced.

As a result of gene manipulation of the SUPT4H gene and/or SUPT5H gene, the expression of a specific gene including an over-amplified repeated sequence may be regulated.

As a result of knockdown of the SUPT4H gene and/or SUPT5H gene, the expression of the specific gene including the over-amplified repeated sequence may be reduced.

As a result of knockout of the SUPT4H gene and/or SUPT5H gene, the expression of the repeated sequence including the over-amplified repeated sequence may be reduced.

As a result of gene manipulation of the SUPT4H gene and/or SUPT5H gene, the transcription of an mRNA from a second repeated part included in an over-amplified repeated sequence may be regulated. As a result of gene manipulation of the SUPT4H gene and/or SUPT5H gene, the expression of a polypeptide from a second repeated part included in an over-amplified repeated sequence may be regulated.

As a result of knockdown of the SUPT4H gene and/or SUPT5H gene, the transcription of an mRNA from a second repeated part included in an over-amplified repeated sequence may be reduced. As a result of gene manipulation of the SUPT4H gene and/or SUPT5H gene, the expression of a polypeptide from a second repeated part included in an over-amplified repeated sequence may be reduced.

As a result of knockout of the SUPT4H gene and/or SUPT5H gene, the transcription of an mRNA from a second repeated part included in an over-amplified repeated sequence may be reduced. As a result of gene manipulation of the SUPT4H gene and/or SUPT5H gene, the expression of a polypeptide from a second repeated part included in an over-amplified repeated sequence may be reduced.

As a result of gene manipulation of the SUPT4H gene and/or SUPT5H gene, the expression of the specific gene including the over-amplified repeated sequence is regulated, and thus, a repeat expansion disorder may be treated or alleviated.

As a result of knockdown of the SUPT4H gene and/or SUPT5H gene, the expression of the specific gene including the over-amplified repeated sequence is reduced, and thus, a repeat expansion disorder may be treated or alleviated.

As a result of knockout of the SUPT4H gene and/or SUPT5H gene, the expression of the specific gene including the over-amplified repeated sequence is reduced, and thus, a repeat expansion disorder may be treated or alleviated.

The "over-amplified repeated sequence" is a nucleotide sequence in which a specific nucleotide sequence unit is highly repeated. The repeated sequence may be a repeat of 2- to 12-nucleotide sequence units. The repeated sequence may be a repeat of tri-, quad-, penta-, hexa- or dodeca-nucleotide sequence units, but is not limited thereto.

The over-amplified repeated sequence may be a sequence in which the repeated sequence is highly repeated in a specific nucleotide sequence or specific gene.

The over-amplified repeated sequence may be present in the coding or non-coding region of the gene. The coding region may include an exon. The over-amplified repeated sequence present in the exon may be present in a translation reading frame of a gene transcript. The over-amplified repeated sequence present in the exon may serve to provide a repeat codon. The repeat codon may encode polyglutamine (PolyQ). The polyglutamine may be caused by the over-repetition of a CAG-nucleotide sequence unit. The repeat codon may be non-polyglutamine. The non-coding region may be a promoter, 3'-UTR, intron or 5'-UTR region, but is not limited thereto.

The over-amplified repeated sequence may vary in terms of nucleotide composition.

The over-amplified repeated sequence may be a repetition of a 3-nucleotide sequence unit. The repetition of the 3-nucleotide sequence unit may be a repetition of a CAG-, CCG-, CTG-, CGG-, GAA-, GAC-, GCG-, GCA-, GCC- or GCT-nucleotide sequence unit, but is not limited thereto.

The over-amplified repeated sequence may be a repetition of a 4-nucleotide sequence unit. The repetition of the 4-nucleotide sequence unit may be a repetition of a CCTG-nucleotide sequence unit, but is not limited thereto.

The over-amplified repeated sequence may be a repetition of a 5-nucleotide sequence unit. The repetition of the 5-nucleotide sequence unit may be a repetition of an ATTCT- or TGGAA-nucleotide sequence unit, but is not limited thereto.

The over-amplified repeated sequence may be a repetition of a 6-nucleotide sequence unit. The repetition of the 6-nucleotide sequence unit may be a repetition of a GGCCTG- or GGGGCC-nucleotide sequence unit, but is not limited thereto.

The over-amplified repeated sequence may be a repetition of a 12-nucleotide sequence unit. The repetition of the 12-nucleotide sequence unit may be a repetition of a CCCCGCCCCGCG-nucleotide sequence unit, but is not limited thereto.

The genes may be derived from mammals including primates such as humans and monkeys, rodents such as rats and mice, and the like.

The over-amplified repeated sequence may be generated by DNA replication slippage. The replication slippage may be generated by a replication slippage event. Several repetitions of the replication slippage may generate duplication and over-duplication of a repeated sequence by forming a loop in the repeated sequence including a tandem arrangement.

The over-amplified repeated sequence may be preserved during the somatic cell replication process. The number of over-amplified repeated sequences may be preserved or amplified during the somatic cell replication process. The over-amplified repeated sequence may be preserved during the germ cell formation process. The number of over-amplified repeated sequences may be preserved or amplified during the germ cell formation process.

The over-amplified repeated sequence may be inherited. While the over-amplified repeated sequence is inherited through each generation, the number of repeated sequences may be preserved or amplified.

Information on genes including the above-described over-amplified repeated sequence may be obtained from a publicly-known database such as GenBank of the National Center for Biotechnology Information (NCBI).

Even a normal subject which is not suffering from a disease caused by over-duplication of the repeated sequence may have repetition of the repeated sequence. For the normal subject, the duplication of the repeated sequence is present at a safe level. The safe level may vary depending on a specific disease. For example, when the disease is Huntington's disease, the case where the duplication of the CAG-repeated sequences in the HTT gene is about 35 and less than 40 may be considered to be at a safe level. For example, in Huntington's disease, when the duplication of the CAG-repeated sequence in the HTT gene is about 35 and 40 or more, there is a possibility of it developing into a disease, thus this may be considered a non-safe level. The number of the duplications and/or over-duplications of the repeated sequence include(s) an error within a range of 20%.

The over-amplified repeated sequence causes an abnormal expression in the gene or affects the function of a protein, and thus may develop a disorder. The disorder caused by the over-amplified repeated sequence may act as a cause of a neurologic disorder, but is not limited thereto.

In general, the greater the number of over-amplified repeated sequences is, the more likely a disease may occur or the severity of the disease may be increased. The over-amplified repeated sequence is inherited, and the number of repeated sequences may be preserved or amplified while the over-amplified repeated sequence is inherited through each generation. When the number of repeated sequences is preserved or amplified, the genetic disease may be preserved or aggravated in a lineage.

A list of genetic diseases caused by specific over-amplified repeated sequences will be provided below (Table 1).

**[Table 1]**

| List of genetic diseases caused by over-amplified repeated sequences | | |
|---|---|---|
| Disease | Gene | Repeated sequence unit |
| Fragile-X site A (FRAXA) | FMR1 | CGG |
| Fragile-X site E (FRAXE) | FMR2 | CCG |
| Friedreich ataxia (FA) | FRDA | GAA |
| Myotonic dystrophy 1 (DM1) | DMPK/SIX | CTG |
| Myotonic dystrophy 2 (DM2) | ZNF9 | CCTG |
| Spinocerebellar ataxia 8 (SCA 8) | SCA8 | CTG |
| Spinocerebellar ataxia 10 (SCA 10) | ATXN10 | ATTCT |
| Spinocerebellar ataxia 12 (SCA 12) | PPP2R2B | CAG |
| Progressive myoclonus epilepsy | CSTB | CCCCGCCCCGCG |
| Fuchs corneal dystrophy | TCF4 | CUG |
| Amyotrophic lateral sclerosis (C9orf72 mutation) | C9orf72 | GGGGCC |
| Breast Cancer | AIB1 | CAG |
| Schizhophrenia | KCNN3 | CAG |
| Cleidocranial dysplasia | CBFA1 | GCG |
| Pseudoachondroplasia (PSACH),Multiple epiphyseal dysplasia (MED) | COMP | GAC |
| Kennedy disease (SBMA) | AR | CAG |
| Huntington's Disease (HD) | HTT | CAG |
| Huntington's Disease-like 2 | JPH3 | CAG |
| Dentatorubral-pallidoluysian atrophy (DRPLA) | DRPLA | CAG |
| Spinocerebellar ataxia 1 (SCA 1) | ATXN 1 | CAG |
| Spinocerebellar ataxia 2 (SCA 2) | ATXN2 | CAG |
| Machado-Joseph disease (SCA 3, MJD) | ATXN3 | CAG |
| Spinocerebellar ataxia 6 (SCA 6) | CACNA1A | CAG |
| Spinocerebellar ataxia 7 (SCA 7) | ATXN7 | CAG |
| Spinocerebellar ataxia 17 (SCA 17) | TBP | CAG |
| Oculopharyngeal muscular dystrophy (OPMD) | PABPN1 | GCN |
| Synpolydactyly type 2 (SPD) | HOXD13 | GCN |
| Cleidocranial dysplasia (CCD) | RUNX2 | GCN |
| hand-foot-genital syndrome (HFG) | HOXA13 | GCN |
| Holoprosencephaly (HPE5) | ZIC2 | GCN |
| Blepharophimosis ptosis epicanthus inversus syndrome (BPES) | FOXL2 | GCN |
| Congenital central hypoventilation syndrome (CCHS) | PHOX2B | GCN |
| Mental retardation with GH deficiency(MRGH) | SOX3 | GCN |
| XLMR spectrum due to ARX mutation | ARX | GCN |

Specific examples of the genetic disease caused by the over-amplified repeated sequence may include: Huntington's Disease (HD); Huntington's Disease-like 2; dentatorubropallidoluysian atrophy (DRPLA); spinal and bulbar muscular atrophy (SBMA); spinocerebellar ataxia (SCA); fragile X syndrome (FXS); fragile X-associated tremor/ataxia syndrome (FXTAS); fragile XE mental retardation; X-linked mental retardation (XLMR) caused by ARX mutations; Fuchs corneal dystrophy; Friedreich's ataxia (FRDA); myotonic dystrophy; amyotrophic lateral sclerosis (C9orf72mutation); cleidocranial dysplasia; oculopharyngeal muscular dystrophy; synpolydactyly type 2; hand-foot-genital syndrome (HFGS); holoprosencephaly; blepharophimosis ptosis epicanthus inversus syndrome; congenital central hypoventilation syndrome; mental retardation with GH deficiency; and the like, but are not limited thereto.

A specific example of the genetic disease caused by the over-amplified repeated sequence may be a disease caused by the over-amplification of a 3-nucleotide sequence unit. The disease caused by the over-amplification of the 3-nucleotide sequence unit may occur while the number of 3-nucleotide sequence units related to a specific gene is unstably and repeatedly increased.

The disease caused by the over-amplification of the 3-nucleotide sequence unit may be polyQ diseases or non-polyQ diseases.

The polyQ diseases may be caused by the over-amplification of a CAG-nucleotide sequence unit. The polyQ diseases may be caused by the over-amplification of the CAG-nucleotide sequence unit in the exon. Examples thereof may be dentatorubropallidoluysian atrophy (DRPLA), Huntington's disease (HD), Huntington's Disease-like 2 (HDL2), spinal and bulbar muscular atrophy (SBMA), spinocerebellar ataxia type 1 (SCA1), spinocerebellar ataxia type 2 (SCA2), spinocerebellar ataxia type 3 (SCA3), spinocerebellar ataxia type 6 (SCA6), spinocerebellar ataxia type 7 (SCA7), and spinocerebellar ataxia type 17 (SCA17), but are not limited thereto.

The non-polyQ diseases may be caused by the over-amplification of the extra CAG-nucleotide sequence unit in the coding region. Further, the non-polyQ diseases may be caused by the over-amplification of the nucleotide sequence in the non-coding region. The non-polyQ diseases may be fragile X syndrome (FXS; FRAXA), fragile X-associated tremor/ataxia syndrome (FXTAS), fragile XE mental retardation (FRAXE), Friedreich's ataxia (FRDA), myotonic dystrophy type 1 (DM1), Fuchs' corneal dystrophy, spinocerebellar ataxia type 8 (SCA8), spinocerebellar ataxia type 12 (SCA12), cleidocranial dysplasia, oculopharyngeal muscular dystrophy, synpolydactyly type 2, hand-foot-genital syndrome (HFGS), holoprosencephaly (HPE5), blepharophimosis ptosis epicanthus inversus syndrome (BPES), congenital central hypoventilation syndrome, mental retardation with GH deficiency (MRGH), X-linked mental retardation (XLMR) caused by ARX mutations, but are not limited thereto.

A specific example of the genetic disease caused by the over-amplification of the repeated sequence may be a disease caused by the over-amplification of a 4-nucleotide sequence. The disease caused by the over-amplification of the 4-nucleotide sequence may occur while the number of 4-nucleotide sequence units related to a specific gene is unstably and repeatedly increased. The disease caused by the over-amplification of the 4-nucleotide sequence may be myotonic dystrophy type 2 (DM 2) caused by the over-amplification of a CCTC-nucleotide sequence, but is not limited thereto.

A specific example of the genetic gene caused by the over-amplification of the repeated sequence may be a disease caused by the over-amplification of a 5-nucleotide sequence unit. The disease caused by the over-amplification of the 5-nucleotide sequence unit may occur while the number of 5-nucleotide sequence units related to a specific gene is unstably and repeatedly increased. The disease caused by the over-amplification of the 5-nucleotide sequence unit may be spinocerebellar ataxia type 10 (SCA10). The disease caused by the over-amplification of the 5-nucleotide sequence unit may be spinocerebellar ataxia type 31 (SCA 31) caused by the over-amplification of a TGGAA-nucleotide sequence unit, but is not limited thereto.

A specific example of the genetic disease by the over-amplified repeated sequence may be a disease caused by the over-amplification of a 6-nucleotide sequence unit. The disease caused by the over-amplification of the 6-nucleotide sequence unit may occur while the number of 6-nucleotide sequence units related to a specific gene is unstably and repeatedly increased. The disease caused by the over-amplification of the 6-nucleotide sequence unit may be spinocerebellar ataxia type 36 (SCA36) caused by the over-amplification of a GGCCTG-nucleotide sequence unit. The disease caused by the over-amplification of the 6-nucleotide sequence unit may be amyotrophic lateral sclerosis (C9orf72 mutation) caused by the over-amplification of a GGGGCC-nucleotide sequence unit, but is not limited thereto.

A specific example of the genetic disease by the over-amplified repeated sequence may be a disease caused by the over-amplification of a 12-nucleotide sequence unit. The disease caused by the over-amplification of the 12-nucleotide sequence unit may occur while the number of 12-nucleotide sequence units related to a specific gene is unstably and repeatedly increased. The disease caused by the over-amplification of the 12-nucleotide sequence unit may be progressive myoclonus epilepsy (PME) caused by the over-amplification of a CCCCGCCCCGCG-nucleotide sequence unit, but is not limited thereto.

According to still another aspect disclosed by the present specification, a composition for gene manipulation to reduce the expression of an over-amplified repeated sequence and a preparation method thereof may be provided.

An embodiment of the content disclosed by the present specification relates to a composition for gene manipulation to reduce the expression of an over-amplified repeated sequence.

The composition for gene manipulation may be target-specific gene scissors. The target-specific gene scissors may be a clustered regularly interspaced short palindromic repeats(CRISPR)- CRISPR associated protein(Cas) system, a zinc finger nuclease(ZFN), a transcription activator-like effector nuclease(TALEN), FokI, endonuclease or a mixture thereof, and may be preferably a CRISPR-Cas system, but is not limited thereto.

The gene may be one or more genes selected from the group consisting of an SPT4 gene, SPT5 gene, SUPT4H gene, and SUPT5H gene, but is not limited thereto.

The CRISPR-Cas system may include a guide RNA and a CRISPR enzyme.

Another embodiment of the content disclosed by the present specification relates to another composition for gene manipulation to reduce the expression of an over-amplified repeated sequence.

The other composition for gene manipulation may be
a composition for gene manipulation including a guide nucleic acid for target sequences of one or more genes selected from the group consisting of an SPT4 gene, SPT5 gene, SUPT4H gene, and SUPT5H gene, in which
the target sequence complementarily binds to the guide nucleic acid; or
the target sequence is complementary to a sequence complementarily binding with the guide nucleic acid;

The term "guide nucleic acid" refers to a nucleic acid capable of recognizing a target nucleic acid, gene, or chromosome, and capable of interact with an editor protein. Here, the guide nucleic acid is able to form a complementary bond with a target nucleic acid, gene, or partial nucleotide sequence of a chromosome. In addition, partial nucleic acid sequence of the guide nucleic acid may interact with amino acids contained in the editor protein, thereby forming a guide nucleic acid-editor protein complex.

The guide nucleic acid may perform a function of inducing the guide nucleic acid-editor proetin to be located in a target region of a target nucleic acid, a gene, or a chromosome.

The guide nucleic acid may be present in the form of DNA, RNA or a DNA/RNA mixture, and have a 5 to 150-nucleic acid sequence.

The guide nucleic acid may be one continuous nucleic acid sequence.

For example, the one continuous nucleic acid sequence may be (N)m, where N is A, T, C or G, or A, U, C or G, and m is an integer of 1 to 150.

The guide nucleic acid may be two or more continuous nucleic acid sequences.

For example, the two or more continuous nucleic acid sequences may be (N)m and (N)o, where N represents A, T, C or G, or A, U, C or G, m and o are an integer of 1 to 150, and may be the same as or different from each other.

The guide nucleic acid may include one or more domains.

The domains may be, but are not limited to, a guide domain, a first complementary domain, a linker domain, a second complementary domain, a proximal domain, or a tail domain.

Here, one guide nucleic acid may includes two or more functional domains. Here, the two or more functional domains may be different from each other. Or, two or more functional domains which are included in one guide nucleic acid may be the same as each other. For example, one guide nucleic acid may have two or more proximal domains, For another example, one guide nucleic acid may have two or more tail domains. However, the meaning of that the functional domains included in one guide nucleic acid are the same domains are not that the sequences of the two functional domains are the same. Even if the sequences are different, two functional domains are the same domains when they perform the same function.

Hereinafter, functional domain will be described in detail.

The term "guide domain" is a domain having a complementary guide sequence which is able to form a complementary bond with a target sequence on a target gene or nucleic acid, and serves to specifically interact with the target gene or nucleic acid. For example, the guide domain may perform a function of inducing the guide nucleic acid-editor protein complex to a region having specific nucleotide sequence of target gene or nucleic acid.

The guide domain may be a sequence of 10 to 35 bases.

In an example, the guide domain may be a sequence of 10 to 35, 15 to 35, 20 to 35, 25 to 35, 30 to 35 bases.

In another example, the guide domain may be a sequence of 10 to 15, 15 to 20, 20 to 25, 25 to 30, 30 to 35 bases.

The guide domain may have a guide sequence.
"the guide sequence" is nucleotide sequence which is able to form a complementary bond with a target gene or a partial sequence of a single strand of double-stranded nucleic acid, wherein the guide sequence may be a nucleotide sequence which has at least 50% or more, 55%, 60%, 65%, 70%, 75%, 80%, 85%, 90% or 95% complementarity or complete complementarity.

The guide sequence may be a sequence of 10 to 25 bases.

In an example, the guide domain may be a sequence of 10 to 25, 15 to 25, or 20 to 25 bases.

In another example, the guide domain may be a sequence of 10 to 15, 15 to 20, or 20 to 25 bases.

In addition, the guide domain may include an additional base sequence.

The additional base sequence may be utilized to improve or degrade the function of the guide domain.

The additional base sequence may be utilized to improve or degrade the function of the guide sequence.

The additional base sequence may be a 1 to 10 base sequence.

In one example, the additional base sequence may be a 2 to 10, 4 to 10, 6 to 10, or 8 to 10 base sequence.

In another example, the additional base sequence may be a 1 to 3, 3 to 6, or 7 to 10 base sequence.

In another example, the additional base sequence may be a 1, 2, 3, 4, 5, 6, 7, 8, 9, or 10 base sequence.

In an example, the additional base sequence may be a 1 base sequence, Guanine(G), or 2 base sequence, GG.

The additional base sequence may be located at the 5'end of the guide sequence.

The additional base sequence may be located at the 3'end of the guide sequence.

The term "first complementary domain" is a nucleic acid sequence including a nucleic acid sequence complementary to a second complementary domain, and has enough complementarity so as to form a double strand with the second complementary domain. For example, The first complementary domain may be a nucleic acid sequence complementary to the second complementary domain, which has, at least 50%, 55%, 60%, 65%, 70%, 75%, 80%, 85%, 90% or 95% or more complementarity or complete complementarity.

The first complementary domain is able to form a complementary bond with the second complementary domain to form double strands. Here, the double strands may interact with amino acids contained in the editor protein, thereby forming a guide nucleic acid-editor protein complex.

The first complementary domain may be a 5 to 35-base sequence.

In an example, the first complementary domain may be a 5 to 35, 10 to 35, 15 to 35, 20 to 35, 25 to 35, or 30 to 35-base sequence.

In another example, the first complementary domain may be a 1 to 5, 5 to 10, 10 to 15, 15 to 20, 20 to 25, 25 to 30 or 30 to 35-base sequence.

The term "linker domain" is a nucleic acid sequence connecting two or more domains, which are two or more identical or different domains. The linker domain may be connected with two or more domains by covalent bonding or non-covalent bonding, or may connect two or more domains by covalent bonding or non-covalent bonding.

The linker domain may be a 1 to 30-base sequence.

In one example, the linker domain may be a 1 to 5, 5 to 10, 10 to 15, 15 to 20, 20 to 25, or 25 to 30-base sequence.

In another example, the linker domain may be a 1 to 30, 5 to 30, 10 to 30, 15 to 30, 20 to 30, or 25 to 30-base sequence.

The term "second complementary domain" is a nucleic acid sequence including a nucleic acid sequence complementary to the first complementary domain, and has enough complementarity so as to form a double strand with the first complementary domain. For example, The second complementary domain may be a nucleic acid sequence complementary to the first complementary domain, which has, at least 50%, 55%, 60%, 65%, 70%, 75%, 80%, 85%, 90% or 95% or more complementarity or complete complementarity.

The second complementary domain is able to form a complementary bond with the first complementary domain to form double strands. Here, the double strands may interact with amino acids contained in the editor protein, thereby forming a guide nucleic acid-editor protein complex.

The second complementary domain may have a base sequence complementary to the first complementary domain, and a base sequence having no complementarity to the first complementary domain, for example, a base sequence not forming a double strand with the first complementary domain, and may have a longer base sequence than the first complementary domain.

The second complementary domain may have a 5 to 35-base sequence.

In an example, the second complementary domain may be a 1 to 35, 5 to 35, 10 to 35, 15 to 35, 20 to 35, 25 to 35, or 30 to 35-base sequence.

In another example, the second complementary domain may be a 1 to 5, 5 to 10, 10 to 15, 15 to 20, 20 to 25, 25 to 30, or 30 to 35-base sequence.

The term "proximal domain" is a nucleic acid sequence located adjacent to the second complementary domain.

The proximal domain may have a complementary base sequence therein, and may be formed in a double strand due to a complementary base sequence.

The proximal domain may be a 1 to 20-base sequence.

In one example, the proximal domain may be a 1 to 20, 5 to 20, 10 to 20 or 15 to 20-base sequence.

In another example, the proximal domain may be a 1 to 5, 5 to 10, 10 to 15 or 15 to 20-base sequence.

The term "tail domain" is a nucleic acid sequence located at one or more ends of the both ends of the guide nucleic acid.

The tail domain may have a complementary base sequence therein, and may be formed in a double strand due to a complementary base sequence.

The tail domain may be a 1 to 50-base sequence.

In an example, the tail domain may be a 5 to 50, 10 to 50, 15 to 50, 20 to 50, 25 to 50, 30 to 50, 35 to 50, 40 to 50, or 45 to 50-base sequence.

In another example, the tail domain may be a 1 to 5, 5 to 10, 10 to 15, 15 to 20, 20 to 25, 25 to 30, 30 to 35, 35 to 40, 40 to 45, or 45 to 50-base sequence.

Meanwhile, a part or all of the nucleic acid sequences included in the domains, that is, the guide domain, the first complementary domain, the linker domain, the second complementary domain, the proximal domain and the tail domain may selectively or additionally include a chemical modification.

The chemical modification may be, but is not limited to, methylation, acetylation, phosphorylation, phosphorothioate linkage, a locked nucleic acid (LNA), 2'-O-methyl 3'phosphorothioate (MS) or 2'-O-methyl 3'thioPACE (MSP).

The guide nucleic acid includes one or more domains.

The guide nucleic acid may include a guide domain.

The guide nucleic acid may include a first complementary domain.

The guide nucleic acid may include a linker domain.

The guide nucleic acid may include a second complementary domain.

The guide nucleic acid may include a proximal domain.

The guide nucleic acid may include a tail domain.

Here, there may be 1, 2, 3, 4, 5, 6 or more domains.

The guide nucleic acid may include 1, 2, 3, 4, 5, 6 or more guide domains.

The guide nucleic acid may include 1, 2, 3, 4, 5, 6 or more first complementary domains.

The guide nucleic acid may include 1, 2, 3, 4, 5, 6 or more linker domains.

The guide nucleic acid may include 1, 2, 3, 4, 5, 6 or more second complementary domains.

The guide nucleic acid may include 1, 2, 3, 4, 5, 6 or more proximal domains.

The guide nucleic acid may include 1, 2, 3, 4, 5, 6 or more tail domains.

Here, in the guide nucleic acid, one type of domain may be duplicated.

The guide nucleic acid may include several domains with or without duplication.

The guide nucleic acid may include the same type of domain. Here, the same type of domain may have the same nucleic acid sequence or different nucleic acid sequences.

The guide nucleic acid may include two types of domains. Here, the two different types of domains may have different nucleic acid sequences or the same nucleic acid sequence.

The guide nucleic acid may include three types of domains. Here, the three different types of domains may have different nucleic acid sequences or the same nucleic acid sequence.

The guide nucleic acid may include four types of domains. Here, the four different types of domains may have different nucleic acid sequences, or the same nucleic acid sequence.

The guide nucleic acid may include five types of domains. Here, the five different types of domains may have different nucleic acid sequences, or the same nucleic acid sequence.

The guide nucleic acid may include six types of domains. Here, the six different types of domains may have different nucleic acid sequences, or the same nucleic acid sequence.

For example, the guide nucleic acid may consist of [guide domain]-[first complementary domain]-[linker domain]-[second complementary domain]-[linker domain]-[guide domain]-[first complementary domain]-[linker domain]-[second complementary domain]. Here, the two guide domains may include guide sequences for different or the same targets, the two first complementary domains and the two second complementary domains may have the same or different nucleic acid sequences. When the guide domains include guide sequences for different targets, the guide nucleic acids may specifically bind to two different targets, and here, the specific bindings may be performed simultaneously or sequentially. In addition, the linker domains may be cleaved by specific enzymes, and the guide nucleic acids may be divided into two or three parts in the presence of specific enzymes.

As a specific example of the present speicification, the guide nucleic acid may be a gRNA .

The term "gRNA" refers to a nucleic acid capable of specifically targeting a gRNA-CRISPR enzyme complex, that is, a CRISPR complex, with respect to a target gene or nucleic acid. In addition, the gRNA is a nucleic acid-specific RNA which may bind to a CRISPR enzyme and guide the CRISPR enzyme to the target gene or nucleic acid.

The gRNA may include multiple domains. Due to each domain, interactions may occur in a three-dimensional structure or active form of a gRNA strand, or between these strands.

The gRNA may be called single-stranded gRNA (single RNA molecule); or double-stranded gRNA (including more than one, generally, two discrete RNA molecules).

In one exemplary embodiment, the single-stranded gRNA may include a guide domain, that is, a domain including a guide sequence capable of forming a complementary bond with a target gene or nucleic acid; a first complementary domain; a linker domain; a second complementary domain, a domain having a sequence complementary to the first complementary domain sequence, thereby forming a double-stranded nucleic acid with the first complementary domain; a proximal domain; and optionally a tail domain in the 5' to 3' direction.

In another embodiment, the double-stranded gRNA may include a first strand which includes a guide domain, that is, a domain including a guide sequence capable of forming a complementary bond with a target gene or nucleic acid and a first complementary domain; and a second strand which includes a second complementary domain, a domain having a sequence complementary to the first complementary domain sequence, thereby forming a double-stranded nucleic acid with the first complementary domain, a proximal domain; and optionally a tail domain in the 5' to 3' direction.

Here, the first strand may be referred to as crRNA, and the second strand may be referred to as tracrRNA. The crRNA may include a guide domain and a first complementary domain, and the tracrRNA may include a second complementary domain, a proximal domain and optionally a tail domain.

In still another embodiment, the single-stranded gRNA may include a guide domain, that is, a domain including a guide sequence capable of forming a complementary bond with a target gene or nucleic acid; a first complementary domain; a second complementary domain, and a domain having a sequence complementary to the first complementary domain sequence, thereby forming a double-stranded nucleic acid with the first complementary domain in the 3' to 5' direction.

Here, the first complementary domain may have homology with a natural first complementary domain, or may be derived from a natural first complementary domain. In addition, the first complementary domain may have a difference in the base sequence of a first complementary domain depending on the species existing in nature, may be derived from a first complementary domain contained in the species existing in nature, or may have partial or complete homology with the first complementary domain contained in the species existing in nature.

In one exemplary embodiment, the first complementary domain may have partial, that is, at least 50% or more, or complete homology with a first complementary domain of Streptococcus pyogenes, Campylobacter jejuni, Streptococcus thermophilus, Streptococcus aureus or Neisseria meningitides, or a first complementary domain derived therefrom.

For example, when the first complementary domain is the first complementary domain of Streptococcus pyogenes or a first complementary domain derived therefrom, the first complementary domain may be 5'-GUUUUAGAGCUA-3' or a base sequence having partial, that is, at least 50% or more, or complete homology with 5'-GUUUUAGAGCUA-3'. Here, the first complementary domain may further include (X)ₙ, resulting in 5'-GUUUUAGAGCUA(X)ₙ-3'. The X may be selected from the group consisting of bases A, T, U and G, and the n may represent the number of bases, which is an integer of 5 to 15. Here, the (X)ₙ may be n repeats of the same base, or a mixture of n bases of A, T, U and G.

In another embodiment, when the first complementary domain is the first complementary domain of Campylobacter jejuni or a first complementary domain derived therefrom, the first complementary domain may be 5'-GUUUUAGUCCCUUUUUAAAUUUCUU-3', or a base sequence having partial, that is, at least 50% or more, or complete homology with 5'-GUUUUAGUCCCUUUUUAAAUUUCUU-3'. Here, the first complementary domain may further include(X)ₙ, resulting in 5'-GUUUUAGUCCCUUUUUAAAUUUCUU(X)ₙ-3'. The X may be selected from the group consisting of bases A, T, U and G, and the n may represent the number of bases, which is an integer of 5 to 15. Here, the (X)ₙ may represent n repeats of the same base, or a mixture of n bases of A, T, U and G.

In another embodiment, the first complementary domain may have partial, that is, at least 50% or more, or complete homology with a first complementary domain of Parcubacteria bacterium (GWC2011_GWC2_44_17), Lachnospiraceae bacterium (MC2017), Butyrivibrio proteoclasiicus, Peregrinibacteria bacterium (GW2011_GWA_33_10), Acidaminococcus sp. (BV3L6), Porphyromonas macacae, Lachnospiraceae bacterium (ND2006), Porphyromonas crevioricanis, Prevotella disiens, Moraxella bovoculi (237), Smiihella sp. (SC_KO8D17), Leptospira inadai, Lachnospiraceae bacterium (MA2020), Francisella novicida (U112), Candidatus Methanoplasma termitum or Eubacterium eligens, or a first complementary domain derived therefrom.

For example, when the first complementary domain is the first complementary domain of Parcubacteria bacterium or a first complementary domain derived therefrom, the first complementary domain may be 5'-UUUGUAGAU-3', or a base sequence having partial, that is, at least 50% or more homology with 5'-UUUGUAGAU-3'. Here, the first complementary domain may further include (X)ₙ, resulting in 5'-(X)ₙUUUGUAGAU-3'. The X may be selected from the group consisting of bases A, T, U and G, and the n may represent the number of bases, which is an integer of 1 to 5. Here, the (X)ₙ may represent n repeats of the same base, or a mixture of n bases of A, T, U and G.

Here, the linker domain may be a nucleotide sequence that serves to link the first complementary domain and the second complementary domain.

The linker domain is able to form covalent or non-covalent bonding with the first complementary domain and the second complementary domain, respectively.

The linker domain may connect the first complementary domain with second complementary domain by covalent or non-covalent bonding.

The linker domain is suitable to be used in a single-stranded gRNA molecule, and may be used to produce single-stranded gRNA by being connected with a first strand and a second strand of double-stranded gRNA or connecting the first strand with the second strand by covalent or non-covalent bonding.

The linker domain may be used to produce single-stranded gRNA by being connected with crRNA and tracrRNA of double-stranded gRNA or connecting the crRNA with the tracrRNA by covalent or non-covalent bonding.

In addition, the second complementary domain may have homology with a natural second complementary domain, or may be derived from the natural second complementary domain. In addition, the second complementary domain may have a difference in base sequence of a second complementary domain according to a species existing in nature, and may be derived from a second complementary domain contained in the species existing in nature, or may have partial or complete homology with the second complementary domain contained in the species existing in nature.

In an exemplary embodiment, the second complementary domain may have partial, that is, at least 50% or more, or complete homology with a second complementary domain of Streptococcus pyogenes, Campylobacter jejuni, Streptococcus thermophilus, Streptococcus aureus or Neisseria meningitides, or a second complementary domain derived therefrom.

For example, when the second complementary domain is a second complementary domain of Streptococcus pyogenes or a second complementary domain derived therefrom, the second complementary domain may be 5'-UAGCAAGUUAAAAU-3', or a base sequence having partial, that is, at least 50% or more homology with 5'-UAGCAAGUUAAAAU-3' (a base sequence forming a double strand with the first complementary domain is underlined). Here, the second complementary domain may further include (X)ₙ and/or (X)ₘ, resulting in 5'-(X)ₙ UAGCAAGUUAAAAU(X)*ₘ*-3'. The X may be selected from the group consisting of bases A, T, U and G, and each of the n and m may represent the number of bases, in which the n may be an integer of 1 to 15, and the m may be an integer of 1 to 6. Here, the (X)ₙ may represent n repeats of the same base, or a mixture of n bases of A, T, U and G. In addition, (X)ₘ may represent m repeats of the same base, or a mixture of m bases of A, T, U and G.

In another example, when the second complementary domain is the second complementary domain of Campylobacter jejuni or a second complementary domain derived therefrom, the second complementary domain may be 5'-AAGAAAUUUAAAAAGGGACUAAAAU-3', or a base sequence having partial, that is, at least 50% or more homology with 5'-AAGAAAUUUAAAAAGGGACUAAAAU -3' (a base sequence forming a double strand with the first complementary domain is underlined). Here, the second complementary domain may further include (X)ₙ and/or (X)ₘ, resulting in 5'-(X)ₙ AAGAAAUUUAAAAAGGGACUAAAAU(X)ₘ-3'. The X may be selected from the group consisting of bases A, T, U and G, and each of the n and m may represent the number of bases, in which the n may be an integer of 1 to 15, and the m may be an integer of 1 to 6. Here, (X)ₙ may represent n repeats of the same base, or a mixture of n bases of A, T, U and G. In addition, (X)ₘ may represent m repeats of the same base, or a mixture of m bases of A, T, U and G.

In another embodiment, the secondcomplementary domain may have partial, that is, at least 50% or more, or complete homology with a first complementary domain of Parcubacteria bacterium (GWC2011_GWC2_44_17), Lachnospiraceae bacterium (MC2017), Butyrivibrio proteoclasiicus, Peregrinibacteria bacterium (GW2011_GWA_33_10), Acidaminococcus sp. (BV3L6), Porphyromonas macacae, Lachnospiraceae bacterium (ND2006), Porphyromonas crevioricanis, Prevotella disiens, Moraxella bovoculi (237), Smiihella sp. (SC_KO8D17), Leptospira inadai, Lachnospiraceae bacterium (MA2020), Francisella novicida (U112), Candidatus Methanoplasma termitum or Eubacterium eligens, or a secondcomplementary domain derived therefrom.

For example, when the second complementary domain is a second complementary domain of Parcubacteria bacterium or a second complementary domain derived therefrom, the second complementary domain may be 5'-AAAUUUCUACU-3', or a base sequence having partial, that is, at least 50% or more homology with 5'-AAAUUUCUACU-3' (a base sequence forming a double strand with the first complementary domain is underlined). Here, the second complementary domain may further include (X)ₙ and/or (X)ₘ, resulting in 5'-(X)ₙAAAUUUCUACU (X)ₘ-3'. The X may be selected from the group consisting of bases A, T, U and G, and each of the n and m may represent the number of bases, in which the n may be an integer of 1 to 10, and the m may be an integer of 1 to 6. Here, the (X)ₙ may represent n repeats of the same base, or a mixture of n bases of A, T, U and G. In addition, the (X)ₘ may represent m repeats of the same base, or a mixture of m bases of A, T, U and G.

Here, the first complementary domain and the second complementary domain may form a complementary bond.
the fisrt complementary domain and the second complementary domain may form a double strands by the complementary bond.
the double strands may interact with CRISPR enzyme.

Selectively, the first complementary domain may include an additional nucleotide sequence which does not form a complementary bond with the second complementary domain.

Here, the additional nucleotide sequence may be a 1 to 15-base sequence. For example, the additional nucleotide sequence may be a 1 to 5, 5 to 10, or 10 to 15-base sequence.

Here, the proximal domain may be a domain located at the 3'end direction of the second complementary domain.

In addition, the proximal domain may have homology with a natural proximal domain, or may be derived from the natural proximal domain. In addition, the proximal domain may have a difference in base sequence according to a species existing in nature, may be derived from a proximal domain contained in the species existing in nature, or may have partial or complete homology with the proximal domain contained in the species existing in nature.

In an exemplary embodiment, the proximal domain may have partial, that is, at least 50% or more, or complete homology with a proximal domain of Streptococcus pyogenes, Campylobacter j ejuni, Streptococcus thermophilus, Streptococcus aureus or Neisseria meningitides, or a proximal domain derived therefrom.

For example, when the proximal domain is a proximal domain of Streptococcus pyogenes or a proximal domain derived therefrom, the proximal domain may be 5'-AAGGCUAGUCCG-3', or a base sequence having partial, that is, at least 50% or more homology with 5'-AAGGCUAGUCCG-3'. Here, the proximal domain may further include (X)ₙ, resulting in 5'-AAGGCUAGUCCG(X)ₙ-3'. The X may be selected from the group consisting of bases A, T, U and G, and the n may represent the number of bases, which is an integer of 1 to 15. Here, the (X)ₙ may represent n repeats of the same base, or a mixture of n bases of A, T, U and G.

In yet another example, when the proximal domain is a proximal domain of Campylobacter jejuni or a proximal domain derived therefrom, the proximal domain may be 5'-AAAGAGUUUGC-3', or a base sequence having at least 50% or more homology with 5'-AAAGAGUUUGC-3'. Here, the proximal domain may further include (X)ₙ, resulting in 5'-AAAGAGUUUGC(X)ₙ-3'. The X may be selected from the group consisting of bases A, T, U and G, and the n may represent the number of bases, which is an integer of 1 to 40. Here, the (X)ₙ may represent n repeats of the same base, or a mixture of n bases of A, T, U and G.

Here, the tail domain is a domain which is able to be selectively added to the 3' end of single-stranded gRNA or a first or a second strand of double-stranded gRNA.

In addition, the tail domain may have homology with a natural tail domain, or may be derived from the natural tail domain. In addition, the tail domain may have a difference in base sequence according to a species existing in nature, may be derived from a tail domain contained in a species existing in nature, or may have partial or complete homology with a tail domain contained in a species existing in nature.

In one exemplary embodiment, the tail domain may have partial, that is, at least 50% or more, or complete homology with a tail domain of Streptococcus pyogenes, Campylobacter jejuni, Streptococcus thermophilus, Streptococcus aureus or Neisseria meningitides or a tail domain derived therefrom.

For example, when the tail domain is a tail domain of Streptococcus pyogenes or a tail domain derived therefrom, the tail domain may be 5'-UUAUCAACUUGAAAAAGUGGCACCGAGUCGGUGC-3', or a base sequence having partial, that is, at least 50% or more homology with 5'-UUAUCAACUUGAAAAAGUGGCACCGAGUCGGUGC-3'. Here, the tail domain may further include (X)ₙ, resulting in 5'-UUAUCAACUUGAAAAAGUGGCACCGAGUCGGUGC(X)ₙ-3'. The X may be selected from the group consisting of bases A, T, U and G, and the n may represent the number of bases, which is an integer of 1 to 15. Here, the (X)ₙ may represent n repeats of the same base, or a mixture of n bases such as A, T, U and G.

In another example, when the tail domain is a tail domain of Campylobacter jejuni or a tail domain derived therefrom, the tail domain may be 5'-GGGACUCUGCGGGGUUACAAUCCCCUAAAACCGCUUUU-3', or a base sequence having partial, that is, at least 50% or more homology with 5'-GGGACUCUGCGGGGUUACAAUCCCCUAAAACCGCUUUU-3'. Here, the tail domain may further include (X)ₙ, resulting in 5'-GGGACUCUGCGGGGUUACAAUCCCCUAAAACCGCUUUU(X)ₙ-3'. The X may be selected from the group consisting of bases A, T, U and G, and the n may represent the number of bases, which is an integer of 1 to 15. Here, the (X)ₙ may represent n repeats of the same base, or a mixture of n bases of A, T, U and G.

In another embodiment, the tail domain may include a 1 to 10-base sequence at the 3' end involved in an in vitro or in vivo transcription method.

For example, when a T7 promoter is used in in vitro transcription of gRNA, the tail domain may be an arbitrary base sequence present at the 3' end of a DNA template. In addition, when a U6 promoter is used in in vivo transcription, the tail domain may be UUUUUU, when an H1 promoter is used in transcription, the tail domain may be UUUU, and when a pol-III promoter is used, the tail domain may include several uracil bases or alternative bases.

The gRNA may include a plurality of domains as described above, and therefore, the length of the nucleic acid sequence may be regulated according to a domain contained in the gRNA, and interactions may occur in strands in a three-dimensional structure or active form of gRNA or between theses strands due to each domain.

The gRNA may be referred to as single-stranded gRNA (single RNA molecule); or double-stranded gRNA (including more than one, generally two discrete RNA molecules).

The double-stranded gRNA consists of a first strand and a second strand.

Here, the first strand may consist of
5'-[guide domain]-[first complementary domain]-3', and
the second strand may consist of
5'-[second complementary domain]-[proximal domain]-3' or
5'-[second complementary domain]-[proximal domain]-[tail domain]-3'.

Here, the first strand may be referred to as crRNA, and the second strand may be referred to as tracrRNA.

Here, the first strand and the second strand strand may include an additional nucleotide sequence, selectively.

In one example, the first strand may be
5'-(N_{target})-(Q)ₘ-3'; or
5'-(X)ₐ-(N_{target})-(X)_{b}-(Q)ₘ-(X)_{c}-3'.

Here, the N_{target} is a base sequence capable of forming a complementary bond with a target sequence on a target gene or nucleic acid, and a base sequence region which may be changed according to a target sequence on a target gene or nucleic acid.

Here, the (Q)ₘ is a base sequence including the first complementary domain, which is able to form a complementary bond with the second complementary domain of the second strand. The (Q)ₘ may be a sequence having partial or complete homology with the first complementary domain of a species existing in nature, and the base sequence of the first complementary domain may be changed according to the species of origin. The Q may be each independently selected from the group consisting of A, U, C and G, and the m may be the number of bases, which is an integer of 5 to 35.

For example, when the first complementary domain has partial or complete homology with a first complementary domain of Streptococcus pyogenes or a Streptococcus pyogenes-derived first complementary domain, the (Q)ₘ may be 5'-GUUUUAGAGCUA-3', or a base sequence having at least 50% or more homology with 5'-GUUUUAGAGCUA-3'.

In another example, when the first complementary domain has partial or complete homology with a first complementary domain of Campylobacter jejuni or a Campylobacter jejuni-derived first complementary domain, the (Q)ₘ may be 5'-GUUUUAGUCCCUUUUUAAAUUUCUU-3', or a base sequence having at least 50% or more homology with 5'-GUUUUAGUCCCUUUUUAAAUUUCUU-3'.

In still another example, when the first complementary domain has partial or complete homology with a first complementary domain of Streptococcus thermophilus or a Streptococcus thermophilus-derived first complementary domain, the (Q)ₘ may be 5'-GUUUUAGAGCUGUGUUGUUUCG-3', or a base sequence having at least 50% or more homology with 5'-GUUUUAGAGCUGUGUUGUUUCG-3'.

In addition, each of the (X)ₐ, (X)_{b} and (X)_{c} is selectively an additional base sequence, where the X may be each independently selected from the group consisting of A, U, C and G, and each of the a, b and c may be the number of bases, which is 0 or an integer of 1 to 20.

In one exemplary embodiment, the second strand may be,
5'-(Z)ₕ-(P)ₖ-3';
or 5'-(X)_{d}-(Z)ₕ-(X)ₑ-(P)ₖ-(X)_{f}-3'.

In another embodiment, the second strand may be,
5'-(Z)ₕ-(P)ₖ-(F)ᵢ-3'; or
5'-(X)_{d}-(Z)ₕ-(X)ₑ-(P)ₖ-(X)_{f}-(F)ᵢ-3'.

Here, the (Z)ₕ is a base sequence including a second complementary domain, which is able to form a complementary bond with the first complementary domain of the first strand. The (Z)ₕ may be a sequence having partial or complete homology with the second complementary domain of a species existing in nature, and the base sequence of the second complementary domain may be modified according to the species of origin. The Z may be each independently selected from the group consisting of A, U, C and G, and the h may be the number of bases, which is an integer of 5 to 50.

For example, when the second complementary domain has partial or complete homology with a second complementary domain of Streptococcus pyogenes or a second complementary domain derived therefrom, the (Z)ₕ may be 5'-UAGCAAGUUAAAAU-3', or a base sequence having at least 50% or more homology with 5'-UAGCAAGUUAAAAU-3'.

In another example, when the second complementary domain has partial or complete homology with a second complementary domain of Campylobacter jejuni or a second complementary domain derived therefrom, the (Z)ₕ may be 5'-AAGAAAUUUAAAAAGGGACUAAAAU-3', or a base sequence having at least 50% or more homology with 5'-AAGAAAUUUAAAAAGGGACUAAAAU-3'.

In still another example, when the second complementary domain has partial or complete homology with a second complementary domain of Streptococcus thermophilus or a second complementary domain derived therefrom, the (Z)ₕ may be 5'-CGAAACAACACAGCGAGUUAAAAU-3', or a base sequence having at least 50% or more homology with 5'-CGAAACAACACAGCGAGUUAAAAU-3'.

The (P)ₖ is a base sequence including a proximal domain, which may have partial or complete homology with a proximal domain of a species existing in nature, and the base sequence of the proximal domain may be modified according to the species of origin. The P may be each independently selected from the group consisting of A, U, C and G, and the k may be the number of bases, which is an integer of 1 to 20.

For example, when the proximal domain has partial or complete homology with a proximal domain of Streptococcus pyogenes or a proximal domain derived therefrom, the (P)ₖ may be 5'-AAGGCUAGUCCG-3', or a base sequence having at least 50% or more homology with 5'-AAGGCUAGUCCG-3'.

In another example, when the proximal domain has partial or complete homology with a proximal domain of Campylobacter jejuni or a proximal domain derived therefrom, the (P)ₖ may be 5'-AAAGAGUUUGC-3', or a base sequence having at least 50% or more homology with 5'-AAAGAGUUUGC-3'.

In still another example, when the proximal domain has partial or complete homology with a proximal domain of Streptococcus thermophilus or a proximal domain derived therefrom, the (P)ₖ may be 5'-AAGGCUUAGUCCG-3', or a base sequence having at least 50% or more homology with 5'-AAGGCUUAGUCCG-3'.

The (F)ᵢ may be a base sequence including a tail domain, and having partial or complete homology with a tail domain of a species existing in nature, and the base sequence of the tail domain may be modified according to the species of origin. The F may be each independently selected from the group consisting of A, U, C and G, and the i may be the number of bases, which is an integer of 1 to 50.

For example, when the tail domain has partial or complete homology with a tail domain of Streptococcus pyogenes or a tail domain derived therefrom, the (F)ᵢ may be 5'-UUAUCAACUUGAAAAAGUGGCACCGAGUCGGUGC-3', or a base sequence having at least 50% or more homology with 5'-UUAUCAACUUGAAAAAGUGGCACCGAGUCGGUGC-3'.

In another example, when the tail domain has partial or complete homology with a tail domain of Campylobacter jejuni or a tail domain derived therefrom, the (F)ᵢ may be 5'-GGGACUCUGCGGGGUUACAAUCCCCUAAAACCGCUUUU-3', or a base sequence having at least 50% or more homology with 5'-GGGACUCUGCGGGGUUACAAUCCCCUAAAACCGCUUUU-3' .

In still another example, when the tail domain has partial or complete homology with a tail domain of Streptococcus thermophilus or a tail domain derived therefrom, the (F)ᵢ may be 5'-UACUCAACUUGAAAAGGUGGCACCGAUUCGGUGUUUUU-3', or a base sequence having at least 50% or more homology with 5'-UACUCAACUUGAAAAGGUGGCACCGAUUCGGUGUUUUU-3' .

In addition, the (F)ᵢ may include a sequence of 1 to 10 bases at the 3' end involved in an in vitro or in vivo transcription method.

For example, when a T7 promoter is used in in vitro transcription of gRNA, the tail domain may be an arbitrary base sequence present at the 3' end of a DNA template. In addition, when a U6 promoter is used in in vivo transcription, the tail domain may be UUUUUU, when an H1 promoter is used in transcription, the tail domain may be UUUU, and when a pol-III promoter is used, the tail domain may include several uracil bases or alternative bases.

In addition, the (X)_{d}, (X)ₑ and (X)_{f} may be base sequences selectively added, where the X may be each independently selected from the group consisting of A, U, C and G, and each of the d, e and f may be the number of bases, which is 0 or an integer of 1 to 20.

Single-stranded gRNA may be classified into two types: a first single-stranded gRNA, and a second single-stranded gRNA.

The first single-stranded gRNA is a single-stranded gRNA which a first strand or a second strand of the double-stranded gRNA is linked by a linker domain,

Specifically, the single-stranded gRNA may consist of:
5'-[guide domain]-[first complementary domain]-[linker domain]-[second complementary domain]-3' or
5'-[guide domain]-[first complementary domain]-[linker domain]-[second complementary domain]-[proximal domain]-3' or
5'-[guide domain]-[first complementary domain]-[linker domain]-[second complementary domain]-[proximal domain]-[tail domain]-3'.

The first single-stranded gRNA may selectively include an additional base sequence.

In one exemplary embodiment, the first single-stranded gRNA may be
5'-(N_{target})-(Q)ₘ-(L)ⱼ-(Z)ₕ-3';
5'-(N_{target})-(Q)ₘ-(L)ⱼ-(Z)ₕ-(P)ₖ-3'; or
5'-(N_{target})-(Q)ₘ-(L)ⱼ-(Z)ₕ-(P)ₖ-(F)ᵢ-3'.

In another embodiment, the single-stranded gRNA may be
5'-(X)ₐ-(N_{target})-(X)_{b}-(Q)ₘ-(X)_{c}-(L)_j-(X)_{d}-(Z)ₕ-(X)ₑ-3';
5'-(X)ₐ-(N_{target})-(X)_{b}-(Q)ₘ-(X)_c-(L)ⱼ-(X)_{d}-(Z)ₕ-(X)ₑ-(P)ₖ-(X)_{f}-3'; or
5'-(X)ₐ-(N_{target})-(X)_{b}-(Q)ₘ-(X)_{c}-(L)ⱼ-(X)_{d}-(Z)ₕ-(X)ₑ-(P)ₖ-(X)_{f}-(F)ᵢ-3'.

Here, the N_{target} is a base sequence capable of forming a complementary bond with a target sequence on a target gene or nucleic acid, and a base sequence region capable of being changed according to a target sequence on a target gene or nucleic acid.

The (Q)ₘ includes a base sequence including the first complementary domain, which is able to form a complementary bond with a second complementary domain. The (Q)ₘ may be a sequence having partial or complete homology with a first complementary domain of a species existing in nature, and the base sequence of the first complementary domain may be changed according to the species of origin. The Q may be each independently selected from the group consisting of A, U, C and G, and the m may be the number of bases, which is an integer of 5 to 35.

For example, when the first complementary domain has partial or complete homology with a first complementary domain of Streptococcus pyogenes or a first complementary domain derived therefrom, the (Q)ₘ may be 5'-GUUUUAGAGCUA-3', or a base sequence having at least 50% or more homology with 5'-GUUUUAGAGCUA-3' .

In another example, when the first complementary domain has partial or complete homology with a first complementary domain of Campylobacter jejuni or a first complementary domain derived therefrom, the (Q)ₘ may be 5'-GUUUUAGUCCCUUUUUAAAUUUCUU-3', or a base sequence having at least 50% or more homology with 5'-GUUUUAGUCCCUUUUUAAAUUUCUU-3'.

In still another example, when the first complementary domain has partial or complete homology with a first complementary domain of Streptococcus thermophilus or a first complementary domain derived therefrom, the (Q)ₘ may be 5'-GUUUUAGAGCUGUGUUGUUUCG-3', or a base sequence having at least 50% or more homology with 5'-GUUUUAGAGCUGUGUUGUUUCG-3'.

In addition, the (L)ⱼ is a base sequence including the linker domain, and connecting the first complementary domain with the second complementary domain, thereby producing single-stranded gRNA. Here, the L may be each independently selected from the group consisting of A, U, C and G, and the j may be the number of bases, which is an integer of 1 to 30.

The (Z)ₕ is a base sequence including the second complementary domain, which is able to have a complementary bond with the first complementary domain. The (Z)ₕ may be a sequence having partial or complete homology with the second complementary domain of a species existing in nature, and the base sequence of the second complementary domain may be changed according to the species of origin. The Z may be each independently selected from the group consisting of A, U, C and G, and the h is the number of bases, which may be an integer of 5 to 50.

For example, when the second complementary domain has partial or complete homology with a second complementary domain of Streptococcus pyogenes or a second complementary domain derived therefrom, the (Z)ₕ may be 5'-UAGCAAGUUAAAAU-3', or a base sequence having at least 50% or more homology with 5'-UAGCAAGUUAAAAU-3'.

In another example, when the second complementary domain has partial or complete homology with a second complementary domain of Campylobacter jejuni or a second complementary domain derived therefrom, the (Z)ₕ may be 5'-AAGAAAUUUAAAAAGGGACUAAAAU-3', or a base sequence having at least 50% or more homology with 5'-AAGAAAUUUAAAAAGGGACUAAAAU-3'.

In still another example, when the second complementary domain has partial or complete homology with a second complementary domain of Streptococcus thermophilus or a second complementary domain derived therefrom, the (Z)ₕ may be 5'-CGAAACAACACAGCGAGUUAAAAU-3', or a base sequence having at least 50% or more homology with 5'-CGAAACAACACAGCGAGUUAAAAU-3'.

The (P)ₖ is a base sequence including a proximal domain, which may have partial or complete homology with a proximal domain of a species existing in nature, and the base sequence of the proximal domain may be modified according to the species of origin. The P may be each independently selected from the group consisting of A, U, C and G, and the k may be the number of bases, which is an integer of 1 to 20.

For example, when the proximal domain has partial or complete homology with a proximal domain of Streptococcus pyogenes or a proximal domain derived therefrom, the (P)ₖ may be 5'-AAGGCUAGUCCG-3', or a base sequence having at least 50% or more homology with 5'-AAGGCUAGUCCG-3'.

In another example, when the proximal domain has partial or complete homology with a proximal domain of Campylobacter jejuni or a proximal domain derived therefrom, the (P)ₖ may be 5'-AAAGAGUUUGC-3', or a base sequence having at least 50% or more homology with 5'-AAAGAGUUUGC-3'.

In still another example, when the proximal domain has partial or complete homology with a proximal domain of Streptococcus thermophilus or a proximal domain derived therefrom, the (P)ₖ may be 5'-AAGGCUUAGUCCG-3', or a base sequence having at least 50% or more homology with 5'-AAGGCUUAGUCCG-3'.

The (F)ᵢ may be a base sequence including a tail domain, and having partial or complete homology with a tail domain of a species existing in nature, and the base sequence of the tail domain may be modified according to the species of origin. The F may be each independently selected from the group consisting of A, U, C and G, and the i may be the number of bases, which is an integer of 1 to 50.

For example, when the tail domain has partial or complete homology with a tail domain of Streptococcus pyogenes or a tail domain derived therefrom, the (F)ᵢ may be 5'-UUAUCAACUUGAAAAAGUGGCACCGAGUCGGUGC-3', or a base sequence having at least 50% or more homology with 5'-UUAUCAACUUGAAAAAGUGGCACCGAGUCGGUGC-3'

In another example, when the tail domain has partial or complete homology with a tail domain of Campylobacter jejuni or a tail domain derived therefrom, the (F)ᵢ may be 5'-GGGACUCUGCGGGGUUACAAUCCCCUAAAACCGCUUUU-3', or a base sequence having at least 50% or more homology with 5'-GGGACUCUGCGGGGUUACAAUCCCCUAAAACCGCUUUU-3' .

In still another example, when the tail domain has partial or complete homology with a tail domain of Streptococcus thermophilus or a tail domain derived therefrom, the (F)ᵢ may be 5'-UACUCAACUUGAAAAGGUGGCACCGAUUCGGUGUUUUU-3', or a base sequence having at least 50% or more homology with 5'-UACUCAACUUGAAAAGGUGGCACCGAUUCGGUGUUUUU-3' .

In addition, the (F)ᵢ may include a sequence of 1 to 10 bases at the 3' end involved in an in vitro or in vivo transcription method.

For example, when a T7 promoter is used in in vitro transcription of gRNA, the tail domain may be an arbitrary base sequence present at the 3' end of a DNA template. In addition, when a U6 promoter is used in in vivo transcription, the tail domain may be UUUUUU, when an H1 promoter is used in transcription, the tail domain may be UUUU, and when a pol-III promoter is used, the tail domain may include several uracil bases or alternative bases.

In addition, the (X)ₐ, (X)_{b}, (X)_{c}, (X)_{d}, (X)ₑ and (X)_{f} may be base sequences selectively added, where the X may be each independently selected from the group consisting of A, U, C and G, and each of the a, b, c, d, e and f may be the number of bases, which is 0 or an integer of 1 to 20.
the second single-stranded gRNA may be single-stranded gRNA consisting of a guide domain, a first complementary domain and a second complementary domain.

Here, the second single-stranded gRNA may consist of:
5'-[second complementary domain]-[first complementary domain]-[guide domain]-3'; or
5'-[second complementary domain]-[linker domain]-[first complementary domain]-[guide domain]-3'.

The second single-stranded gRNA may selectively include an additional base sequence.

In one exemplary embodiment, the single-stranded gRNA may be
5'-(Z)ₕ-(Q)ₘ-()-3'; or
5'-(X)ₐ-(Z)ₕ-(X)_{b}-(Q)ₘ-(X)_{c}-(N_{target})-3'.

In another embodiment, the single-stranded gRNA may be
5'--(L)_j--()-3'; or
5'-(X)ₐ-(Z)ₕ-(L)ⱼ-(Q)ₘ-(X)_{c}-(N_{target})-3'.

Here, the N_{target} is a base sequence capable of forming a complementary bond with a target sequence on a target gene or nucleic acid, and a base sequence region which may be changed according to a target sequence on a target gene or nucleic acid.

The (Q)ₘ is a base sequence including the first complementary domain, which is able to form a complementary bond with the second complementary domain of the second strand. The (Q)ₘ may be a sequence having partial or complete homology with the first complementary domain of a species existing in nature, and the base sequence of the first complementary domain may be changed according to the species of origin. The Q may be each independently selected from the group consisting of A, U, C and G, and the m may be the number of bases, which is an integer of 5 to 35.

For example, when the first complementary domain has partial or complete homology with a first complementary domain of Parcubacteria bacterium or a first complementary domain derived therefrom, the (Q)ₘ may be 5'-UUUGUAGAU-3', or a base sequence having at least 50% or more homology with 5'-UUUGUAGAU-3'.

The (Z)ₕ is a base sequence including a second complementary domain, which is able to form a complementary bond with the first complementary domain of the first strand. The (Z)ₕ may be a sequence having partial or complete homology with the second complementary domain of a species existing in nature, and the base sequence of the second complementary domain may be modified according to the species of origin. The Z may be each independently selected from the group consisting of A, U, C and G, and the h may be the number of bases, which is an integer of 5 to 50.

For example, when the second complementary domain has partial or complete homology with a second complementary domain of Parcubacteria bacterium or a Parcubacteria bacterium-derived second complementary domain, the (Z)ₕ may be 5'-AAAUUUCUACU-3', or a base sequence having at least 50% or more homology with 5' -AAAUUUCUACU-3'.

In addition, the (L)ⱼ is a base sequence including the linker domain, which connects the first complementary domain with the second complementary domain. Here, the L may be each independently selected from the group consisting of A, U, C and G, and the j may be the number of bases, which is an integer of 1 to 30.

In addition, each of the (X)ₐ, (X)_{b} and (X)_{c} is selectively an additional base sequence, where the X may be each independently selected from the group consisting of A, U, C and G, and the a, b and c may be the number of bases, which is 0 or an integer of 1 to 20.

An embodiment of the content disclosed by the present specification relates to a gRNA in which the guide nucleic acid is capable of complementarily binding with the target sequence of the expression regulatory gene of the over-amplified repeated sequence.

The expression regulatory gene of the over-amplified repeated sequence may be an SPT4 gene, SPT5 gene, SUPT4H gene, and/or SUPT5H gene, but is not limited thereto.

The guide nucleic acid may be a gRNA for a target sequence of the SPT4 gene, SPT5 gene, SUPT4H gene, and/or SUPT5H gene.

"The target sequence" is a nucleotide sequence of target gene or nucleic acid, specifically a partial nucleotide sequence of target region in the target gene or nucleic acid, wherein "the target region" is a region in the target gene or nucleic acid which can be modified by the guide nucleic acid-editor protein.

The target gene disclosed by the present application may be a repeat expansion expression regulatory gene.

The target gene disclosed by the present application may be an SPT4, an SPT5, an SUPT4H, and/or an SUPT5H gene.

Hereinafter, the target sequence may refer to two nucleotide sequence information. For example, in the case of the target gene, the target sequence may refer to the sequence information of transcribed strand of target gene's DNA, or may refer to the sequence information of non-transcribed strand of target gene's DNA.

For example, the target sequence may refer a nucleotide sequence of transcribed strand of the target gene A, which is 5'-ATCATTGGCAGACTAGTTCG-3', or a nucleotide sequence of non-transcribed strand of the target gene A, which is 5'-CGAACTAGTCTGCCAATGAT-3'.
The target sequence may be a 5 to 50-base sequence.

In an embodiment, the target sequence may be a 16, 17, 18, 19, 20, 21, 22, 23, 24 or 25-base sequence.

The target sequence includes a guide nucleic acid binding sequence or a guide nucleic acid non-binding sequence.

"The guide nucleic acid binding sequence" has partial or complete complementarity with the guide sequence contained in the guide domain, which is able to form complementary bond with the guide sequence contained in the guide domain. the target sequence and the guide nucleic acid binding sequence varies according to the target gene or nucleic acid, that is, a subject for gene manipulation or correction, which may be designed in various forms according to the target gene or nucleic acid.

"The guide nucleic acid non-binding sequence" has partial or complete homology with the guide sequence contained in the guide domain, which is not able to form complementary bond with the guide sequence contained in the guide domain. In addition, the guid nucleic acid non-binding sequence has complementarity with the guide nucleic acid binding sequence, which is able to form complementary bond with the guide nucleic acid binding sequence.

The guide nucleic acid binding sequence may be a nucleotide sequence, which is one of two different nucleotide sequences of the target sequence, that is, one sequence is able to form complemantary bond with the other. Here, the guide nucleic acid non-binding sequence may be the other nucleotide sequence of the target sequence which is different from the guide nucleic acid binding sequence.

For example, if the target sequence is a nucleotide sequence in a target region of the target gene A, which is 5'-ATCATTGGCAGACTAGTTCG-3' and 5'-CGAACTAGTCTGCCAATGAT-3'(complementary sequence), the guid nucleic acid binding sequence may be one of the two sequences, 5'-ATCATTGGCAGACTAGTTCG-3' or 5'-CGAACTAGTCTGCCAATGAT-3'. Here, the guid nucleic acid non-binding sequence may be 5'-CGAACTAGTCTGCCAATGAT-3' when the guid nucleic acid binding sequence is 5'-ATCATTGGCAGACTAGTTCG-3', or 5'-ATCATTGGCAGACTAGTTCG-3' when the guid nucleic acid binding sequence is 5'-CGAACTAGTCTGCCAATGAT-3'.

The guide nucleic acid binding sequence may be selected from one of the two nucleotide sequences of the target sequence: a nucleotide sequence which has the same sequence with transcribed strand; and a nucleotide sequence which has the same sequence with non-transcribed strand. Here, the guide nucleic acid non-binding sequence may be one of the other nucleotide sequence of the target sequences, different from the guide nucleic acid binding sequence.

A length of the guide nucleic acid binding sequence may be the same as that of the target sequence.

A length of the guide nucleic acid non-binding sequence may be the same as that of the target sequence or guide nucleic acid binding sequence.

The guide nucleic acid binding sequence may be a 5 to 50-base sequence.

In an embodiment, the guide nucleic acid binding sequence may be a 16, 17, 18, 19, 20, 21, 22, 23, 24 or 25-base sequence.

The guide nucleic acid non-binding sequence may be a 5 to 50-base sequence.

In an embodiment, the guide nucleic acid non-binding sequence may be a 16, 17, 18, 19, 20, 21, 22, 23, 24 or 25-base sequence.

The guide nucleic acid binding sequence is able to completely, or partially complementarily bind to a the guide sequence contained in the guide domain of the guide nucleic acid, and a lenth of the guide nucleic acid non-binding sequence may be the same as that of the target sequence or guide nucleic acid binding sequence.

The guide nucleic acid binding sequence may be a nucleic acid sequence complementary to the guide sequence contained in the guide domain of the guide nucleic acid, which has, for example, at least 70%, 75%, 80%, 85%, 90% or 95% or more complementarity or complete complementarity.

In one example, the guide nucleic acid binding sequence may be or include a 1 to 8-base sequence, which is not complementary to the guide sequence contained in the guide domain of the guide nucleic acid.

The guide nucleic acid non-binding sequence may have partial or complete homology, and a length of the guide nucleic acid non-binding sequence may be the same as that of the guide sequence.

The guide nucleic acid non-binding sequence may be a nucleic acid sequence homology to the guide sequence contained in the guide domain of the guide nucleic acid, which has, for example, at least 70%, 75%, 80%, 85%, 90% or 95% or more homology or complete homology.

In one example, the guide nucleic acid non-binding sequence may include an additional 1 to 8-base sequence which is not homologous with guide sequence contained in the guide domain.

Here, the guide nucleic acid non-binding sequence is capable of forming a complementary bond with the guide nucleic acid binding sequence, the guide nucleic acid binding sequence, and a length of the guide nucleic acid non-binding sequence may be the same as that of the guide nucleic acid binding sequence.

The guide nucleic acid non-binding sequence may be a nucleic acid sequence complementary to the guide nucleic acid binding sequence, which has, for example, at least 90% or 95% or more complementarity or complete complementarity.

In one example, the guide nucleic acid non-binding sequence may include 1 to 2-base sequence which is not complementary with the guide nucleic acid binding sequence.

In addition, the guide nucleic acid binding sequence may be a base sequence adjacent to a nucleic acid sequence that is able to be recognized by an editor protein.

In one example, the guide nucleic acid binding sequence may be a continuous 5 to 50-base sequence adjacent to the 5' end and/or 3' end of the nucleic acid sequence that is able to be recognized by the editor protein.

In addition, the guide nucleic acid non-binding sequence may be a base sequence adjacent to a nucleic acid sequence that is able to be recognized by an editor protein.

In one example, the guide nucleic acid non-binding sequence may be a continuous 5 to 50-base sequence adjacent to the 5' end and/or 3' end of the nucleic acid sequence that is able to be recognized by the editor protein.

In one embodiment,
as disclosed herein, a target sequence may be a continuous 10 to 35-base nucleotide sequence which is located at the promoter region of a repeat expansion expression regulatory gene.

Here, the target sequence may be a 5 to 35, 10 to 35, 15 to 35, 20 to 35, 25 to 35 or 30 to 35-base sequence.

Or, the target sequence may be a 10 to 15, 15 to 20, 20 to 25, 25 to 30 or 30 to 35-base sequence.

For example, the target sequence may be a continuous 10 to 25-base nucleic acid sequence which is located at the promoter region of an SPT4 gene

In another example, the target sequence may be a continuous 10 to 25-base nucleic acid sequence which is located at the promoter region of an SPT5 gene

For example, the target sequence may be a continuous 10 to 25-base nucleic acid sequence which is located at the promoter region of an SUPT4H gene

In another example, the target sequence may be a continuous 10 to 25-base nucleic acid sequence which is located at the promoter region of an SUPT5H gene

A target sequence disclosed by the present application may be a continuous 10 to 35-base nucleotide sequence which is located at the intron region of a repeat expansion expression regulatory gene.

Here, the target sequence may be a 5 to 35, 10 to 35, 15 to 35, 20 to 35, 25 to 35 or 30 to 35-base sequence.

Or, the target sequence may be a 10 to 15, 15 to 20, 20 to 25, 25 to 30 or 30 to 35-base sequence.

For example, a target sequence may be a continuous 10 to 25-base nucleotide sequence which is located at the intron region of an SPT4.

For example, a target sequence may be a continuous 10 to 25-base nucleotide sequence which is located at the intron region of an SPT5.

For example, a target sequence may be a continuous 10 to 25-base nucleotide sequence which is located at the intron region of an SUPT4H.

For example, a target sequence may be a continuous 10 to 25-base nucleotide sequence which is located at the intron region of an SUPT5H.

A target sequence disclosed by the present application may be a continuous 10 to 35-base nucleotide sequence which is located at the exon region of a repeat expansion expression regulatory gene.

Here, the target sequence may be a 5 to 35, 10 to 35, 15 to 35, 20 to 35, 25 to 35 or 30 to 35-base sequence.

Or, the target sequence may be a 10 to 15, 15 to 20, 20 to 25, 25 to 30 or 30 to 35-base sequence.

For example, a target sequence may be a continuous 10 to 25-base nucleotide sequence which is located at the exon region of an SPT4 gene.

For example, a target sequence may be a continuous 10 to 25-base nucleotide sequence which is located at the exon region of an SPT5 gene.

For example, a target sequence may be a continuous 10 to 25-base nucleotide sequence which is located at the exon region of an SUPT4H gene.

For example, a target sequence may be a continuous 10 to 25-base nucleotide sequence which is located at the exon region of an SUPT5H gene.

A target sequence disclosed by the present application may be a continuous 10 to 35-base nucleotide sequence which is located at the enhancer region of a repeat expansion expression regulatory gene.

Here, the target sequence may be a 5 to 35, 10 to 35, 15 to 35, 20 to 35, 25 to 35 or 30 to 35-base sequence.

Or, the target sequence may be a 10 to 15, 15 to 20, 20 to 25, 25 to 30 or 30 to 35-base sequence.

For example, a target sequence may be a continuous 10 to 25-base nucleotide sequence which is located at the enhancer region of an SPT4.

For example, a target sequence may be a continuous 10 to 25-base nucleotide sequence which is located at the enhancer region of an SPT5.

For example, a target sequence may be a continuous 10 to 25-base nucleotide sequence which is located at the enhancer region of an SUPT4H.

For example, a target sequence may be a continuous 10 to 25-base nucleotide sequence which is located at the enhancer region of an SUPT5H.

A target sequence disclosed by the present application may be a continuous 10 to 35-base nucleotide sequence which is located at the coding or non-coding region or a mixture thereof of a repeat expansion expression regulatory gene.

Here, the target sequence may be a 5 to 35, 10 to 35, 15 to 35, 20 to 35, 25 to 35 or 30 to 35-base sequence.

Or, the target sequence may be a 10 to 15, 15 to 20, 20 to 25, 25 to 30 or 30 to 35-base sequence.

For example, a target sequence may be a continuous 10 to 25-base nucleotide sequence which is located at the coding or non-coding region or a mixture thereof of an SPT4 gene.

For example, a target sequence may be a continuous 10 to 25-base nucleotide sequence which is located at the coding or non-coding region or a mixture thereof of an SPT5 gene.

For example, a target sequence may be a continuous 10 to 25-base nucleotide sequence which is located at the coding or non-coding region or a mixture thereof of an SUPT4H gene.

For example, a target sequence may be a continuous 10 to 25-base nucleotide sequence which is located at the coding or non-coding region or a mixture thereof of an SUPT5H gene.

A target sequence disclosed by the present application may be a continuous 10 to 35-base nucleotide sequence which is located at the promoter, enhancer, 3'UTR or polyadenyl (polyA) region or a mixture thereof of a repeat expansion expression regulatory gene.

Here, the target sequence may be a 5 to 35, 10 to 35, 15 to 35, 20 to 35, 25 to 35 or 30 to 35-base sequence.

Or, the target sequence may be a 10 to 15, 15 to 20, 20 to 25, 25 to 30 or 30 to 35-base sequence.

For example, a target sequence may be a continuous 10 to 25-base nucleotide sequence which is located at the promoter, enhancer, 3'UTR or polyadenyl (polyA) region or a mixture thereof of an SPT4 gene.

For example, a target sequence may be a continuous 10 to 25-base nucleotide sequence which is located at the promoter, enhancer, 3'UTR or polyadenyl (polyA) region or a mixture thereof of an SPT5 gene.

For example, a target sequence may be a continuous 10 to 25-base nucleotide sequence which is located at the promoter, enhancer, 3'UTR or polyadenyl (polyA) region or a mixture thereof of an SUPT4H gene.

For example, a target sequence may be a continuous 10 to 25-base nucleotide sequence which is located at the promoter, enhancer, 3'UTR or polyadenyl (polyA) region or a mixture thereof of an SUPT5H gene.

A target sequence disclosed by the present application may be a continuous 10 to 35-base nucleotide sequence which is located at the exon or intron region or a mixture thereof of a repeat expansion expression regulatory gene.

Here, the target sequence may be a 5 to 35, 10 to 35, 15 to 35, 20 to 35, 25 to 35 or 30 to 35-base sequence.

Or, the target sequence may be a 10 to 15, 15 to 20, 20 to 25, 25 to 30 or 30 to 35-base sequence.

For example, a target sequence may be a continuous 10 to 25-base nucleotide sequence which is located at the exon or intron region or a mixture thereof of an SPT4 gene.

For example, a target sequence may be a continuous 10 to 25-base nucleotide sequence which is located at the exon or intron region or a mixture thereof of an SPT5 gene.

For example, a target sequence may be a continuous 10 to 25-base nucleotide sequence which is located at the exon or intron region or a mixture thereof of an SUPT4H gene.

For example, a target sequence may be a continuous 10 to 25-base nucleotide sequence which is located at the exon or intron region or a mixture thereof of an SUPT5H gene.

A target sequence disclosed by the present application may be a continuous 10 to 35-base nucleotide sequence which includes or adjacent to a repeat expansion expression regulatory gene.

Here, the target sequence may be a 5 to 35, 10 to 35, 15 to 35, 20 to 35, 25 to 35 or 30 to 35-base sequence.

Or, the target sequence may be a 10 to 15, 15 to 20, 20 to 25, 25 to 30 or 30 to 35-base sequence.

For example, a target sequence may be a continuous 10 to 25-base nucleotide sequence which includes or adjacent to an SPT4 gene.

For example, a target sequence may be a continuous 10 to 25-base nucleotide sequence which includes or adjacent to an SPT5 gene.

For example, a target sequence may be a continuous 10 to 25-base nucleotide sequence which includes or adjacent to an SUPT4H gene.

For example, a target sequence may be a continuous 10 to 25-base nucleotide sequence which includes or adjacent to an SUPT5H gene.

A target sequence disclosed by the present application may be a continuous 5 to 35-base nucleotide sequence adjacent to the 5' end and/or 3' end of the proto-spacer-adjacent motif(PAM) sequence in an nucleotide sequence of a repeat expansion expression regulatory gene.

"The PAM sequence" is a nucleotide sequence which may be recognized by the editor protein. Here, the PAM sequence may vary according to the origin of the species or type of the editor protein.
the PAM sequence is, one or more of the following sequences (described in the 5' to 3' direction).
NGG (N is A, T, C or G);
NNNNRYAC (each N is independently A, T, C or G, R is A or G, and Y is C or T);
NNAGAAW (each N is independently A, T, C or G, and W is A or T);
NNNNGATT (each N is independently A, T, C or G);
NNGRR(T) (each N is independently A, T, C or G, R is A or G); and
TTN (N is A, T, C or G).

Here, the guide domain may be a sequence of 10 to 35, 15 to 35, 20 to 35, 25 to 35, 30 to 35 bases.

Or, the guide domain may be a sequence of 10 to 15, 15 to 20, 20 to 25, 25 to 30, 30 to 35 bases.

For example, a target sequence may be a continuous 5 to 25-base nucleotide sequence adj acent to the 5' end and/or 3' end of the proto-spacer-adj acent motif(PAM) sequence in an nucleotide sequence of SPT4 gene.

In one examplary embodiment, if the PAM sequence recognized by the editor protein is 5'-NGG-3', 5'-NAG-3', and/or 5'-NGA-3' (N is A, T, C or C; or A, U, G or C), the target sequence may be a continuous 5 to 25-base nucleotide sequence adjacent to the 5' end and/or 3' end of 5'-NGG-3', 5'-NAG-3', and/or 5'-NGA-3' (N is A, T, C or C; or A, U, G or C) in an nucleotide sequence of an SPT4 gene.

In another examplary embodiment, if the PAM sequence recognized by the editor protein is 5'-NGGNG-3' and/or 5'-NNAGAAW-3' (W is A or T, N is A, T, G or C; or A, U, G or C), the target sequence may be a continuous 5 to 25-base nucleotide sequence adjacent to the 5' end and/or 3' end of 5'-NGGNG-3' and/or 5'-NNAGAAW-3' (W is A or T, N is A, T, G or C; or A, U, G or C) in an nucleotide sequence of an SPT4 gene.

In yet another examplary embodiment, if the PAM sequence recognized by the editor protein is 5'-NNNNGATT-3' and/or 5'-NNNGCTT-3' (N is A, T, G or C; or A, U, G or C), the target sequence may be a continuous 5 to 25-base nucleotide sequence adjacent to the 5' end and/or 3' end of 5'-NNNNGATT-3' and/or 5'-NNNGCTT-3' (N is A, T, G or C; or A, U, G or C) in an nucleotide sequence of an SPT4 gene.

In one examplary embodiment, if the PAM sequence recognized by the editor protein is 5'-NNNVRYAC-3' (V is G, C or A; R is A or G, Y is C or T, N is A, T, G or C; or A, U, G or C), the target sequence may be a continuous 5 to 25-base nucleotide sequence adjacent to the 5' end and/or 3' end of 5'-NNNVRYAC-3' (V is G, C or A; R is A or G, Y is C or T, N is A, T, G or C; or A, U, G or C) in an nucleotide sequence of an SPT4 gene.

In another examplary embodiment, if the PAM sequence recognized by the editor protein is 5'-NAAR-3'(R is A or G, N is A, T, G or C; or A, U, G or C), the target sequence may be a continuous 5 to 25-base nucleotide sequence adjacent to the 5' end and/or 3' end of 5'-NAAR-3'(R is A or G, N is A, T, G or C; or A, U, G or C) in an nucleotide sequence of an SPT4 gene.

In yet another examplary embodiment, if the PAM sequence recognized by the editor protein is 5'-NNGRR-3', 5'-NNGRRT-3' and/or 5'-NNGRRV-3' (R is A or G, V is G, C or A, N is A, T, G or C; or A, U, G or C), the target sequence may be a continuous 5 to 25-base nucleotide sequence adjacent to the 5' end and/or 3' end of 5'-NNGRR-3', 5'-NNGRRT-3' and/or 5'-NNGRRV-3' (R is A or G, V is G, C or A, N is A, T, G or C; or A, U, G or C) in an nucleotide sequence of an SPT4 gene.

In one examplary embodiment, if the PAM sequence recognized by the editor protein is 5'-TTN-3' (N is A, T, G or C; or A, U, G or C), the target sequence may be a continuous 5 to 25-base nucleotide sequence adjacent to the 5' end and/or 3' end of 5'-TTN-3' (N is A, T, G or C; or A, U, G or C) in an nucleotide sequence of an SPT4 gene.

For example, a target sequence may be a continuous 5 to 25-base nucleotide sequence adj acent to the 5' end and/or 3' end of the proto-spacer-adj acent motif(PAM) sequence in an nucleotide sequence of SPT5 gene.

In one examplary embodiment, if the PAM sequence recognized by the editor protein is 5'-NGG-3', 5'-NAG-3', and/or 5'-NGA-3' (N is A, T, C or C; or A, U, G or C), the target sequence may be a continuous 5 to 25-base nucleotide sequence adjacent to the 5' end and/or 3' end of 5'-NGG-3', 5'-NAG-3', and/or 5'-NGA-3' (N is A, T, C or C; or A, U, G or C) in an nucleotide sequence of an SPT5 gene.

In another examplary embodiment, if the PAM sequence recognized by the editor protein is 5'-NGGNG-3' and/or 5'-NNAGAAW-3' (W is A or T, N is A, T, G or C; or A, U, G or C), the target sequence may be a continuous 5 to 25-base nucleotide sequence adjacent to the 5' end and/or 3' end of 5'-NGGNG-3' and/or 5'-NNAGAAW-3' (W is A or T, N is A, T, G or C; or A, U, G or C) in an nucleotide sequence of an SPT5 gene.

In yet another examplary embodiment, if the PAM sequence recognized by the editor protein is 5'-NNNNGATT-3' and/or 5'-NNNGCTT-3' (N is A, T, G or C; or A, U, G or C), the target sequence may be a continuous 5 to 25-base nucleotide sequence adjacent to the 5' end and/or 3' end of 5'-NNNNGATT-3' and/or 5'-NNNGCTT-3' (N is A, T, G or C; or A, U, G or C) in an nucleotide sequence of an SPT5 gene.

In one examplary embodiment, if the PAM sequence recognized by the editor protein is 5'-NNNVRYAC-3' (V is G, C or A; R is A or G, Y is C or T, N is A, T, G or C; or A, U, G or C), the target sequence may be a continuous 5 to 25-base nucleotide sequence adjacent to the 5' end and/or 3' end of 5'-NNNVRYAC-3' (V is G, C or A; R is A or G, Y is C or T, N is A, T, G or C; or A, U, G or C) in an nucleotide sequence of an SPT5 gene.

In another examplary embodiment, if the PAM sequence recognized by the editor protein is 5'-NAAR-3'(R is A or G, N is A, T, G or C; or A, U, G or C), the target sequence may be a continuous 5 to 25-base nucleotide sequence adjacent to the 5' end and/or 3' end of 5'-NAAR-3'(R is A or G, N is A, T, G or C; or A, U, G or C) in an nucleotide sequence of an SPT5 gene.

In yet another examplary embodiment, if the PAM sequence recognized by the editor protein is 5'-NNGRR-3', 5'-NNGRRT-3' and/or 5'-NNGRRV-3' (R is A or G, V is G, C or A, N is A, T, G or C; or A, U, G or C), the target sequence may be a continuous 5 to 25-base nucleotide sequence adjacent to the 5' end and/or 3' end of 5'-NNGRR-3', 5'-NNGRRT-3' and/or 5'-NNGRRV-3' (R is A or G, V is G, C or A, N is A, T, G or C; or A, U, G or C) in an nucleotide sequence of an SPT5 gene.

In one examplary embodiment, if the PAM sequence recognized by the editor protein is 5'-TTN-3' (N is A, T, G or C; or A, U, G or C), the target sequence may be a continuous 5 to 25-base nucleotide sequence adjacent to the 5' end and/or 3' end of 5'-TTN-3' (N is A, T, G or C; or A, U, G or C) in an nucleotide sequence of an SPT5 gene.

For example, a target sequence may be a continuous 5 to 25-base nucleotide sequence adj acent to the 5' end and/or 3' end of the proto-spacer-adj acent motif(PAM) sequence in an nucleotide sequence of an SUPT4H gene.

In one examplary embodiment, if the PAM sequence recognized by the editor protein is 5'-NGG-3', 5'-NAG-3', and/or 5'-NGA-3' (N is A, T, C or C; or A, U, G or C), the target sequence may be a continuous 5 to 25-base nucleotide sequence adjacent to the 5' end and/or 3' end of 5'-NGG-3', 5'-NAG-3', and/or 5'-NGA-3' (N is A, T, C or C; or A, U, G or C) in an nucleotide sequence of an SUPT4H gene.

In another examplary embodiment, if the PAM sequence recognized by the editor protein is 5'-NGGNG-3' and/or 5'-NNAGAAW-3' (W is A or T, N is A, T, G or C; or A, U, G or C), the target sequence may be a continuous 5 to 25-base nucleotide sequence adjacent to the 5' end and/or 3' end of 5'-NGGNG-3' and/or 5'-NNAGAAW-3' (W is A or T, N is A, T, G or C; or A, U, G or C) in an nucleotide sequence of an SUPT4H gene.

In yet another examplary embodiment, if the PAM sequence recognized by the editor protein is 5'-NNNNGATT-3' and/or 5'-NNNGCTT-3' (N is A, T, G or C; or A, U, G or C), the target sequence may be a continuous 5 to 25-base nucleotide sequence adjacent to the 5' end and/or 3' end of 5'-NNNNGATT-3' and/or 5'-NNNGCTT-3' (N is A, T, G or C; or A, U, G or C) in an nucleotide sequence of an SUPT4H gene.

In one examplary embodiment, if the PAM sequence recognized by the editor protein is 5'-NNNVRYAC-3' (V is G, C or A; R is A or G, Y is C or T, N is A, T, G or C; or A, U, G or C), the target sequence may be a continuous 5 to 25-base nucleotide sequence adjacent to the 5' end and/or 3' end of 5'-NNNVRYAC-3' (V is G, C or A; R is A or G, Y is C or T, N is A, T, G or C; or A, U, G or C) in an nucleotide sequence of an SUPT4H gene.

In another examplary embodiment, if the PAM sequence recognized by the editor protein is 5'-NAAR-3'(R is A or G, N is A, T, G or C; or A, U, G or C), the target sequence may be a continuous 5 to 25-base nucleotide sequence adjacent to the 5' end and/or 3' end of 5'-NAAR-3'(R is A or G, N is A, T, G or C; or A, U, G or C) in an nucleotide sequence of an SUPT4H gene.

In yet another examplary embodiment, if the PAM sequence recognized by the editor protein is 5'-NNGRR-3', 5'-NNGRRT-3' and/or 5'-NNGRRV-3' (R is A or G, V is G, C or A, N is A, T, G or C; or A, U, G or C), the target sequence may be a continuous 5 to 25-base nucleotide sequence adjacent to the 5' end and/or 3' end of 5'-NNGRR-3', 5'-NNGRRT-3' and/or 5'-NNGRRV-3' (R is A or G, V is G, C or A, N is A, T, G or C; or A, U, G or C) in an nucleotide sequence of an SUPT4H gene.

In one examplary embodiment, if the PAM sequence recognized by the editor protein is 5'-TTN-3' (N is A, T, G or C; or A, U, G or C), the target sequence may be a continuous 5 to 25-base nucleotide sequence adjacent to the 5' end and/or 3' end of 5'-TTN-3' (N is A, T, G or C; or A, U, G or C) in an nucleotide sequence of an SUPT4H gene.

For example, a target sequence may be a continuous 5 to 25-base nucleotide sequence adj acent to the 5' end and/or 3' end of the proto-spacer-adj acent motif(PAM) sequence in an nucleotide sequence of an SUPT5H gene.

In one examplary embodiment, if the PAM sequence recognized by the editor protein is 5'-NGG-3', 5'-NAG-3', and/or 5'-NGA-3' (N is A, T, C or C; or A, U, G or C), the target sequence may be a continuous 5 to 25-base nucleotide sequence adjacent to the 5' end and/or 3' end of 5'-NGG-3', 5'-NAG-3', and/or 5'-NGA-3' (N is A, T, C or C; or A, U, G or C) in an nucleotide sequence of an SUPT5H gene.

In another examplary embodiment, if the PAM sequence recognized by the editor protein is 5'-NGGNG-3' and/or 5'-NNAGAAW-3' (W is A or T, N is A, T, G or C; or A, U, G or C), the target sequence may be a continuous 5 to 25-base nucleotide sequence adjacent to the 5' end and/or 3' end of 5'-NGGNG-3' and/or 5'-NNAGAAW-3' (W is A or T, N is A, T, G or C; or A, U, G or C) in an nucleotide sequence of an SUPT5H gene.

In yet another examplary embodiment, if the PAM sequence recognized by the editor protein is 5'-NNNNGATT-3' and/or 5'-NNNGCTT-3' (N is A, T, G or C; or A, U, G or C), the target sequence may be a continuous 5 to 25-base nucleotide sequence adjacent to the 5' end and/or 3' end of 5'-NNNNGATT-3' and/or 5'-NNNGCTT-3' (N is A, T, G or C; or A, U, G or C) in an nucleotide sequence of an SUPT5H gene.

In one examplary embodiment, if the PAM sequence recognized by the editor protein is 5'-NNNVRYAC-3' (V is G, C or A; R is A or G, Y is C or T, N is A, T, G or C; or A, U, G or C), the target sequence may be a continuous 5 to 25-base nucleotide sequence adjacent to the 5' end and/or 3' end of 5'-NNNVRYAC-3' (V is G, C or A; R is A or G, Y is C or T, N is A, T, G or C; or A, U, G or C) in an nucleotide sequence of an SUPT5H gene.

In another examplary embodiment, if the PAM sequence recognized by the editor protein is 5'-NAAR-3'(R is A or G, N is A, T, G or C; or A, U, G or C), the target sequence may be a continuous 5 to 25-base nucleotide sequence adjacent to the 5' end and/or 3' end of 5'-NAAR-3'(R is A or G, N is A, T, G or C; or A, U, G or C) in an nucleotide sequence of an SUPT5H gene.

In yet another examplary embodiment, if the PAM sequence recognized by the editor protein is 5'-NNGRR-3', 5'-NNGRRT-3' and/or 5'-NNGRRV-3' (R is A or G, V is G, C or A, N is A, T, G or C; or A, U, G or C), the target sequence may be a continuous 5 to 25-base nucleotide sequence adjacent to the 5' end and/or 3' end of 5'-NNGRR-3', 5'-NNGRRT-3' and/or 5'-NNGRRV-3' (R is A or G, V is G, C or A, N is A, T, G or C; or A, U, G or C) in an nucleotide sequence of an SUPT5H gene.

In one examplary embodiment, if the PAM sequence recognized by the editor protein is 5'-TTN-3' (N is A, T, G or C; or A, U, G or C), the target sequence may be a continuous 5 to 25-base nucleotide sequence adjacent to the 5' end and/or 3' end of 5'-TTN-3' (N is A, T, G or C; or A, U, G or C) in an nucleotide sequence of an SUPT5H gene.

Hereinafter, examples of target sequences which are able to be used in embodiments of the present invention are shown in the Table 2, the target sequences listed in Table 2 are guide nucleic acid non-binding sequences, complementary sequences, which is, guide nucleic acid binding sequences can be expected from the sequences described in Table 2.

**[Table 2]**

| Target sequence of an expression regulatory gene of an over-amplified repeated sequence. | | |
|---|---|---|
| | #RGEN Target (5' to 3') | SEQ ID No. |
| Sp-hSUPT4H1 sgRNA1 | CGCAGATGCCGCAGGTCCTTC GG | SEQ ID 1 |
| Sp-hSUPT4H1 sgRNA2 | AATGAAGGGTAACCGAGAGA TGG | SEQ ID 2 |
| CjGX22-hSUPT4H1 sgRNA1 | AACTGGTCTATAGTCTTGACC AGCGAACAC | SEQ ID 3 |
| CjGX22-hSUPT4H1 sgRNA2 | CAGGCGACCAGTGACTGACA CCGCATATAC | SEQ ID 4 |
| CjGX22-hSUPT4H1 sgRNA3 | GGCCCGCAGATGCCGCAGGT CCTTCGGCAC | SEQ ID 5 |
| CjGX22-hSUPT4H1 sgRNA4 | GGGTAACCGAGAGATGGTAT ATGACTGCAC | SEQ ID 6 |
| CjGX22-hSUPT4H1 sgRNA5 | GGCGACCAGTGACTGACACC GCATATACAC | SEQ ID 7 |
| Sp-mSupt4a sgRNA1 | CTAGCTCTCAAGCTGAGCAGG GG | SEQ ID 8 |
| Sp-mSupt4a sgRNA2 | GAGCAGGGGACACAAGTGTC GGG | SEQ ID 9 |
| Sp-mSupt4a sgRNA3 | CGAGTCAGTAACTTTAAGCCA GG | SEQ ID 10 |
| CjGX22-mSupt4a sgRNA1 | GAGAGCTAGGACTGAAGGCT GCATCAACAC | SEQ ID 11 |
| CjGX22-mSupt4a sgRNA2 | CTCATGATGCAGCAGAAGGCT ACCCGACAC | SEQ ID 12 |
| CjGX22-mSupt4a sgRNA3 | GTCCTAGCTCTCAAGCTGAGC AGGGGACAC | SEQ ID 13 |
| Supt4a-FVB-Ex1-Sp1 | TTTGCTGTGCTCGTTAGTCAA GG | SEQ ID 14 |
| Supt4a-FVB-Ex 1-Sp2 | TCGTTAGTCAAGGTGTCCGTT GG | SEQ ID 15 |
| Supt4a-FVB-Ex 1-Sp3 | CGTTAGTCAAGGTGTCCGTTG GG | SEQ ID 16 |
| Supt4a-FVB-Ex 1-Sp4 | GTTAGTCAAGGTGTCCGTTGG GG | SEQ ID 17 |
| Supt4a-FVB-Ex2-Sp 1 | ATTCGAACTGGTCTATAGTCT GG | SEQ ID 18 |
| Supt4a-FVB-Ex2-Sp2 | GCACCAGCTCTTCATTTGATG GG | SEQ ID 19 |
| Supt4a-FVB-Ex2-Sp3 | TTACCCATCAAATGAAGAGCT GG | SEQ ID 20 |
| FVB-Supt4a-Ex1-Cj 1-F | TACCGTCTCCAGGGCCATCCT CACCGATAC | SEQ ID 21 |
| FVB-Supt4a-Ex1-Cj2-F | GCCCCAACGGACACCTTGACT AACGAGCAC | SEQ ID 22 |
| FVB-Supt4a-Ex1-Cj3-F | GTACCGTCTCCAGGGCCATCC TCACCGATA | SEQ ID 23 |
| SUPT4a-FVB-Ex3-Cj1F | CTCCCCAAAGCAACAGCCCG AGCAAGGCAC | SEQ ID 24 |

In yet another embodiment as disclosed herin is a composition for gene manipulation which includes an editor protein, wherein the composition may act as a gene scissors for the target sequence of one or more genes selected from the group consisting of SPT4, SPT5, SUPT4H, and SUPT5H gene.

The composition for gene manipulation may be used to produce a repeat expansion expression regulatory gene.

A repeat expansion expression regulatory gene which is manipulated by the composition for gene manipulation may construct a system for regulating an expression of repeat expansion.

The term "artificially modified, engineered, or artificially engineered" means an artificially modified state, which is not a naturally occurring state. Hereinafter, the term unnaturally, artificially modified or engineered repeat expansion expression regulatory gene may be used interchangeably with the term artificial repeat expansion expression regulatory gene.

"Regulation of the expression of an over-amplified repeated sequence" is a term that includes all phenomena involved in the mechanisms that affect disease expression by altering the function of an artificially manipulated over-amplified repeated sequence expression regulator gene, and includes all substances, compositions, methods and uses that are directly or indirectly involved in such an over-amplified repeated sequence expression system. For example, the term includes both a gene involved in transcription, post-transcriptional modification, translation, or post-translational modification of an over-amplified repeated sequence and a cell and organ/tissue including the gene.

A composition for gene manipulation disclosed by the present application may include a guide nucleic acid and an editor protein.

A composition for gene manipulation may comprise:
(a) a guide nucleic acid capable of forming complementary bonds with respect to the target sequence of a repeat expansion expression regulatory gene or nucleic acid sequence encoding the same; and
(b) one or more editor proteins and nucleic acid sequence encoding the same.

The repeat expansion expression regulatory gene is the same as described above.

The target sequence is the same as described above.

The composition for gene manipulation may include guide nucleic acid-editor protein complex.

The term "guide nucleic acid-editor protein complex" refers to a complex formed through the interaction between a guide nucleic acid and an editor protein.

The guide nucleic acid is the same as described above.

The term "editor protein" refers to a peptide, polypeptide or protein which is able to directly bind to or interact with, without direct binding to, a nucleic acid.

The nucleic acid may be a nucleic acid contained in a target nucleic acid, gene or chromosome.

The nucleic acid may be a guide nucleic acid.

The editor protein may be an enzyme.

The enzyme refers to a protein including a domain which is able to cleave a nucleic acid, gene, chromosome or protein.

The enzyme may be a nuclease or restriction enzyme.

The editor protein may include a complete active enzyme.

Here, the "complete active enzyme" refers to an enzyme having the same function as a function of a wild-type enzyme, and for example, the wild-type enzyme cleaving the double strand of DNA has complete enzyme activity of entirely cleaving the double strand of DNA.

In addition, the complete active enzyme includes an enzyme having an improved function compares to the function of the wild-type enzyme, and for example, a specific modified or manipulated type of the wild-type enzyme cleaving the double strand of DNA has full enzyme activity which is improved compares to the wild-type enzyme, that is, activity of cleaving the double strand of DNA.

The editor protein may include an incomplete or partially active enzyme.

The editor protein may include an incompletely or partially acitve enzyme.

Here, the "incompletely or partially active enzyme" refers to an enzyme having some of the functions of the wild-type enzyme. For example, a specificcally modified or manipulated type of the wild-type enzyme cleaving the double strand of DNA is an enzyme with a first function, or an enzyme with a second function. Here, the first function may be cleaving a first single strand of the double-stranded DNA, the second function may be cleaving a second single strand of the double-stranded DNA. Here, the enzyme which has the first function or the second function may be the incompletely or partially active enzyme.

The editor protein may include inactive enzyme.

Here, the "inactive enzyme" refers to an enzyme in which the function of a wild-type enzyme is completely inactivated. For example, a specifically modified or manipulated type of the wild-type enzyme cleaving the double strand of DNA is an enzyme which loses both the first and second functions. Here, the enzyme which loses both the first and second functions may be the inactive enzyme.

The editor protein may be a fusion protein.

Here, the "fusion protein" refers to a protein that is produced by fusing an enzyme with an additional domain, peptide, polypeptide or protein.

The additional domain, peptide, polypeptide or protein may be a functional domain, peptide, polypeptide or protein, which has a function the same as or different from the enzyme.

The fusion protein may include a functional domain, peptide, polypeptide or protein at one or more regions of the N-terminus of the enzyme or the vicinity thereof; the C-terminus or the vicinity thereof; the middle part of the enzyme; and a combination thereof.

Here, the functional domain, peptide, polypeptide or protein may be a domain, peptide, polypeptide or protein having methylase activity, demethylase activity, transcription activation activity, transcription repression activity, transcription release factor activity, histone modification activity, RNA cleavage activity or nucleic acid binding activity, or a tag or reporter gene for isolation and purification of a protein (including a peptide), but the present invention is not limited thereto.

The functional domain, peptide, polypeptide or protein may be a deaminase.

The tag includes a histidine (His) tag, a V5 tag, a FLAG tag, an influenza hemagglutinin (HA) tag, a Myc tag, a VSV-G tag and a thioredoxin (Trx) tag, and the reporter gene includes glutathione-S-transferase (GST), horseradish peroxidase (HRP), chloramphenicol acetyltransferase (CAT) β-galactosidase, β-glucoronidase, luciferase, autofluorescent proteins including the green fluorescent protein (GFP), HcRed, DsRed, cyan fluorescent protein (CFP), yellow fluorescent protein (YFP) and blue fluorescent protein (BFP), but the present invention is not limited thereto.

In addition, the functional domain, peptide, polypeptide or protein may be a nuclear localization sequence or signal (NLS) or a nuclear export sequence or signal (NES).

The NLS may be NLS of SV40 virus large T-antigen with an amino acid sequence PKKKRKV; NLS derived from nucleoplasmin (e.g., nucleoplasmin bipartite NLS with a sequence KRPAATKKAGQAKKKK); c-myc NLS with an amino acid sequence PAAKRVKLD or RQRRNELKRSP; hRNPA1 M9 NLS with a sequence NQSSNFGPMKGGNFGGRSSGPYGGGGQYFAKPRNQGGY; an importin-α-derived IBB domain sequence RMRIZFKNKGKDTAELRRRRVEVSVELRKAKKDEQILKRRNV; myoma T protein sequences VSRKRPRP and PPKKARED; human p53 sequence POPKKKPL; a mouse c-abl IV sequence SALIKKKKKMAP; influenza virus NS1 sequences DRLRR and PKQKKRK; a hepatitis virus-δ antigen sequence RKLKKKIKKL; a mouse Mx1 protein sequence REKKKFLKRR; a human poly(ADP-ribose) polymerase sequence KRKGDEVDGVDEVAKKKSKK; or steroid hormone receptor (human) glucocorticoid sequence RKCLQAGMNLEARKTKK, but the present invention is not limited thereto.

The additional domain, peptide, polypeptide, or protein may be a nonfunctional domain, peptide, polypeptide, or protein. Here, the nonfunctional domiain, peptide, polypeptide, or protein does not affact the function of the enzyme.

The fusion protein may include an nonfunctional domain, peptide, polypeptide or protein at one or more regions of the amino terminus (N-terminus) of the enzyme or the vicinity thereof; the carboxyl terminus (C-terminus) or the vicinity thereof; the middle part of the enzyme; and a combination thereof.

The editor protein may be a natural enzyme or fusion protein.

The editor protein may be present in the form of a partially modified natural enzyme or fusion protein.

The editor protein may be an artificially produced enzyme or fusion protein, which does not exist in nature.

The editor protein may be present in the form of a partially modified artificial enzyme or fusion protein, which does not exist in nature.

Here, the modification may be substitution, removal, addition of amino acids contained in the editor protein, or a combination thereof.

In addition, the modification may be substitution, removal, addition of some bases in the base sequence encoding the editor protein, or a combination thereof.

Another embodiment of the content disclosed by the present specification relates to a composition for gene manipulation, including: a guide nucleic acid capable of forming bonds with target sequences of one or more genes selected from the group consisting of an SPT4 gene, SPT5 gene, SUPT4H gene, and SUPT5H gene; and
an editor protein capable of acting as gene scissors for the target sequences.

The composition for gene manipulation may be a composition for gene manipulation.

As one embodiment of the contents disclosed by the present application, a composition for gene manipulation may include gRNA and CRISPR enzyme.

A composition for gene manipulation comprises:
(a) a gRNA capable of forming complementary bonds with respect to the target sequences of a repeat expansion expression regulatory gene or a nucleic acid sequence encoding the same; and
(b) one or more CRISPR enzyme or a nucleic acid sequence encoding the same.

The repeat expansion expression regulatory gene is the same as described above.

The target sequence is the same as described above.

A composition for gene manipulation may include gRNA-CRISPR enzyme complex.

The term "gRNA-CRISPR enzyme complex" refers to a complex formed through the interaction between a gRNA and a CRISPR enzyme.

The gRNA is the same as described above.

The term "CRISPR enzyme" is a main protein component of a CRISPR-Cas system, and forms a complex with gRNA, resulting in the CRISPR-Cas system.

The CRISPR enzyme may be a nucleic acid or polypeptide (or a protein) having a sequence encoding the CRISPR enzyme.

The CRISPR enzyme may be Type II CRISPR enzyme.

The crystal structure of the type II CRISPR enzyme was determined according to studies on two or more types of natural microbial type II CRISPR enzyme molecules (Jinek et al., Science, 343(6176):1247997, 2014) and studies on Streptococcus pyogenes Cas9 (SpCas9) complexed with gRNA (Nishimasu et al., Cell, 156:935-949, 2014; and Anders et al., Nature, 2014, doi: 10.1038/nature13579).

The type II CRISPR enzyme includes two lobes, that is, recognition (REC) and nuclease (NUC) lobes, and each lobe includes several domains.

The REC lobe includes an arginine-rich bridge helix (BH) domain, an REC1 domain and an REC2 domain.

Here, the BH domain is a long α-helix and arginine-rich region, and the REC1 and REC2 domains play an important role in recognizing a double strand formed in gRNA, for example, single-stranded gRNA, double-stranded gRNA or tracrRNA.

The NUC lobe includes an RuvC domain, an HNH domain and a PAM-interaction (PI) domain. Here, the RuvC domain encompasses RuvC-like domains, or the HNH domain is used to include HNH-like domains.

Here, the RuvC domain shares structural similarity with members of the microorganism family existing in nature having the type II CRISPR enzyme, and cleaves a single strand, for example, a non-complementary strand of a target gene or nucleic acid, that is, a strand not forming a complementary bond with gRNA. The RuvC domain is sometimes referred to as an RuvCI domain, RuvCII domain or RuvCIII domain in the art, and generally called an RuvC I, RuvCII or RuvCIII.

The HNH domain shares structural similarity with the HNH endonuclease, and cleaves a single strand, for example, a complementary strand of a target nucleic acid molecule, that is, a strand forming a complementary bond with gRNA. The HNH domain is located between RuvC II and III motifs.

The PI domain recognizes a specific base sequence in a target gene or nucleic acid, that is, a protospacer adjacent motif (PAM) or interacts with PAM.Here, the PAM may vary according to the origin of the type II CRISPR enzyme. For example, when the CRISPR enzyme is SpCas9, PAM may be 5'-NGG-3', when the CRISPR enzyme is Streptococcus thermophilus Cas9 (StCas9), PAM may be 5'-NNAGAAW-3'(W = A or T), when the CRISPR enzyme is Neisseria meningitides Cas9 (NmCas9), PAM may be 5'-NNNNGATT-3', and when the CRISPR enzyme is Campylobacter jejuni Cas9 (CjCas9), PAM may be 5'-NNNVRYAC-3' (V = G or C or A, R = A or G, Y = C or T), where the N may be A, T, G or C; or A, U, G or C.

The Type II CRISPR enzyme may be Cas9.

The Cas9 may be derived from various microorganisms such as Streptococcus pyogenes, Streptococcus thermophilus, Streptococcus sp., Staphylococcus aureus, Nocardiopsis dassonvillei, Streptomyces pristinaespiralis, Streptomyces viridochromogenes, Streptosporangium roseum, AlicyclobacHlus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium arabaticum, Ammonifex degensii, Caldicelulosiruptor bescii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus and Acaryochloris marina.

The term "Cas9" is an enzyme which binds to gRNA so as to cleave or modify a target sequence or position on a target gene or nucleic acid, and may consist of an HNH domain capable of cleaving a nucleic acid strand forming a complementary bond with gRNA, an RuvC domain capable of cleaving a nucleic acid strand forming a complementary bond with gRNA, an REC domain recognizing a target and a PI domain recognizing PAM. Hiroshi Nishimasu et al. (2014) Cell 156:935-949 may be referenced for specific structural characteristics of Cas9.

The Cas9 may be isolated from a microorganism existing in nature or non-naturally produced by a recombinant or synthetic method.

In addition, the CRISPR enzyme may be Type V CRISPR enzyme.

Type V CRISPR enzyme includes similar RuvC domains corresponding to the RuvC domains of the type II CRISPR enzyme, and may consist of an Nuc domain, instead of the HNH domain of the type II CRISPR enzyme, REC and WED domains, which recognize a target, and a PI domain recognizing PAM. For specific structural characteristics of the type V CRISPR enzyme, Takashi Yamano et al. (2016) Cell 165:949-962 may be referenced.

The type V CRISPR enzyme may interact with gRNA, thereby forming a gRNA-CRISPR enzyme complex, that is, a CRISPR complex, and may allow a guide sequence to approach a target sequence including a PAM sequence in cooperation with gRNA. Here, the ability of the type V CRISPR enzyme for interaction with a target gene or nucleic acid is dependent on the PAM sequence.

The PAM sequence is a sequence present in a target gene or nucleic acid, and may be recognized by the PI domain of the type V CRISPR enzyme. The PAM sequence may vary according to the origin of the type V CRISPR enzyme. That is, there are different PAM sequences which are able to be specifically recognized depending on a species. For example, when the CRISPR enzyme is a Cpfl protein, the PAM sequence is 5'-TTN-3' (N is A, T, C or G). However, although it is generally understood that the PAM sequence is determined according to the origin of the enzyme described above, the PAM sequence may varies as the study of the mutant of the enzyme proceeds.

The Type V CRISPR enzyme may be Cpf1.

The Cpfl may be derived from Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium, Corynebacter, Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opitutaceae, Tuberibacillus, Bacillus, Brevibacillus, Methylobacterium or Acidaminococcus.

The Cpfl may consist of an RuvC domain similar and corresponding to the RuvC domain of Cas9, an Nuc domain without the HNH domain of Cas9, an REC domain recognizing a target, a WED domain and a PI domain recognizing PAM. For specific structural characteristics of Cpfl, Takashi Yamano et al. (2016) Cell 165:949-962 may be referenced.

The Cpfl may be isolated from a microorganism existing in nature or non-naturally produced by a recombinant or synthetic method.

The CRISPR enzyme may be a nuclease or restriction enzyme which has a function of cleaving the double strand of target gene or nucleic acid's DNA.

The CRISPR enzyme may be complete active CRISPR enzyme.

The "completely active" refers to a state having the same function as a function of a wild-type CRISPR enzyme, and the complete active CRISPR enzyme refers to an enzyme having the same function as a function of a wild-type CRISPR enzyme. Here, the "function of a wild-type CRISPR enzyme" is a function of cleaving the double strand of DNA. In other words, it refers to have both of first and second functions, wherein the first function is a function of cleaving a first strand of the double-stranded DNA, and the second function is a function of cleaving a second strand of the double-stranded DNA.

The complete active CRISPR enzyme may be the wild-type CRISPR enzyme cleaving the double strand of DNA.

The complete active CRISPR enzyme may be a CRISPR enzyme variant which is a modified or manipulated wild-type CRISPR enzyme cleaving the double strand of DNA.

The CRISPR enzyme mutant may be an enzyme in which one ore more amino acids in the amino acid sequence of the wild-type CRISPR enzyme are removed, or substituted with anothers.

The CRISPR enzyme mutant may be an enzyme in which one or more amino acids are inserted at the amino acid sequence of the wild-type CRISPR enzyme. Here, the inserted amino acid may be located in the amino terminus (N-terminus), in the carboxyl terminus (C-terminus), or in the middle of the amino acid sequence of the wild-type CRISPR enzyme.

The CRISPR enzyme mutant may be an complete active CRISPR enzyme having an improved function compares to the function of the wild-type CRISPR enzyme.

For example, a modified or manipulated wild-type CRISPR enzyme - the CRISPR enzyme mutant - can cleave DNA double strands without binding to the DNA double strands, or maintaining a constant distant apart. In this case, the modified or manipulated CRISPR enzyme may be an complete active CRISPR enzyme having an improved function activation compares to the function activation of the wild-type CRISPR enzyme.

The CRISPR enzyme mutant may be an complete active CRISRP enzyme having an reduced function compares to the function of the wild-type CRISPR enzyme.

For example, a modified or manipulated wild-type CRISPR enzyme - CRISPR enzyme variant - can cleave DNA double strands within certain distance, or in the presence of specific bonds. In this case, the modified or manipulated CRISPR enzyme may be a complete active CRISPR enzyme having an reduced function activation compares to the function activation of the wild-type CRISPR enzyme.

The CRISPR enzyme may be an incomplete or partial CRISPR enzyme.
the "incomplete or partial active" refers to a state which has a function selected from first and second functions of the wild-type enzyme. Here, the first function is cleaving a first single strand of the double-stranded DNA, the second function is cleaving a second single strand of the double-stranded DNA. a CRISPR enzyme in the state described is called incomplete or partial active CRISPR enzyme. In addition, the incomplete or partial active CRISPR enzyme can be called a nickase.

The term "nickase" refers to a CRISPR enzyme manipulated or modified to cleave only one strand of the double strand of the target gene or nucleic acid, and the nickase has nuclease activity of cleaving a single strand, for example, a strand that is not complementary or complementary to gRNA of the target gene or nucleic acid. Therefore, to cleave the double strand, nuclease activity of the two nickases is needed.

For example, the nickase may have nuclease activity by the RuvC domain. That is, the nickase may include nuclease activity of the HNH domain, and to this end, the HNH domain may be manipulated or modified.

In one example, provided that the CRISPR enzyme is the type II CRISPR enzyme, the nickase may be a Type II CRISPR enzyme which includes modified HNH domain.

For example, provided that the Type II CRISPR enzyme is wild-type SpCas9, the nickase may be a manipulated SpCas9 that the nuclease activity of the HNH domain thereof is inactivated, by mutating the residue 840 in the amino acid sequence of SpCas9 from histidine to alanine. Since the nickase produced thereby has nuclease activity of the RuvC domain, it is able to cleave a strand which does not form a complementary bond with a non-complementary strand of the target gene or nucleic acid, that is, gRNA.

For example, provided that the Type II CRISPR enzyme is wild-type CjCas9, the nickase may be a manipulated CjCas9 that the nuclease activity of the HNH domain thereof is inactivated, by mutating the residue 559 in the amino acid sequence of CjCas9 from histidine to alanine. Since the nickase produced thereby has nuclease activity of the RuvC domain, it is able to cleave a strand which does not form a complementary bond with a non-complementary strand of the target gene or nucleic acid, that is, gRNA.

In addition, the nickase may have nuclease activity by the HNH domain. That is, the nickase may include the nuclease activity of the RuvC domain, and to this end, the RuvC domain may be manipulated or modified.

In one example, provided that the CRISPR enzyme is the type II CRISPR enzyme, the nickase may be a Type II CRISPR enzyme which includes modified RuvC domain.

For example, provided that the Type II CRISPR enzyme is wild-type SpCas9, the nickase may be a manipulated SpCas9 that the nuclease activity of the HNH domain thereof is inactivated, by mutating the residue 10 in the amino acid sequence of SpCas9 from aspartic acid to alanine. The nickase produced thereby has the nuclease activity of the HNH domain, and thus is able to cleave a complementary strand of the target gene or nucleic acid, that is, a strand that forms a complementary bond with gRNA.

For example, provided that the Type II CRISPR enzyme is wild-type CjCas9, the nickase may be a manipulated CjCas9 that the nuclease activity of the HNH domain thereof is inactivated, by mutating the residue 8 in the amino acid sequence of CjCas9 from aspartic acid to alanine. The nickase produced thereby has the nuclease activity of the HNH domain, and thus is able to cleave a complementary strand of the target gene or nucleic acid, that is, a strand that forms a complementary bond with gRNA.

The CRISPR enzyme may be inactive CRISPR enzyme.

The "inactive" refers to a state which loses all of the functions of the wild-type enzyme, wherein the functions refers a first function cleaving a first single strand of the double-stranded DNA, and a second function cleaving a second single strand of the double-stranded DNA. a CRISPR enzyme in the state described is called inactive CRISPR enzyme.
the inactive CRISPR enzyme has nuclease inactivity due to the mutation in the domain with nuclease activity of the wild-type CRISPR enzyme.

The inactive CRISPR enzyme has nuclease inactivity due to the mutation in the RuvC domain and HNH domain. That is, the inactive CRISPR enzyme may not include nuclease activity of the CRISPR enzyme due to the RuvC domain and HNH domain. For this purpose, the RuvC domain and the HNH domain may be manipulated or modified.

In one example, when the CRISPR enzyme is Type II CRISPR enzyme, the CRISPR enzyme may be a type II CRISPR enzyme which includes modified RuvC domain and modified HNH domain.

For example, provided that the Type II CRISPR enzyme is wild-type SpCas9, the inactive CRISPR enzyme may be a manipulated SpCas9 that the nuclease activity of the RuvC domain and HNH domain thereof are inactivated, by mutating the residues 10 and 840 in the amino acid sequence of SpCas9 from aspartic acid and histidine to alanine, respectively. Since nuclease activities by the RuvC domain and the HNH domain of the inacitve CRISPR enzyme produced thereby are inactivated, such that the double strand may not cleave completely the double strand of the target gene or nucleic acid.

In another example, provided that the Type II CRISPR enzyme is wild-type CjCas9, the inactive CRISPR enzyme may be a manipulated CjCas9 that the nuclease activity of the RuvC domain and HNH domain thereof are inactivated, by mutating the residues 8 and 559 in the amino acid sequence of SpCas9 from aspartic acid and histidine to alanine, respectively. Since nuclease activities by the RuvC domain and the HNH domain of the inacitve CRISPR enzyme produced thereby are inactivated, such that the double strand may not cleave completely the double strand of the target gene or nucleic acid.

The CRISPR enzyme may have endonuclease activity, exonuclease activity or helicase activity, that is, an ability to anneal the helix structure of the double-stranded nucleic acid, in addition to the above-described nuclease activity.

In addition, the CRISPR enzyme may be modified to completely, incompletely, or partially activate helicase activity.

The CRISPR enzyme may be a CRISPR enzyme mutant, which is artificially modified or manipulated wild-type CRISPR enzyme.

The CRISPR enzyme mutant may be artificially modified or manipulated CRISPR enzyme mutant, to modify a first function cleaving a first single strand of the double-stranded DNA and/or a second function cleaving a second single strand of the double-stranded DNA.

For example, the CRISPR enzyme mutant may be a CRISPR enzyme in which the first function of the wild-type CRISPR enzyme is lost.

Or, the CRISPR enzyme mutant may be a CRISPR enzyme in which the second function of the wild-type CRISPR enzyme is lost.

For example, the CRISPR enzyme mutant may be a CRISPR enzyme in which the functions of the wild-type CRISPR enzyme, that is, the first and second functions are lost.

The CRISPR enzyme mutant may form gRNA-CRISPR enzyme complex through the interaction with a gRNA.

The CRISPR enzyme mutant may be artificially modified or manipulated CRISPR enzyme mutant, to modify a function interacting with gRNA of wild-type CRISPR enzyme.

For example, the CRISPR enzyme mutant may have reduced interaction with gRNA compares to wild-type CRISPR enzyme.

In addition, the CRISPR enzyme mutant may have increased interaction with gRNA compares to wild-type CRISPR enzyme.

For example, the CRISPR enzyme mutant may have reduced interaction with gRNA compares to wild-type CRISPR enzyme, while having the first function of the wild-type CRISPR enzyme.

Or, the CRISPR enzyme mutant may have increased interaction with gRNA compares to wild-type CRISPR enzyme, while having the first function of the wild-type CRISPR enzyme.

For example, the CRISPR enzyme mutant may have reduced interaction with gRNA compares to wild-type CRISPR enzyme, while having the second function of the wild-type CRISPR enzyme.

Or, the CRISPR enzyme mutant may have increased interaction with gRNA compares to wild-type CRISPR enzyme, while having the second function of the wild-type CRISPR enzyme.

For example, the CRISPR enzyme mutant may have reduced interaction with gRNA compares to wild-type CRISPR enzyme, while not having the first and second functions of the wild-type CRISPR enzyme.

For example, the CRISPR enzyme mutant may have increased interaction with gRNA compares to wild-type CRISPR enzyme, while not having the first and second functions of the wild-type CRISPR enzyme.

Here, various gRNA-CRISPR enzyme complexes may be formed according to the interaction strength between the gRNA and the CRISPR enzyme mutant, and the function of accessing or cleaving the target sequence may be vary according to the CRISPR enzyme mutant.

For example, gRNA-CRISPR enzyme complex formed by a CRISPR enzyme mutant with reduced interaction with gRNA may cleave a single or double strands only when the gRNA-CRISPR enzyme complex is close to or locallized to a target sequence forming a complete complementary bond with gRNA.

The CRISPR enzyme mutant may be a CRISPR enzyme in which one or more amino acids of amino acid sequence of wild-type CRISPR enzyme is modified.

In one example, the CRISPR enzyme mutant may be a CRISPR enzyme in which one or more amino acids of amino acid sequence of wild-type CRISPR enzyme is substituted.

In another example, the CRISPR enzyme mutant may be a CRISPR enzyme in which one or more amino acids of amino acid sequence of wild-type CRISPR enzyme is deleted.

In yet another example, the CRISPR enzyme mutant may be a CRISPR enzyme in which one or more amino acids of amino acid sequence of wild-type CRISPR enzyme is added.

In one example, the CRISPR enzyme mutant may be a CRISPR enzyme in which one or more amino acids of amino acid sequence of wild-type CRISPR enzyme is substituted, deleted and/or added.

In addition, the CRISPR enzyme mutant may selectively include additional functional domain, in addition to the original function of the wild-type CRISPR enzyme, which is, a first function cleaving a first single strand of the double-stranded DNA, and a second function cleaving a second single strand of the double-stranded DNA. Here, the CRISPR enzyme mutant may have additional functions in addition to the original function of the wild-type CRISPR enzyme.
the functional domain may be a domain having methylase activity, demethylase activity, transcription activation activity, transcription repression activity, transcription release factor activity, histone modification activity, RNA cleavage activity or nucleic acid binding activity, or a tag or reporter gene for isolating and purifying a protein (including a peptide), but the present invention is not limited thereto.

The tag includes a histidine (His) tag, a V5 tag, a FLAG tag, an influenza hemagglutinin (HA) tag, a Myc tag, a VSV-G tag and a thioredoxin (Trx) tag, and the reporter gene includes glutathione-S-transferase (GST), horseradish peroxidase (HRP), chloramphenicol acetyltransferase (CAT) β-galactosidase, β-glucoronidase, luciferase, autofluorescent proteins including the green fluorescent protein (GFP), HcRed, DsRed, cyan fluorescent protein (CFP), yellow fluorescent protein (YFP) and blue fluorescent protein (BFP), but the present invention is not limited thereto.

The functional domain, peptide, polypeptide or protein may be a deaminase.

For example, an incomplete or partial CRISPR enzyme may additionally include a cytidine deaminase as a functional domain. In one exemplary embodiment, a cytidine deaminase, for example, apolipoprotein B editing complex 1 (APOBEC1) may be added to SpCas9 nickase, thereby producing a fusion protein. The [SpCas9 nickase]-[APOBEC1] formed thereby may be used in base repair or editing of C into T or U, or G into A.

For another example, an incomplete or partial CRISPR enzyme may additionally include a adenine deaminase as a functional domain. In one exemplary embodiment, a adenine deaminase, for example, TadA variants, ADAR2 variants, or ADAT2 variants may be added to SpCas9 nickase, thereby producing a fusion protein. The [SpCas9 nickase]-[TadA variant], [SpCas9 nickase]-[ADAR2 variant] or [SpCas9 nickase]-[ADAT2 variant] formed thereby transforms nucleotide A to inosine, and the modified inosine is recognized as nucleotide G by the polymerase, and thus having the effect of manipulating or editing nucleotide A into G. Therefore, the fusion protein described above may be used in editing of A into G, or T into C.

In addition, the functional domain, peptide, polypeptide or protein may be a nuclear localization sequence or signal (NLS) or a nuclear export sequence or signal (NES).

The NLS may be NLS of SV40 virus large T-antigen with an amino acid sequence PKKKRKV; NLS derived from nucleoplasmin (e.g., nucleoplasmin bipartite NLS with a sequence KRPAATKKAGQAKKKK); c-myc NLS with an amino acid sequence PAAKRVKLD or RQRRNELKRSP; hRNPA1 M9 NLS with a sequence NQSSNFGPMKGGNFGGRSSGPYGGGGQYFAKPRNQGGY; an importin-α-derived IBB domain sequence RMRIZFKNKGKDTAELRRRRVEVSVELRKAKKDEQILKRRNV; myoma T protein sequences VSRKRPRP and PPKKARED; human p53 sequence POPKKKPL; a mouse c-abl IV sequence SALIKKKKKMAP; influenza virus NS1 sequences DRLRR and PKQKKRK; a hepatitis virus-δ antigen sequence RKLKKKIKKL; a mouse Mx1 protein sequence REKKKFLKRR; a human poly(ADP-ribose) polymerase sequence KRKGDEVDGVDEVAKKKSKK; or steroid hormone receptor (human) glucocorticoid sequence RKCLQAGMNLEARKTKK, but the present invention is not limited thereto.

In addition, the CRISPR enzyme mutant may include a split-type CRISPR enzyme prepared by dividing the CRISPR enzyme into two or more parts. The term "split" refers to functional or structural division of a protein or random division of a protein into two or more parts.

Here, the split-type CRISPR enzyme may be a completely, incompletely or partially active enzyme or inactive enzyme.

For example, the SpCas9 may be divided into two parts between the residue 656, tyrosine, and the residue 657, threonine, thereby generating split SpCas9. In addition, the split-type CRISPR enzyme may selectively include an additional domain, peptide, polypeptide or protein for reconstitution.

The split-type CRISPR enzyme may selectively include additional domain, peptide, polypeptide, or protein for reconstitution.

The additional domain, peptide, polypeptide, or protein for reconstitution may assemble to make the split-type CRISPR enzyme into be structurally the same or similar to the wild-type CRISPR enzyme.

The additional domain, peptide, polypeptide or protein for reconstitution may be FRB and FKBP dimerization domains; intein; ERT and VPR domains; or domains which form a heterodimer under specific conditions.

For example, the SpCas9 may be divided into two parts between the residue 713, serine, and the residue 714, glycine, thereby generating split SpCas9. The FRB domain may be connected to one of the two parts, and the FKBP domain may be connected to the other one. In the split SpCas9 produced thereby, the FRB domain and the FKBP domain may be formed in a dimer in an environment in which rapamycine is present, thereby producing a reconstituted CRISPR enzyme.

The CRISPR enzyme or CRISPR enzyme mutant described in the present invention may be a polypeptide, protein or nucleic acid having a sequence encoding the same, and may be codon-optimized for a subject to introduce the CRISPR enzyme or CRISPR enzyme mutant.

The term "codon optimization" refers to a process of modifying a nucleic acid sequence by maintaining a native amino acid sequence while replacing at least one codon of the native sequence with a codon more frequently or the most frequently used in host cells so as to improve expression in the host cells. A variety of species have a specific bias to a specific codon of a specific amino acid, and the codon bias (the difference in codon usage between organisms) is frequently correlated with efficiency of the translation of mRNA, which is considered to be dependent on the characteristic of a translated codon and availability of a specific tRNA molecule. The dominance of tRNA selected in cells generally reflects codons most frequently used in peptide synthesis. Therefore, a gene may be customized by optimal gene expression in a given organism based on codon optimization.

The gRNA, CRISPR enzyme or gRNA-CRISPR enzyme complex may be delivered or introduced into a subject by various forms.

The subject is the same as described above.

In one embodiment, the nucleic acid sequence encoding the gRNA and/or CRISPR enzyme may be delivered or introduced into a subject by a vector.

The vector may include a nucleic acid sequence encoding a gRNA and/or CRISPR enzyme.

In one example, the vector may simultaneously include nucleic acid sequences, which encode the gRNA and the CRISPR enzyme, respectively.

In another example, the vector may include the nucleic acid sequence encoding the gRNA.

In one example, domains contained in the gRNA may be contained in one vector, or may be divided and then contained in different vectors.

For example, the vector may include the nucleic acid sequence encoding the CRISPR enzyme.

In one example, in the case of the CRISPR enzyme, the nucleic acid sequence encoding the CRISPR enzyme may be contained in one vector, or may be divided and then contained in several vectors.

The vector may include one or more regulatory/control components.

Here, the regulatory/control components may include a promoter, an enhancer, an intron, a polyadenylation signal, a Kozak consensus sequence, an internal ribosome entry site (IRES), a splice acceptor and/or a 2A sequence.

The promoter may be a promoter recognized by RNA polymerase II.

The promoter may be a promoter recognized by RNA polymerase III.

The promoter may be an inducible promoter.

The promoter may be a subject-specific promoter.

The promoter may be a viral or non-viral promoter.

The promoter may use a suitable promoter according to a control region (that is, a nucleic acid sequence encoding a guide nucleic acid or editor protein).

For example, a promoter useful for the guide nucleic acid may be a HI, EF-la, tRNA or U6 promoter. For example, a promoter useful for the editor protein may be a CMV, EF-la, EFS, MSCV, PGK or CAG promoter.

The vector may be a viral vector or recombinant viral vector.

The virus may be a DNA virus or an RNA virus.

Here, the DNA virus may be a double-stranded DNA (dsDNA) virus or single-stranded DNA (ssDNA) virus.

Here, the RNA virus may be a single-stranded RNA (ssRNA) virus.

The virus may be a retrovirus, a lentivirus, an adenovirus, adeno-associated virus (AAV), vaccinia virus, a poxvirus or a herpes simplex virus, but the present invention is not limited thereto.

In one example, a nucleic acid sequence encoding a guide nucleic acid and/or editor protein may be delivered or introduced by a recombinant lentivirus.

In another example, a nucleic acid sequence encoding a guide nucleic acid and/or editor protein may be delivered or introduced by a recombinant adenovirus.

In still another example, a nucleic acid sequence encoding a guide nucleic acid and/or editor protein may be delivered or introduced by recombinant AAV.

In yet another example, a nucleic acid sequence encoding a guide nucleic acid and/or editor protein may be delivered or introduced by a hybrid virus, for example, one or more hybrids of the virus listed herein.

In one embodiment, The gRNA and the CRISPR enzyme may be delivered or introduced into a subject in the form of a gRNA-CRISPR enzyme complex.

For example, the guide nucleic acid may be DNA, RNA or a mixture thereof. The CRISPR enzyme may be a peptide, polypeptide or protein.

In one example, the gRNA and the CRISPR enzyme may be delivered or introduced into a subject in the form of a gRNA-CRISPR complex containing an RNA-type gRNA and a protein-type CRISPR, that is, a ribonucleoprotein (RNP).

The CRISPR gRNA-CRISPR enzyme complex may be delivered or introduced into a subject by electroporation, microinjection, transient cell compression or squeezing (e.g., described in the literature [Lee, et al, (2012) Nano Lett., 12, 6322-6327]), lipid-mediated transfection, nanoparticles, a liposome, peptide-mediated delivery or a combination thereof.

As diclosed herein, a gRNA-CRISPR enzyme complex may be used in manipulation or modification of the target gene, that is, a repeat expansion expression regulatory gene.

The target gene or nucleic acid may be manipulated or corrected using the above-described gRNA-CRISPR enzyme complex, that is, the CRISPR complex. Here, the manipulation or correction of the target gene or nucleic acid includes all of the stages of i) cleaving or damaging the target gene or nucleic acid and ii) repairing the damaged target gene or nucleic acid.

The i) The cleavage or damage of the target gene or nucleic acid may be cleavage or damage of the target gene or nucleic acid using the CRISPR complex, and particularly, cleavage or damage of a target sequence in the target gene or nucleic acid.

The target sequence may be a target for the gRNA-CRISPR enzyme complex, the target sequence may include or not include a PAM sequence recognized by the CRISPR enzyme. Such a target sequence may provide a critical standard in a gRNA designing stage to those of ordinary skill in the art.

The target sequence may be able to specifically recognized by the gRNA of the gRNA-CRISPR enzyme complex, thus the gRNA-CRISPR enzyme complex may located adjacent to the recognized target sequence.

The term "cleavage" in a target region refers to breakage of a covalent backbone of polynucleotides. The cleavage includes enzymatic or chemical hydrolysis of a phosphodiester bond, but the present invention is not limited thereto, and also include various other methods. The cleavage is able to be performed on both of a single strand and a double strand, and the cleavage of a double strand may result from distinct single-strand cleavage. The double-strand cleavage may generate blunt ends or staggered ends(or sticky end).

In one example, the cleavage or damage of the target gene or nucleic acid using the CRISPR complex may be complete cleavage or damage to the double strand of a target sequence.

In one exemplary embodiment, when wild-type SpCas9 is used, the double strand of a target sequence forming a complementary bond with gRNA may be completely cleaved.

In another exemplary embodiment, when SpCas9 nickase (D10A) and SpCas9 nickase (H840A) are used, a complementary single strand of a target sequence forming a complementary bond with gRNA may be cleaved by the SpCas9 nickase (D10A), and a non-complementary single strand of the target sequence forming a complementary bond with gRNA may be cleaved by the SpCas9 nickase (H840A), and the cleavages may take place sequentially or simultaneously.

In another example, the cleavage or damage of a target gene or nucleic acid using the CRISPR complex may be cleavage or damage to only the single strand of a target sequence. Here, the single strand may be a complementary single strand of a target sequence forming a complementary bond with gRNA, or a non-complementary single strand of the target sequence not forming a complementary bond with gRNA.

In one exemplary embodiment, when SpCas9 nickase (D10A) is used, a complementary single strand of a target sequence forming a complementary bond with gRNA may be cleaved by the SpCas9 nickase (D10A), but a non-complementary single strand of the target sequence forming a complementary bond with gRNA may not be cleaved.

In another exemplary embodiment, when SpCas9 nickase (H840A) is used, a complementary single strand of a target sequence forming a complementary bond with gRNA may be cleaved by the SpCas9 nickase (H840A), but a non-complementary single strand of the target sequence forming a complementary bond with gRNA may not be cleaved.

In yet another example, the cleavage or damage of a target gene or nucleic acid using the CRISPR complex may be partial removal of a nucleic acid fragment.

In one exemplary embodiment, when two gRNAs having different target sequences and wild-type SpCas9 are used, a double strand of a target sequence forming a complementary bond with the first gRNA may be cleaved, and a double strand of a target sequence forming a complementary bond with the second gRNA may be cleaved, resulting in the removal of nucleic acid fragments by the first and second gRNAs and SpCas9.

The target gene or nucleic acid cleaved or damaged by the CRISPR complex may be repaired or restored through NHEJ and homology-directed repairing (HDR).

The non-homologous end joining (NHEJ) is a method of restoration or repairing double strand breaks in DNA by joining both ends of a cleaved double or single strand together, and generally, when two compatible ends formed by breaking of the double strand (for example, cleavage) are frequently in contact with each other to completely join the two ends, the broken double strand is recovered. The NHEJ is a restoration method that is able to be used in the entire cell cycle, and usually occurs when there is no homologous genome to be used as a template in cells, like the G1 phase.

In the repair process of the damaged gene or nucleic acid using NHEJ, some insertions and/or deletions (indels) in the nucleic acid sequence occur in the NHEJ-repaired region, such insertions and/or deletions cause the leading frame to be shifted, resulting in frame-shifted transcriptome mRNA. As a result, innate functions are lost because of nonsense-mediated decay or the failure to synthesize normal proteins. In addition, while the leading frame is maintained, mutations in which insertion or deletion of a considerable amount of sequence may be caused to destroy the functionality of the proteins. The mutation is locus-dependent because mutations in a significant functional domain is probably less tolerated than mutations in a non-significant region of a protein.

While it is impossible to expect indel mutations produced by NHEJ in a natural state, a specific indel sequence is preferred in a given broken region, and can come from a small region of micro homology. Conventionally, the deletion length ranges from 1 bp to 50 bp, insertions tend to be shorter, and frequently include a short repeat sequence directly surrounding a broken region.

In addition, the NHEJ is a process causing a mutation, and when it is not necessary to produce a specific final sequence, may be used to delete a motif of the small sequence.

A specific knockout of a gene targeted by the CRISPR complex may be performed using such NHEJ. A double strand or two single strands of a target gene or nucleic acid may be cleaved using the CRISPR enzyme such as Cas9 or Cpfl, and the broken double strand or two single strands of the target gene or nucleic acid may have indels through the NHEJ, thereby inducing specific knockout of the target gene or nucleic acid. Here, the site of a target gene or nucleic acid cleaved by the CRISPR enzyme may be a non-coding or coding region, and in addition, the site of the target gene or nucleic acid restored by NHEJ may be a non-coding or coding region.

In one example, various indels may occur at repaired regeion due to the process comprising: cleaving the double strands of the target gene by the gRNA-CRISPR complex; and repairing them by NHEJ.

The term "indel" is the generic term for an insertion or deletion mutation occurring in-between some bases in a DNA base sequence. The indel may be introduced into a target sequence during repair by an HDR or NHEJ mechanism when the gRNA-CRISPR enzyme complex cleaves the nucleic acid (DNA or RNA) of the expression regulatory factor for the over-amplified repeated sequence as described above.

HDR is a correction method without an error, which uses a homologous sequence as a template to repair or restoration a damaged gene or nucleic acid, and generally, to repair or restoration broken DNA, that is, to restore innate information of cells, the broken DNA is repaired using information of a complementary base sequence which is not modified or information of a sister chromatid. The most common type of HDR is homologous recombination (HR). HDR is a repair or restoration method usually occurring in the S or G2/M phase of actively dividing cells.

To repair or restore damaged DNA using HDR, rather than using a complementary base sequence or sister chromatin of the cells, a DNA template artificially synthesized using information of a complementary base sequence or homologous base sequence, that is, a nucleic acid template including a complementary base sequence or homologous base sequence may be provided to the cells, thereby repairing the broken DNA. Here, when a nucleic acid sequence or nucleic acid fragment is further added to the nucleic acid template to repair the broken DNA, the nucleic acid sequence or nucleic acid fragment further added to the broken DNA may be subjected to knockin. The further added nucleic acid sequence or nucleic acid fragment may be a nucleic acid sequence or nucleic acid fragment for correcting the target gene or nucleic acid modified by a mutation to a normal gene or nucleic acid, or a gene or nucleic acid to be expressed in cells, but the present invention is not limited thereto.

An in-frame indel or an out-frame indel may be produced by the indel.

The "in-frame indel" collectively refers to a mutation in which 3 n (n is an integer) nucleotides are inserted or deleted when some nucleotides in the nucleotide arrangement of DNA are inserted into the middle or deleted. During the translation of an RNA transcribed from double strands of a target gene or nucleic acid in which the in-frame indel is induced, a reading frame may be maintained. The double strands of the target gene or nucleic acid may be mutated into a sequence in which some polypeptides around a nucleoide insertion or deletion locus are inserted or deleted, and the subsequent polypeptide encodes the same polypeptide as that before the insertion or deletion of the nucleotide by an in-frame indel.

The "out-frame indel" collectively refers to a mutation in which 3n+1 or 3n+2 (n is an integer) nucleotides are inserted or deleted when some nucleotides in the nucleotide arrangement of a DNA are inserted into the middle or deleted. During the translation of an RNA transcribed from double strands of a target gene or nucleic acid in which the out-frame indel is induced, a reading frame may be altered. The double strands of the target gene or nucleic acid may encode a polypeptide in which a polypeptide after a nucleotide insertion or deletion locus is substituted or deleted by an out-frame indel.

In one example, a double or single strand of a target gene or nucleic acid may be cleaved using the CRISPR complex, a nucleic acid template including a base sequence complementary to a base sequence adjacent to the cleavage site may be provided to cells, and the cleaved base sequence of the target gene or nucleic acid may be repaired or restored through HDR.

Here, the nucleic acid template including the complementary base sequence may have broken DNA, that is, a cleaved double or single strand of a complementary base sequence, and further include a nucleic acid sequence or nucleic acid fragment to be inserted into the broken DNA. An additional nucleic acid sequence or nucleic acid fragment may be inserted into a cleaved site of the broken DNA, that is, the target gene or nucleic acid using the nucleic acid template including a nucleic acid sequence or nucleic acid fragment to be inserted into the complementary base sequence. Here, the nucleic acid sequence or nucleic acid fragment to be inserted and the additional nucleic acid sequence or nucleic acid fragment may be a nucleic acid sequence or nucleic acid fragment for correcting a target gene or nucleic acid modified by a mutation to a normal gene or nucleic acid or a gene or nucleic acid to be expressed in cells. The complementary base sequence may be a base sequence having complementary bonds with broken DNA, that is, right and left base sequences of the cleaved double or single strand of the target gene or nucleic acid. Alternatively, the complementary base sequence may be a base sequence having complementary bonds with broken DNA, that is, 3' and 5' ends of the cleaved double or single strand of the target gene or nucleic acid. The complementary base sequence may be a 15 to 3000-base sequence, a length or size of the complementary base sequence may be suitably designed according to a size of the nucleic acid template or the target gene. Here, as the nucleic acid template, a double- or single-stranded nucleic acid may be used, or it may be linear or circular, but the present invention is not limited thereto.

In another example, a double- or single-stranded target gene or nucleic acid is cleaved using the CRISPR complex, a nucleic acid template including a homologous base sequence with a base sequence adjacent to a cleavage site is provided to cells, and the cleaved base sequence of the target gene or nucleic acid may be repaired or restored by HDR.

Here, the nucleic acid template including the homologous base sequence may be broken DNA, that is, a cleaved double- or single-stranded homologous base sequence, and further include a nucleic acid sequence or nucleic acid fragment to be inserted into the broken DNA. An additional nucleic acid sequence or nucleic acid fragment may be inserted into broken DNA, that is, a cleaved site of a target gene or nucleic acid using the nucleic acid template including a homologous base sequence and a nucleic acid sequence or nucleic acid fragment to be inserted. Here, the nucleic acid sequence or nucleic acid fragment to be inserted and the additional nucleic acid sequence or nucleic acid fragment may be a nucleic acid sequence or nucleic acid fragment for correcting a target gene or nucleic acid modified by a mutation to a normal gene or nucleic acid or a gene or nucleic acid to be expressed in cells. The homologous base sequence may be broken DNA, that is, a base sequence having homology with cleaved double-stranded base sequence or right and left single-stranded base sequences of a target gene or nucleic acid. Alternatively, the complementary base sequence may be a base sequence having homology with broken DNA, that is, the 3' and 5' ends of a cleaved double or single strand of a target gene or nucleic acid. The homologous base sequence may be a 15 to 3000-base sequence, and a length or size of the homologous base sequence may be suitably designed according to a size of the nucleic acid template or a target gene or nucleic acid. Here, as the nucleic acid template, a double- or single-stranded nucleic acid may be used and may be linear or circular, but the present invention is not limited thereto.

Other than the NHEJ and HDR, there are methods of repairing or restoring broken DNA. For example, Single-strand annealing (SSA), Single-strand break repair (SSBA), Mismatch repair (MMR), Base excision repair (BER), or Nucleotide excision repair (NER).

The SSA is a method of repairing double strand breaks between two repeat sequences present in a target nucleic acid, and generally uses a repeat sequence of more than 30 bases. The repeat sequence is cleaved (to have sticky ends) to have a single strand with respect to a double strand of the target nucleic acid at each of the broken ends, and after the cleavage, a single-strand overhang containing the repeat sequence is coated with an RPA protein such that it is prevented from inappropriately annealing the repeat sequences to each other. RAD52 binds to each repeat sequence on the overhang, and a sequence capable of annealing a complementary repeat sequence is arranged. After annealing, a single-stranded flap of the overhang is cleaved, and synthesis of new DNA fills a certain gap to restore a DNA double strand. As a result of this repair, a DNA sequence between two repeats is deleted, and a deletion length may be dependent on various factors including the locations of the two repeats used herein, and a path or degree of the progress of cleavage.

SSA, similar to HDR, utilizes a complementary sequence, that is, a complementary repeat sequence, and in contrast, does not requires a nucleic acid template for modifying or correcting a target nucleic acid sequence.

Single strand breaks in a genome are repaired through a separate mechanism, SSBR, from the above-described repair mechanisms. In the case of single-strand DNA breaks, PARP1 and/or PARP2 recognizes the breaks and recruits a repair mechanism. PARP1 binding and activity with respect to the DNA breaks are temporary, and SSBR is promoted by promoting the stability of an SSBR protein complex in the damaged regions. The most important protein in the SSBR complex is XRCC1, which interacts with a protein promoting 3' and 5' end processing of DNA to stabilize the DNA. End processing is generally involved in repairing the damaged 3' end to a hydroxylated state, and/or the damaged 5' end to a phosphatic moiety, and after the ends are processed, DNA gap filling takes place. There are two methods for the DNA gap filling, that is, short patch repair and long patch repair, and the short patch repair involves insertion of a single base. After DNA gap filling, a DNA ligase promotes end joining.

The MMR works on mismatched DNA bases. Each of an MSH2/6 or MSH2/3 complex has ATPase activity and thus plays an important role in recognizing a mismatch and initiating a repair, and the MSH2/6 primarily recognizes base-base mismatches and identifies one or two base mismatches, but the MSH2/3 primarily recognizes a larger mismatch.

BER is a repair method which is active throughout the entire cell cycle, and used to remove a small non-helix-distorting base damaged region from the genome. In the damaged DNA, damaged bases are removed by cleaving an N-glycoside bond joining a base to the phosphate-deoxyribose backbone, and then the phosphodiester backbone is cleaved, thereby generating breaks in single-strand DNA. The broken single strand ends formed thereby were removed, a gap generated due to the removed single strand is filled with a new complementary base, and then an end of the newly-filled complementary base is ligated with the backbone by a DNA ligase, resulting in repair of the damaged DNA.

NER is an excision mechanism important for removing large helix-distorting damage from DNA, and when the damage is recognized, a short single-strand DNA segment containing the damaged region is removed, resulting in a single strand gap of 22 to 30 bases. The generated gap is filled with a new complementary base, and an end of the newly filled complementary base is ligated with the backbone by a DNA ligase, resulting in the repair of the damaged DNA.

Manipulation or correction of a target gene or nucleic acid may largely lead to effects of knockout, knockdown.

The term "knockout" refers to inactivation of a target gene or nucleic acid, and the "inactivation of a target gene or nucleic acid" refers to a state in which transcription and/or translation of a target gene or nucleic acid does not occur. Transcription and translation of a gene causing a disease or a gene having an abnormal function may be inhibited through knockout, resulting in the prevention of protein expression.

For example, when manipulating or editing a target gene or nucleic acid using a CRISPR complex, the target gene or nucleic acid may be cleaved by the CRISPR complex. The damaged target gene or nucleic acid may be repaired through NHEJ using the CRISPR complex. The damaged target gene or nucleic acid may have indels due to NHEJ, and thereby, specific knockout for the target gene or nucleic acid may be induced.

For another example, when manipulating or editing a target gene or nucleic acid using a CRISPR complex and donor, the target gene or nucleic acid may be cleaved by the CRISPR complex. The damaged target gene or nucleic acid by the CRISPR complex may be repaired using the donor through HDR. Here, the donor may include a homologous nucleotide sequence and a nucleotide sequence desired to insert.Here, a number of bases of the nucleotide sequence desired to insert may be vary according to an insertion site or purpose. When damaged gene or chromosome is repaired by the donor, the nucleotide sequences desired to insert is inserted into a part of the damaged nucleotide sequence. This may induce specific knockout of the target gene or chromosome.

The term "knockdown" refers to a decrease in transcription and/or translation of a target gene or nucleic acid or the expression of a target protein. The onset of a disease may be prevented or a disease may be treated by regulating the overexpression of a gene or protein through the knockdown.

For example, when a target gene or nucleic acid is edited or corrected using a gRNA-CRISPR inactive enzyme-transcription inhibitory activity domain complex, that is, a CRISPR inactive complex including a transcription inhibitory activity domain, the CRISPR inactive complex may specifically bind to the target gene or nucleic acid, transcription of the target gene or nucleic acid may be inhibited by the transcription inhibitory activity domain included in the CRISPR inactive complex, thereby inducing knockdown in which expression of the corresponding gene or nucleic acid is inhibited.

For another example, when a target gene or nucleic acid is manipulated or edited using a gRNA-CRISPR enzyme complex, that is, a CRISPR complex, the CRISPR complex may cleave a promoter and/or an enhancer region of a target gene or chromosome. Here, the gRNA may recognize a part of nucleotide sequences of promoter and/or the enhancer region of the target gene or chromosome as a target sequence. The target gene or chromosome cleaved or damaged by the CRISPR complex may be repaired through NHEJ. the damaged target gene or chromosome may have indels due to NHEJ, and thereby, specific knockout for the target gene or chromosome may be induced. Or, when using a donor selectively, the damaged target gene or chromosome by the CRISPR complex may be repaired through HDR. When damaged gene or chromosome is repaired by the donor, a nucleotide sequences desired to insert is inserted into a part of the damaged nucleotide sequence. This may induce specific knockout of the target gene or chromosome.

In one embodiment, a gRNA-CRISPR enzyme complex may artificially modify or edit an SPT4, SPT5, SUPT4H, and/or SUPT5H gene.

The gRNA-CRISPR enzyme complex may specifically recognize a target sequence of an SPT4, an SPT5, an SUPT4H, and/or an SUPT5H gene.

The target sequence may be recognized specifically by the gRNA-CRISPR enzyme complex, therefore gRNA-CRISPR enzyme complex may located adjacent to the recognized target sequence.

The target sequence may be a part or a region of an SPT4, an SPT5, an SUPT4H, and/or an SUPT5H in which artificial modification will be occured.

The target sequence may be continuous 10 to 25-base sequence of the promoter region of the SPT4, the SPT5, the SUPT4H, and/or the SUPT5H

The target sequence may be continuous 10 to 25-base sequence of the intron region of the SPT4, the SPT5, the SUPT4H, and/or the SUPT5H

The target sequence may be continuous 10 to 25-base sequence of the exon region of the SPT4, the SPT5, the SUPT4H, and/or the SUPT5H

The target sequence may be continuous 10 to 25-base sequence of the enhancer region of the SPT4, the SPT5, the SUPT4H, and/or the SUPT5H

The target sequence may be continuous 10 to 25-base sequence of the 3'-UTR region of the SPT4, the SPT5, the SUPT4H, and/or the SUPT5H

The target sequence may be continuous 10 to 25-base sequence of the 5'-UTR region of the SPT4, the SPT5, the SUPT4H, and/or the SUPT5H

The target sequence may be continuous 10 to 25-base sequence adjacent to the 5' end and/or 3' end of the proto-spacer-adjecent Motif(PAM) sequence of the SPT4, the SPT5, the SUPT4H, and/or the SUPT5H.

Here, the PAM sequence is at least one of the following sequence:
5'-NGG-3' (N is A, T, G, or C);
5'-NNNNRYAC-3' (each N is independently A, T, C or G, R is A or G, and Y is C or T);
5'-NNAGAAW-3' (each N is independently A, T, C or G, and W is A or T);
5'-NNNNGATT-3'(each N is independently A, T, C or G);
5'-NNGRR(T)-3'(each N is independently A, T, C or G, R is A or G, and (T) is a randomly addable sequence); and
5'-TTN-3' (N is A, T, C or G).

In one embodiment, the target sequence may be one ore more nucleic acid sequences selected from sequences listed in Table 1.

The gRNA-CRISPR enzyme complex may consist of gRNA and CRISPR enzyme.

The gRNA may include a guide domain which is able to form a partial or complete complementary bond with the guide nucleic acid binding sequence of the target sequence of the SPT4, the SPT5, the SUPT4H, and/or the SUPT5H gene.

The guide nucleic acid has at least 70%, 75%, 80%, 85%, 90% or 95% or more complementarity or complete complementarity.

The guide domain may comprise complementary nucleotide sequence with the guide nucleic acid binding sequence of the target sequence of the SPT4 gene. Here, the complementary nucleotide sequence may create 0 to 5, 0 to 4, 0 to 3, 0 to 2 mismatches.

The guide domain may comprise complementary nucleotide sequence with the guide nucleic acid binding sequence of the target sequence of the SPT5 gene. Here, the complementary nucleotide sequence may create 0 to 5, 0 to 4, 0 to 3, 0 to 2 mismatches.

The guide domain may comprise complementary nucleotide sequence with the guide nucleic acid binding sequence of the target sequence of the SUPT4H gene. Here, the complementary nucleotide sequence may create 0 to 5, 0 to 4, 0 to 3, 0 to 2 mismatches.

The guide domain may comprise complementary nucleotide sequence with the guide nucleic acid binding sequence of the target sequence of the SUPT5H gene. Here, the complementary nucleotide sequence may create 0 to 5, 0 to 4, 0 to 3, 0 to 2 mismatches.

The guide nucleic acid includes one or more domains selected from the group consisting of a first complementary domain, a second complementary domain, a linker domain, a proximal domain, and a tail domain.
the CRISPR enzyme is may be one or more selected from the group consisting of a Streptococcus pyogenes-derived Cas9 protein, a Campylobacter jejuni-derived Cas9 protein, a Streptococcus thermophilus-derived Cas9 protein, a Streptococcus aureus-derived Cas9 protein, a Neisseria meningitidis-derived Cas9 protein, and a Cpfl protein. In one example, the CRISPR enzyme is may be a Campylobacter jejuni-derived Cas9 protein or a Streptococcus aureus-derived Cas9 protein.

The gRNA-CRISPR enzyme complex may add various artificial modification or manipulation to the SPT4, the SPT5, the SUPT4H, and/or SUPT5H gene, according to the type of gRNA and CRISPR enzyme.

For example, when the CRISPR enzyme is SpCas9 protein, the artificially modified or manipulated SPT4, SPT5, SUPT4H, and/or SUPT5H gene may include one or more modifications in a continuous 1bp to 50bp, 1bp to 40bp, 1bp to 30bp, preferabley, 1bp to 25bp base sequence adjacent to the 5' end and/or 3' end of the 5'-NGG-3'(N is A, T, G, or C) PAM sequence of the each of the target gene's target region, comprisig:
i) deletion of one or more nucleotides;
ii) a substitution with one or more nucleotides different from a wild-type gene; or
iii) combination of i) and ii)

For another example, when the CRISPR enzyme is CjCas9 protein, the artificially modified or edited SPT4, SPT5, SUPT4H, and/or SUPT5H gene may include one or more modifications in a continuous 1bp to 50bp, 1bp to 40bp, 1bp to 30bp, preferabley, 1bp to 25bp base sequence adjacent to the 5' end and/or 3' end of the NNNNRYAC-3' (each N is independently A, T, C or G, R is A or G, and Y is C or T)(PAM) sequence of the each of the target gene's target region, comprisig:
i) deletion of one or more nucleotides;
ii) a substitution with one or more nucleotides different from a wild-type gene; or
iii) combination of i) and ii)

For another example, when the CRISPR enzyme is StCas9 protein, the artificially modified or edited SPT4, SPT5, SUPT4H, and/or SUPT5H gene may include one or more modifications in a continuous 1bp to 50bp, 1bp to 40bp, 1bp to 30bp, preferabley, 1bp to 25bp base sequence adjacent to the 5' end and/or 3' end of the NNAGAAW-3' (each N is independently A, T, C or G, and W is A or T) PAM sequence of the each of the target gene's target region, comprisig:
i) deletion of one or more nucleotides;
ii) a substitution with one or more nucleotides different from a wild-type gene; or
iii) combination of i) and ii)

For example, when the CRISPR enzyme is NmCas9 protein, the artificially modified or edited SPT4, SPT5, SUPT4H, and/or SUPT5H gene may include one or more modifications in a continuous 1bp to 50bp, 1bp to 40bp, 1bp to 30bp, preferabley, 1bp to 25bp base sequence adjacent to the 5' end and/or 3' end of the 5'-NNNNGATT-3'(each N is independently A, T, C or G) PAM sequence of the each of the target gene's target region, comprisig:
i) deletion of one or more nucleotides;
ii) a substitution with one or more nucleotides different from a wild-type gene; or
iii) combination of i) and ii)

For another example, when the CRISPR enzyme is SaCas9 protein, the artificially modified or edited SPT4, SPT5, SUPT4H, and/or SUPT5H gene may include one or more modifications in a continuous 1bp to 50bp, 1bp to 40bp, 1bp to 30bp, preferabley, 1bp to 25bp base sequence adjacent to the 5' end and/or 3' end of the 5'-NNGRR(T)-3'(each N is independently A, T, C or G, R is A or G, and (T) PAM sequence of the each of the target gene's target region, comprisig:
i) deletion of one or more nucleotides;
ii) a substitution with one or more nucleotides different from a wild-type gene; or
iii) combination of i) and ii)

For another example, when the CRISPR enzyme is Cpfl protein, the artificially modified or edited SPT4, SPT5, SUPT4H, and/or SUPT5H gene may include one or more modifications in a continuous 1bp to 50bp, 1bp to 40bp, 1bp to 30bp, preferabley, 1bp to 25bp base sequence adjacent to the 5' end and/or 3' end of the 5'-TTN-3' (N is A, T, C or G) PAM sequence of the each of the target gene's target region, comprisig:
i) deletion of one or more nucleotides;
ii) a substitution with one or more nucleotides different from a wild-type gene; or
iii) combination of i) and ii)

The artificial manipulation of the SPT4, the SPT5, the SUPT4H, and/or the SUPT5H by the gRNA-CRISPR enzyme complex may lead to effects of knockout.

The artificial manipulation of the SPT4, the SPT5, the SUPT4H, and/or the SUPT5H gene by the gRNA-CRISPR enzyme complex may suppress an expression of protein which is encoded by the SPT4, the SPT5, the SUPT4H, and/or SUPT5H gene.

The artificial manipulation of the SPT4, the SPT5, the SUPT4H, and/or the SUPT5H by the gRNA-CRISPR enzyme complex may lead to effects of knockdown.

The guide domain may be at least 70%, 75%, 80%, 85%, 90%, 95% or more complementary or fully complementary to the guide nucleic acid sequence.

The guide domain may include a nucleotide sequence complementary to a guide nucleic acid binding sequence in a target sequence of an SPT4 gene, SPT5 gene, SUPT4H gene, and/or SUPT5H gene. In this case, the complementary nucleotide sequence may include 0 to 5, 0 to 4, 0 to 3, 0 to 2 mismatches.

The guide RNA may include a nucleic acid sequence complementarily binding with a gene or nucleic acid sequence to be targeted.

The guide RNA may include a crRNA including a sequence complementary to a gene or nucleic acid sequence to be targeted and a tracrRNA binding with a CRISPR enzyme.

In this case, the crRNA includes a guide sequence which is a part complementarily binding with a gene or nucleic acid sequence to be targeted. The guide sequence has a sequence complementary to a gene or nucleic acid sequence to be targeted, and may serve to recognize the gene or nucleic acid sequence to be targeted.

The size of the guide sequence may be 5 to 50 bps, but is not limited thereto.

The nucleic acid sequence of the guide sequence may include a nucleic acid sequence with 50 to 100% complementary to a sequence or position of a gene or nucleic acid to be targeted, but is not limited thereto.

In this case, the nucleic acid sequence of the guide sequence may include a nucleic acid sequence complementarily binding with a target gene, for example, SPT4 gene and/or SPT5 gene.

Further, the nucleic acid sequence of the guide sequence may include a nucleic acid sequence complementarily binding with a target gene, for example, SUPT4H gene and/or SUPT5H gene.

In addition, the crRNA includes a part having a sequence complementary to a portion of a tracrRNA, and accordingly, the crRNA may be partially complementary to the tracrRNA.

The guide RNA may be a dual guide RNA in which the crRNA and the tracrRNA are each separately present.

The guide RNA may be a single guide RNA in which the crRNA and the tracrRNA are connected to each other. In this case, the single guide RNA may include a linker.

Further, the guide RNA may include only a crRNA according to the type of CRISPR enzyme.

The guide RNA may include a chemical modification. In this case, the chemical modification may include those in which a phosphorothioate linkage, a locked nucleic acid(LNA), 2'-O-methyl 3'phosphorothioate (MS) or 2'-O-methyl 3'thioPACE (MSP) is modified in one or two more nucleic acids among nucleic acids including the guide RNA.

The guide RNA may be a guide RNA in which a partial sequence of the 5' end is truncated.

The guide RNA may design a nucleic acid sequence of the guide RNA according to the target gene or nucleic acid sequence.

The guide RNA may be included in a vector, and in this case, the vector may include a promoter suitable for the expression of the guide RNA.

The guide RNA may be an artificially synthesized guide RNA.

The CRISPR enzyme may be a nucleic acid having a sequence encoding the CRISPR enzyme.

A nucleic acid having the sequence encoding the CRISPR enzyme may be included in a vector. In this case, the vector may include a promoter suitable for the expression of a CRISPR enzyme, such as CMV or CAG.

The CRISPR enzyme may be a polypeptide or protein.

The CRISPR enzyme may be codon-optimized so as to be suitable for a subject to be introduced.

The CRISPR enzyme may be a Type II CRISPR enzyme or a Type V CRISPR enzyme.

The Type II CRISPR enzyme may be a Cas9 enzyme.

The Type V CRISPR enzyme may be a Cpfl enzyme.

The Cas9 enzyme may be Streptococcus pyogenes Cas9 (SpCas9), Staphylococcus aureus Cas9 (SaCas9), Streptococcus thermophiles Cas9 (StCas9), Neisseria meningitides Cas9 (NmCas9), Campylobacter jejuni Cas9 (CjCas9) or orthologs thereof, but is not limited thereto. Preferably, the Cas9 enzyme may be Streptococcus pyogenes Cas9 (SpCas9) or Campylobacter jejuni Cas9 (CjCas9).

The Cas9 enzyme may be an active Cas9 enzyme or an inactive Cas9 enzyme.

The inactive Cas9 enzyme may include a completely inactivated Cas9 enzyme and a partially inactivated Cas9 enzyme (for example, nickase).

With respect to the Cas9 enzyme, one, two or more amino acids present in RuvC, HNH, REC and/or PI domains may be mutated.

The Cas9 enzyme may include the mutation of one or two or more amino acids in an amino acid group consisting of D10, E762, H840, N854, N863, and D986 among amino acids of SpCas9 or an amino acid group of other Cas9 orthologs corresponding thereto.

The Cas9 enzyme may include the mutation of one or two or more amino acids in an amino acid group consisting of R780, K810, K848, K855 and H982 among amino acids of SpCas9 or an amino acid group of other Cas9 orthologs corresponding thereto.

The Cas9 enzyme may include the mutation of one or two or more amino acids in an amino acid group consisting of G1104, S1109, L1111, D1135, S1136, G1218, N1317, R1335 and T1337 among amino acids of SpCas9 or an amino acid group of other Cas9 orthologs corresponding thereto.

The Cpfl enzyme may be Francisella novicida Cpfl (FnCpf1), Acidaminococcus sp. Cpfl (AsCpf1), Lachnospiraceae bacterium Cpfl (LbCpf1) or orthologs thereof, but is not limited thereto.

The Cpfl enzyme may be an active Cpfl enzyme or an inactive Cpfl enzyme.

The inactive Cpfl enzyme may include a completely inactivated Cpfl enzyme and a partially inactivated Cpfl enzyme (for example, nickase).

With respect to the Cpfl enzyme, one, two or more amino acids present in RuvC, Nuc, WED, REC and/or PI domains may be mutated.

The Cpfl enzyme may include the mutation of one or more amino acids in D917, E1006 or D1255 among amino acids of FnCpf1; D908, E993 or D1263 among amino acids of AsCpf1; D832, E925, D947 or D1180 among amino acids of LbCpf1; or an amino acid group of other Cpfl orthologs corresponding thereto.

The CRISPR enzyme can recognize a protospacer adjacent motif (PAM) in a gene or nucleic acid sequence.

The PAM may vary according to the source of the CRISPR enzyme.

For example, the PAM may be 5'-NGG-3' when the CRISPR enzyme is SpCas9, the PAM may be 5'-NNAGAAW-3' (W = A or T) when the CRISPR enzyme is StCas9, the PAM may be 5'-NNNNGATT-3' when the CRISPR enzyme is NmCas9, the PAM may be 5'-NNNVRYAC-3' (V = G or C or A, R = A or G, Y = C or T) when the CRISPR enzyme is CjCas9, and in this case, the N may be A, T, G or C; or A, U, G or C. Furthermore, the PAM may be 5' TTN-3' when the CRISPR enzyme is FnCpf1, the PAM may be 5'-TTTN-3' when the CRISPR enzyme is AsCpf1 or LbCpf1, and in this case, the N may be A, T, G or C; or A, U, G or C.

The CRISPR enzyme may additionally include a functional domain. In this case, the CRISPR enzyme may be an active CRISPR enzyme or an inactive CRISPR enzyme.

The functional domain may be a heterologous functional domain (HFD).

The functional domain may be selected from the group consisting of methylase activity, demethylase activity, transcription activation activity, transcription repression activity, transcription release factor activity, histone modification activity, RNA cleavage activity, DNA cleavage activity, nucleic acid binding activity and molecular switches (e.g., light inducible).

The functional domain may be selected from the group consisting of methylases, demethylases, phosphases, thymidine kinase, cysteine deaminase, and cytidine deaminase.

In order to link the functional domain to the CRISPR enzyme, a linker between the CRISPR enzyme and the functional domain may be additionally included.

The linker may be (A)ₙ, (G)ₙ, GGGS, (GGS)ₙ, (GGGGS)ₙ, (EAAAK)ₙ, SGGGS, GGSGGSGGS, SGSETPGTSESATPES, XTEN or (XP)ₙ, and in this case, n may be 1, 2, 3, 4, 5, 6, 7, or higher. However, the linker and n are not limited thereto.

The CRISPR enzyme may additionally include a nuclear localization sequence (NLS).

The CRISPR enzyme and the guide RNA may form a CRISPR complex.

The CRISPR complex may be formed outside a cell.

The CRISPR complex may be formed in the cytoplasm in a cell.

The CRISPR complex may be formed in the nucleus in a cell.

In the CRISPR complex, the CRISPR enzyme can recognize a PAM present in a gene or nucleic acid sequence to be targeted.

In the CRISPR complex, a guide RNA may complimentarily bind with respect to a gene or nucleic acid sequence to be targeted.

When the CRISPR complex binds with a gene or nucleic acid sequence to be targeted, the gene or nucleic acid sequence to be targeted can be cleaved or modified by the CRISPR enzyme of the CRISPR complex.

In another embodiment, a CRISPR-Cas system may be present in the form of a ribonucleoprotein (RNP) in which a guide RNA and a CRISPR enzyme form a complex.

The target-specific gene scissors may be introduced into a cell or tissue by a publicly known method.

The introduction of the target-specific gene scissors into the cell can be carried out by transfection using a viral vector system, a ribonucleoprotein (RNP), nanoparticles, a liposome, and the like, microinjection, electroporation, and the like, but the introduction method is not limited thereto.

For example, the introduction method include calcium phosphate-mediated transfection, nucleofection, electroporation, cationic polymer transfection (for example, DEAE-dextran or polyethyleneimine), viral transfection, virosome transfection, virion transfection, liposome transfection, cationic liposome transfection, immunoliposome transfection, non-liposome transfection, dendrimer transfection, heat shock transfection, magnetofection, lipofection, gene gun delivery, impalefection, sonoporation, optical transfection, and proprietary agent-enhanced uptake of nucleic acids.

The cells may be eukaryotic cells or prokaryotic cells, and may be preferably eukaryotic cells.

The eukaryotic cells may be plant, animal, or human cells, and may be preferably human cells.

The tissue may be an animal or human body tissue such as skin, the liver, kidneys, heart, lungs, brain, and muscle.

A CRISPR-Cas system may be codon-optimized. The CRISPR-Cas system may include a coding for at least one nuclear localization signal (NLS); or may include at least one NLS.

Further, the therapeutic agent may additionally include a pharmaceutically acceptable carrier or adjuvant.

The term "subject" refers to an organism into which a guide nucleic acid, editor protein or guide nucleic acid-editor protein complex is introduced, an organism in which a guide nucleic acid, editor protein or guide nucleic acid-editor protein complex operates, or a specimen or sample obtained from the organism.

The subject may be an organism including a target nucleic acid, gene, chromosome or protein of the guide nucleic acid-editor protein complex.

The organism may be may be an animal, an animal organ, an animal tissue, or an animal cell.

The organism may be may be a human, a human organ, a human tissue, or a human cell.

The organ may be: the kidneys; a digestive system including the stomach, pancreas, duodenum, ileum, and/or colon; the heart; the lungs; the brain, particularly neuron, and/or generally the CNS; eyes including retinal tissues; ears including the inner ear; skin; muscle; bone; and/or liver.

The tissue may be a tissue such as eyeball, skin, liver, kidney, heart, lung, brain, muscle or blood tissue.

The cells may be nerve cells, myocytes, hemocytes, immunocytes, adipocytes, osteocytes, germ cells, skin cells, or stem cells thereof.

Preferably, the subject may be an organism including an expression regulatory gene of an over-amplified repeated sequence.

The guide nucleic acid may be delivered or introduced into a subject in the form of DNA, RNA or a mixed form.

The form of DNA, RNA or a mixture thereof, which encodes the guide nucleic acid and/or editor protein may be delivered or introduced into a subject by a method known in the art.

Or, the form of DNA, RNA or a mixture thereof, which encodes the guide nucleic acid and/or editor protein may be delivered or introduced into a subject by a vector, a non-vector or a combination thereof.

The vector may be a viral or non-viral vector (e.g., a plasmid).

The non-vector may be naked DNA, a DNA complex or mRNA.

In one embodiment, the nucleic acid sequence encoding the guide nucleic acid and/or editor protein may be delivered or introduced into a subject by a vector.

The vector may include a nucleic acid sequence encoding a guide nucleic acid and/or editor protein.

For example, the vector may simultaneously include nucleic acid sequences, which encode the guide nucleic acid and the editor protein, respectively.

For example, the vector may include the nucleic acid sequence encoding the guide nucleic acid.

As an example, domains included in the guide nucleic acid may be contained all in one vector, or may be divided and then contained in different vectors.

For example, the vector may include the nucleic acid sequence encoding the editor protein.

In one example, in the case of the editor protein, the nucleic acid sequence encoding the editor protein may be contained in one vector, or may be divided and then contained in several vectors.

The vector may include one or more regulatory/control components.

Here, the regulatory/control components may include a promoter, an enhancer, an intron, a polyadenylation signal, a Kozak consensus sequence, an internal ribosome entry site (IRES), a splice acceptor and/or a 2A sequence.

The promoter may be a promoter recognized by RNA polymerase II.

The promoter may be a promoter recognized by RNA polymerase III.

The promoter may be an inducible promoter.

The promoter may be a subject-specific promoter.

The promoter may be a viral or non-viral promoter.

The promoter may use a suitable promoter according to a control region (that is, a nucleic acid sequence encoding a guide nucleic acid or editor protein).

For example, a promoter useful for the guide nucleic acid may be a HI, EF-1a, tRNA or U6 promoter. For example, a promoter useful for the editor protein may be a CMV, EF-1a, EFS, MSCV, PGK or CAG promoter.

The vector may be a viral vector or recombinant viral vector.

The virus may be a DNA virus or an RNA virus.

Here, the DNA virus may be a double-stranded DNA (dsDNA) virus or single-stranded DNA (ssDNA) virus.

Here, the RNA virus may be a single-stranded RNA (ssRNA) virus.

The virus may be a retrovirus, a lentivirus, an adenovirus, adeno-associated virus (AAV), vaccinia virus, a poxvirus or a herpes simplex virus, but the present invention is not limited thereto.

Generally, the virus may infect a host (e.g., cells), thereby introducing a nucleic acid encoding the genetic information of the virus into the host or inserting a nucleic acid encoding the genetic information into the host genome. The guide nucleic acid and/or editor protein may be introduced into a subject using a virus having such a characteristic. The guide nucleic acid and/or editor protein introduced using the virus may be temporarily expressed in the subject (e.g., cells). Alternatively, the guide nucleic acid and/or editor protein introduced using the virus may be continuously expressed in a subject (e.g., cells) for a long time (e.g., 1, 2 or 3 weeks, 1, 2, 3, 6 or 9 months, 1 or 2 years, or permanently).

The packaging capability of the virus may vary from at least 2 kb to 50 kb according to the type of virus. Depending on such a packaging capability, a viral vector including a guide nucleic acid or an editor protein or a viral vector including both of a guide nucleic acid and an editor protein may be designed. Alternatively, a viral vector including a guide nucleic acid, an editor protein and additional components may be designed.

In one example, a nucleic acid sequence encoding a guide nucleic acid and/or editor protein may be delivered or introduced by a recombinant lentivirus.

In another example, a nucleic acid sequence encoding a guide nucleic acid and/or editor protein may be delivered or introduced by a recombinant adenovirus.

In still another example, a nucleic acid sequence encoding a guide nucleic acid and/or editor protein may be delivered or introduced by recombinant AAV.

In yet another example, a nucleic acid sequence encoding a guide nucleic acid and/or editor protein may be delivered or introduced by a hybrid virus, for example, one or more hybrids of the virus listed herein.

A nucleic acid sequence encoding a guide nucleic acid and/or editor protein may be delivered or introduced into a subject using a non-vector.

The non-vector may include a nucleic acid sequence encoding a guide nucleic acid and/or editor protein.

The non-vector may be naked DNA, a DNA complex, mRNA, or a mixture thereof.

The non-vector may be delivered or introduced into a subject by electroporation, particle bombardment, sonoporation, magnetofection, transient cell compression or squeezing (e.g., described in the literature [Lee, et al, (2012) Nano Lett., 12, 6322-6327]), lipid-mediated transfection, a dendrimer, nanoparticles, calcium phosphate, silica, a silicate (Ormosil), or a combination thereof.

As an example, the delivery through electroporation may be performed by mixing cells and a nucleic acid sequence encoding a guide nucleic acid and/or editor protein in a cartridge, chamber or cuvette, and applying electrical stimuli with a predetermined duration and amplitude to the cells.

In another example, the non-vector may be delivered using nanoparticles. The nanoparticles may be inorganic nanoparticles (e.g., magnetic nanoparticles, silica, etc.) or organic nanoparticles (e.g., a polyethylene glycol (PEG)-coated lipid, etc.). The outer surface of the nanoparticles may be conjugated with a positively-charged polymer which is attachable (e.g., polyethyleneimine, polylysine, polyserine, etc.).

In a certain embodiment, the non-vector may be delivered using a lipid shell.

In a certain embodiment, the non-vector may be delivered using an exosome. The exosome is an endogenous nano-vesicle for transferring a protein and RNA, which can deliver RNA to the brain and another target organ.

In a certain embodiment, the non-vector may be delivered using a liposome. The liposome is a spherical vesicle structure which is composed of single or multiple lamellar lipid bilayers surrounding internal aqueous compartments and an external, lipophilic phospholipid bilayer which is relatively non-transparent. While the liposome may be made from several different types of lipids; phospholipids are most generally used to produce the liposome as a drug carrier.

In addition, a composition to deliver non-vector may includes other additives.

The editor protein may be delivered or introduced into a subject in the form of a peptide, polypeptide or protein.

The editor protein in the form of a peptide, polypeptide or protein may be delivered or introduced into a subject by a method known in the art

The peptide, polypeptide or protein form may be delivered or introduced into a subject by electroporation, microinjection, transient cell compression or squeezing (e.g., described in the literature [Lee, et al, (2012) Nano Lett., 12, 6322-6327]), lipid-mediated transfection, nanoparticles, a liposome, peptide-mediated delivery or a combination thereof.

The peptide, polypeptide or protein may be delivered with a nucleic acid sequence encoding a guide nucleic acid.

In one example, the transfer through electroporation may be performed by mixing cells into which the editor protein will be introduced with or without a guide nucleic acid in a cartridge, chamber or cuvette, and applying electrical stimuli with a predetermined duration and amplitude to the cells.

The guide nucleic acid and the editor protein may be delivered or introduced into a subject in the form of nucleic acid-protein mixture.

The guide nucleic acid and the editor protein may be delivered or introduced into a subject in the form of a guide nucleic acid-editor protein complex.

For example, the guide nucleic acid may be DNA, RNA or a mixture thereof. The editor protein may be a peptide, polypeptide or protein.

In one example, the guide nucleic acid and the editor protein may be delivered or introduced into a subject in the form of a guide nucleic acid-editor protein complex containing an RNA-type guide nucleic acid and a protein-type editor protein, that is, a ribonucleoprotein (RNP).

The content disclosed in the present specification may use a "gene editing" technology for gene manipulation of an expression regulatory gene of an over-amplified repeated sequence. As the most preferred embodiment of the gene editing technology, a CRISPR-Cas system may be used.

The gene manipulation of the expression regulatory gene of the over-amplified repeated sequence may be used for the above-described genetic diseases caused by the over-amplified repeated sequence.

In the content disclosed in the present specification, "therapeutic agent for a genetic disease caused by an over-amplified repeated sequence" is a concept including all of a gene editing technology for the treatment of the above diseases, a material which may be used to use, particularly, a CRISPR-Cas system, a composition containing the same, and a system using the same.

Therefore, the therapeutic agent of the content disclosed in the present specification includes target-specific gene scissors, and the "target-specific gene scissors" refers to a nuclease which can recognize and cleave a specific position of a nucleic acid (DNA or RNA) on a desired (targeted or target) genome. In the content disclosed in the present specification, the target-specific gene scissors are also used as a concept including other elements required to enable a specific nuclease to perform a desired function.

The target-specific gene scissors are characterized by targeting a gene directly or indirectly involved in the expression of an over-amplified repeated sequence. Genes targeted in the content disclosed in the specification include all genes involved in the regulation of the expression of the over-amplified repeated sequence.

The target of the target-specific gene scissors may be, for example, an SPT4 gene and/or SPT5 gene.

The target of the target-specific gene scissors may be, for example, SUPT4H gene and/or SUPT5H gene.

For the targets, the expression of the gene may be regulated by the action of the target gene scissors of the content disclosed in the present specification.

For example, the expression of genes positively involved in the expression of the over-amplified repeated sequence may be reduced or suppressed.

As another example, the expression of genes negatively involved in the expression of the over-amplified repeated sequence may be increased or promoted.

The target-specific gene scissors disclosed in the present specification and a composition including the same may be designed and changed such that the expression of the target genes is regulated so as to be suitable for the purpose.

A guide nucleic acid-editor protein complex may modify a target nucleic acid, gene, or chromosome.

For example, a guide nucleic acid-editor protein complex induces modification at a sequence of a target nucleic acid, gene, or chromosome. As a result, a protein expressed by the target nucleic acid, gene, or chromosome can be modified in structure and/or function, or the expression of the protein can be regulated or removed.

Here, the guide nucleic acid-editor protein complex may act at a DNA, RNA, gene or chromosomal level.

For example, the guide nucleic acid-editor protein complex may regulate (e.g., inhibit, suppress, reduce, increase or promote) the expression of a protein encoded by target gene, regulate (e.g., inhibit, suppress, reduce, increase or promote) protein activity, or express a modified protein through manipulation or modification of the target gene.

The guide nucleic acid-editor protein complex may act at gene transcription and translation stages.

In one example, the guide nucleic acid-editor protein complex may promote or suppress the transcription of a target gene, thereby regulating (e.g., inhibiting, suppressing, reducing, increasing or promoting) the expression of a protein encoded by the target gene.

In another example, the guide nucleic acid-editor protein complex may promote or suppress the translation of a target gene, thereby regulating (e.g., inhibiting, suppressing, reducing, increasing or promoting) the expression of a protein encoded by the target gene.

According to an embodiment disclosed in the present specification, the expression of the gene characterized by an over-amplified repeated sequence may be regulated by the composition for gene manipulation.

The gene characterized by the over-amplified repeated sequence may have a repeated sequence over-amplified in one region of the gene.

The gene may be, for example, any one or more of an HTT gene, FMR1 gene, FMR2 gene, FRDA gene, DMPK/SIX gene, ZNF9 gene, SCA8 gene, ATXN10 gene, PPP2R2B gene, CSTB gene, TCF4 gene, C9orf72 gene, AIB1 gene, KCNN3 gene, CBFA1 gene, COMP gene, AR gene, JPH3 gene, DRPLA gene, ATXN1 gene, ATXN2 gene, ATXN3 gene, CACNA1A gene, ATXN7 gene, TBP gene, PABPN1 gene, HOXD13 gene, RUNX2 gene, HOXA13 gene, ZIC2 gene, FOXL2 gene, PHOX2B gene, SOX3 gene, and ARX gene, but is not limited thereto.

The one region of the gene may be, for example, any one or more of an exon, an intron, a 3'-UTR, a 5'-UTR, and a polyadenylation signal sequence, but is not limited thereto.

The over-amplified repeated sequence includes:
a first repeated part including duplication of the repeated sequence; and
a second repeated part including over-duplication of the repeated sequence,
the first repeated part is included in a diseased subject and a normal subject, and
the second repeated part is not included in a normal subject.

The first repeated part and the second repeated part may be nucleic acid sequences encoding the repetition of a certain amino acid sequence.

A polypeptide having the repetition of the certain amino acid sequence may be translated from the first repeated part and the second repeated part.

The composition for gene manipulation may artificially manipulate a gene which regulates the expression of an over-amplified repeated sequence.

The gene which regulates the expression of the over-amplified repeated sequence may be one or more selected from the group consisting of an SPT4 gene, SPT5 gene, SUPT4H gene, and SUPT5H gene.

The composition for gene manipulation may reduce the expression of the gene which regulates the expression of the over-amplified repeated sequence. For example, the composition for gene manipulation may reduce the expression of any one or more of the SPT4 gene, SPT5 gene, SUPT4H gene, and SUPT5H gene.

The reduction in expression of the gene which regulates the expression of the over-amplified repeated sequence may be a reduction in expression of a transcription elongation factor for the over-amplified repeated sequence. For example, the reduction in expression of any one or more of the SPT4 gene, SPT5 gene, SUPT4H gene, and SUPT5H gene may be a reduction in expression of a transcription elongation factor for the over-amplified repeated sequence.

The reduction in expression of the gene which regulates the expression of the over-amplified repeated sequence may reduce the expression of the over-amplified repeated sequence. The reduction in expression of the gene which regulates the expression of the over-amplified repeated sequence may reduce the expression of the second repeated part in the over-amplified repeated sequence. The reduction in expression of the gene which regulates the expression of the over-amplified repeated sequence may reduce the transcription of the second repeated part in the over-amplified repeated sequence.

For example, the reduction in expression of any one or more of the SPT4 gene, SPT5 gene, SUPT4H gene, and SUPT5H gene may reduce the expression of the over-amplified repeated sequence. The reduction in expression of any one or more of the SPT4 gene, SPT5 gene, SUPT4H gene, and SUPT5H gene may reduce the expression of the second repeated part in the over-amplified repeated sequence. The reduction in expression of any one or more of the SPT4 gene, SPT5 gene, SUPT4H gene, and SUPT5H gene may reduce the transcription of the second repeated part in the over-amplified repeated sequence.

The reduction in expression of the second repeated part in the over-amplified repeated sequence may reduce the transcription of an mRNA from a nucleic acid sequence.

As an embodiment disclosed by the present specification, manipulated animal cells are provided.

The manipulated animal cells may be animal cells in which the expression of a specific gene including an over-amplified repeated sequence is inhibited or suppressed.

The manipulated animal cells may be animal cells in which the expression of an expression regulatory factor for an over-amplified repeated sequence is inhibited or suppressed.

The manipulated animal cells may be artificially manipulated animal cells including an expression regulatory factor for one or more artificially manipulated or modified over-amplified repeated sequences and/or expression products thereof.

The expression product may be an mRNA or protein expressed by an expression regulatory factor for one or more artificially manipulated or modified over-amplified repeated sequences.

In this case, the expression regulatory factor for the over-amplified repeated sequence may be a polypeptide or protein expressed by an expression regulatory gene of an over-amplified repeated sequence, that is, the SPT4 gene, SPT5 gene, SUPT4H gene, or SUPT5H gene.

As another embodiment disclosed by the present specification, a manipulated animal subject including manipulated animal cells is provided.

The manipulated animal subject may be a manipulated animal subject including the manipulated animal cells in one region or the entire region of an organ.

The manipulated animal subject may be a manipulated animal subject including the manipulated animal cells in which an expression of the expression regulatory factor for an over-amplified repeated sequence is inhibited or suppressed in one region or the entire region of an organ.

According to an embodiment disclosed in the present specification, a use of a composition for gene manipulation, including a guide nucleic acid capable of forming bonds with a target sequence of an expression regulatory gene of an over-amplified gene may be provided.

An embodiment disclosed by the present specification provides a pharmaceutical composition to be used for treating a disease caused by an over-amplified repeated sequence using the composition for gene manipulation.

The content disclosed in the present specification provides a use of a CRISPR-Cas composition for gene (genome) manipulation, for treating the disease, or preparing a drug or pharmaceutical composition.

Provided is a use of a sequence, vector, enzyme, or system of the content disclosed in the present specific in medicine. Further, also provided is a use of those described above in the gene or genome editing.

In addition, the content disclosed in the present specification provides a method for alleviating or treating a genetic disease caused by a specific gene including an over-amplified repeated sequence, using target-specific gene scissors.

The treatment method is characterized by using target-specific gene scissors targeting an expression regulatory gene of an over-amplified repeated sequence.

The treatment method is characterized by including introducing target-specific gene scissors into cells including an expression regulatory gene of an over-amplified repeated sequence to be targeted.

The treatment method is characterized by including introducing target-specific gene scissors into cells including a specific gene including an over-amplified repeated sequence.

For example, target-specific gene scissors may be introduced using an adeno-associated virus (AAV), a lentivirus, an adeno virus, or other plasmid or viral vector types.

Further, the content disclosed in the present specification provides a composition or kit for regulating the expression of a specific gene including an over-amplified repeated sequence.

In this case, the composition or kit includes target-specific gene scissors, in which the target-specific gene scissors are characterized by targeting a gene involved in the expression of an over-amplified repeated sequence.

Further, the composition may additionally include a pharmaceutically acceptable carrier or adjuvant.

According to another aspect disclosed by the present specification, a method for treating a repeat expansion disorder may be provided.

An embodiment disclosed by the present specification is a method for treating a repeat expansion disorder, the method including:
administering, to a subject to be treated, a composition including a composition for gene manipulation to reduce an expression of an over-amplified repeated sequence as an active ingredient,
in which the composition for gene manipulation includes any one of a clustered regularly interspaced short palindromic repeats (CRISPR)-CRISPR associated protein (Cas) system, a zinc finger nuclease (ZFN), a transcription activator-like effector nuclease(TALEN), FokI, and an endonuclease, and
the gene is characterized by being one or more genes selected from the group consisting of an SPT4 gene, SPT5 gene, SUPT4H gene, and SUPT5H gene.

Another embodiment disclosed by the present specification is a method for treating a repeat expansion disorder, the method including:
administering, to a subject to be treated, a composition including a composition for gene manipulation to reduce the expression of an over-amplified repeated sequence as an active ingredient,
in which the composition for gene manipulation is a composition for gene manipulation, including:
   a guide nucleic acid for target sequences of one or more genes selected from the group consisting of an SPT4 gene, SPT5 gene, SUPT4H gene, and SUPT5H gene; and
   one or more editor proteins selected from the group consisting of a Streptococcus pyogenes-derived Cas9 protein, a Campylobacter jejuni-derived Cas9 protein, a Streptococcus thermophilus-derived Cas9 protein, a Streptocuccus aureus-derived Cas9 protein, a Neisseria meningitidis-derived Cas9 protein, and a Cpfl protein, or a nucleic acid encoding the editor protein.

For the administration, the administering of the composition to the subject to be treated may be a method of electroporation, injection, transfusion, implantation or transplantation.

The administration may be administering the composition one or more of subcutaneously, intradermally, intratumorally, intranodally, intramedullarily, intramuscularly, intravenously, intralymphatically, and intraperitoneally, but the administration route is not limited thereto.

For the administration, the composition may be administered locally. Further, for the administration, the composition may be administered topically.

The target sequence of the guide nucleic acid may be one or more selected from SEQ ID Nos. 1 to 24.

The subject to be treated may be mammals including primates such as humans and monkeys, rodents such as rats, and the like.

For the administration, the composition may be administered to any one or more organs of the kidneys; a digestive system including the stomach, pancreas, duodenum, ileum, and/or colon; the heart; the lungs; the brain, particularly neurons, and/or generally the CNS; eyes including retinal tissues; ears including the inner ear; skin; muscle; bone; and/or the liver of the subject to be treated, but the organ is not limited thereto.

The cells of the subject to be treated may be any one or more of nerve cells, myocytes, hemocytes, immunocytes, adipocytes, osteocytes, germ cells, skin cells, or stem cells thereof, but are not limited thereto.

For the administration, the composition may be administered to an organ of the subject to be treated. For example, the organ may be the brain. In the brain, other tissues including any one or more of the hippocampal dentate gyrus, visual cortex, primary motor cortex, primary auditory cortex, primary somatosensory cortex, cerebellum, cerebellum, main olfactory bulb, frontal substantia nigralobe cortex, endopiriform nucleus, tonsils, substantia nigra, corpus striatum, pallidum, thalamus, hypothalamus, parabrachial nucleus, superior olivary complex, cochlear nucleus, and mammillary nucleus may be preferred in some embodiments.

Cells from the brain may be nerve cells or glial cells. The nerve cells may be neurons, and the glial cells may be astrocytes, oligodendrocytes, Schwann cells, olfactory ensheathing cells, ependymal cells, or satellite cells, but are not limited thereto.

The composition for gene manipulation may be administered by one or more methods of a ribonucleoprotein (RNP), a liposome, a plasmid, viral vector, nanoparticles, and a protein translocation domain (PTD) fusion protein method.

The composition for gene manipulation to be administered by the method may be, for example, a guide nucleic acid and/or editor protein, or a nucleic acid encoding the editor protein.

The composition for gene manipulation may be administered in vivo.

The guide nucleic acid may be delivered to cells in the form of DNA or RNA.

The editor protein may be delivered to cells in the form of a polypeptide.

The nucleic acid encoding the editor protein may be delivered to cells in the form of DNA.

Among the forms, the nucleic acid form may be included in a vector system including one or more viral vectors.

The viral vector may be one or more selected from the group consisting of a retrovirus, a lentivirus, an adenovirus, an adeno-associated virus (AAV), a vaccinia virus, a poxvirus, and a herpes simplex virus, but is not limited thereto.

Still another embodiment disclosed by the present specification is a method for treating a repeat expansion disorder, the method including:
administering, to a subject to be treated, a composition including a composition for gene manipulation to reduce the expression of an over-amplified repeated sequence as an active ingredient; and
contacting the composition for gene manipulation with cells of the subject to be treated;

The contact may be contacting:
(a) cells of a subject to be treated; and
(b) a composition for gene manipulation capable of artificially manipulating one or more expression regulatory genes of an over-amplified repeated sequence selected from the group consisting of an SPT4 gene, SPT5 gene, SUPT4H gene, and SUPT5H gene.

In this case, the (a) cells of the subject to be treated may be nerve cells, myocytes, hemocytes, immunocytes, adipocytes, osteocytes, germ cells, skin cells, or stem cells thereof. The cells of the subject to be treated may be derived from the human body.

The (b) composition for gene manipulation may include:
(b') guide nucleic acids for target sequences for one or more expression regulatory genes of an over-amplified repeated sequence selected from the group consisting of the SPT4 gene, SPT5 gene, SUPT4H gene, and SUPT5H gene; and
(b") one or more editor proteins selected from the group consisting of a Streptococcus pyogenes-derived Cas9 protein, a Campylobacter jejuni-derived Cas9 protein, a Streptococcus thermophilus-derived Cas9 protein, a Streptocuccus aureus-derived Cas9 protein, a Neisseria meningitidis-derived Cas9 protein, and a Cpfl protein.

The description for the composition for genetic recombination is as described above.

The target sequence of the guide nucleic acid may be one or more selected from SEQ ID Nos. 1 to 24.

The contacting step may be carried out in vivo.

The contacting step may include introducing (b) a composition for gene manipulation into (a) animal cells.

The method may be carried out in vivo or ex vivo, for example, in the human body.

The guide nucleic acid may be brought into contact with cells in the form of DNA or RNA.

The editor protein may be brought into contact with cells in the form of a polypeptide.

The nucleic acid encoding the editor protein may be brought into contact with cells in the form of DNA.

Among the forms, the nucleic acid form may be included in a vector system including one or more viral vectors.

The viral vector may be one or more selected from the group consisting of a retrovirus, a lentivirus, an adenovirus, an adeno-associated virus (AAV), a vaccinia virus, a poxvirus, and a herpes simplex virus, but is not limited thereto.

The animal cells used for the method may be animal cells derived from mammals including primates such as humans and monkeys and rodents such as mice and rats.

For the treatment of a disease through the above-described gene manipulation or editing, there may be a treatment method affecting the expression of a gene of animal cells by directly manipulating a gene of an organism. The treatment method may be performed by directly injecting a composition for gene manipulation to manipulate a gene of an organism in vivo.

When the composition for gene manipulation is directly injected in vivo, in an embodiment of an administration amount of the composition for gene manipulation,
a single dose of the composition (a pharmaceutically effective amount for obtaining a desired effect) is for a human, and when the composition is delivered in vivo through AAV, about 20 ml to about 50 ml of a saline solution containing about 1 x 10⁸ to about 1 x 10¹⁸ functional AAV/ml may be administered. In an embodiment of the present specification, an AAV dose is generally within a concentration range of about 1 x 10⁵ to 1 x 10⁵⁰ genomic AAV, about 1 x 10⁸ to 1 x 10²⁰ genomic AAV, about 1 x 10¹⁰ to about 1 x 10¹⁶ genomic AAV, or about 1 x 10¹¹ to about 1 x 10¹⁶ genomic AAV. A human dose may be about 1 x 10¹³ genomic AAV. The concentration may be delivered in about 0.001 ml to about 100 ml, about 0.05 ml to about 50 ml, or about 10 ml to about 25 ml of a carrier solution. The concentration may be selected from all the integer values within the numerical ranges, but is not limited thereto, and the composition may be prescribed appropriately in consideration of the age, health, and body weight of a subject to be administered, type of concurrent treatment, if any, frequency of treatment, characteristics of a desired effect, and the like.

When the composition for gene manipulation is directly injected in vivo, in another embodiment of the administration amount of the composition for gene manipulation,
a single dose of the composition (a pharmaceutically effective amount for obtaining a desired effect) is for a human, and when the composition is delivered in vivo through RNP, a dose of about 0.01 to about 1 mg of the composition administered in vivo per kg of body weight may be contemplated. For example, a dose of about 0.01 to 0.05 mg, 0.05 to 0.10 mg, 0.10 to 0.20 mg, 0.20 to 0.30 mg, 0.30 to 0.50 mg, 0.50 to 0.70 mg, or 0.70 to 1 mg of the composition administered in vivo per kg of body weight may be administered. The concentration may be selected from all the integer values within the numerical ranges, but is not limited thereto, and the composition may be prescribed appropriately in consideration of the age, health, and body weight of a subject to be administered, type of concurrent treatment, if any, frequency of treatment, characteristics of a desired effect, and the like.

The administration amount may further contain, for example, a carrier (water, saline, ethanol, glycerol, lactose, sucrose, calcium phosphate, gelatin, dextran, agar, pectin, peanut oil, sesame oil, and the like), a diluent, a pharmaceutically-acceptable carrier (for example, phosphate-buffered saline), a pharmaceutically-acceptable excipient, an adjuvant to enhance antigenicity, an immunostimulatory compound or molecule, and/or other compounds known in the art. The adjuvant of the present specification may include immunoregulatory molecules, such as cytokines, costimulatory molecules, and for example, immunoregulatory DNA or RNA molecules, such as CpG oligonucleotides. Such a dosage formulation is readily ascertainable by the person skilled in the art.

The dosage may further contain one or more pharmaceutically acceptable salts, for example, an inorganic salt such as hydrochloric acid, hydrobromic acid, phosphates, sulfates, and the like; and salts of organic acid, for example, acetates, propionates, malonates, benzoates, and the like. Additionally, the dosage may contain an auxiliary substance, for example, a wetting or emulsifying agent, a pH buffering substance, a gel or gelling material, a flavoring agent, a colorant, a microsphere, a polymer, a suspending agent, and the like. Additionally, when the dosage form is in a reconstitutable form, one or more typical pharmaceutical ingredients, for example, a preservative, a wetting agent, a suspending agent, a surfactant, an antioxidant, an anticaking agent, a filler, a chelating agent, a coating agent, a chemical stabilizer, and the like may also be present. Suitable illustrative ingredients include microcrystalline cellulose, sodium carboxymethyl cellulose, polysorbate 80, phenylethyl alcohol, chlorobutanol, potassium sorbate, sorbic acid, sulfur dioxide, propyl gallate, parabens, ethylvanillin, glycerin, phenol, parachlorophenol, gelatin, albumin, and a combination thereof.

Still another embodiment disclosed by the present specification is a treatment method, including administering the composition for gene manipulation to cells including a gene characterized by an over-amplified repeated sequence.

The cells were described above.

The gene characterized by the over-amplified repeated sequence may have a repeated sequence over-amplified in one region of the gene.

The gene may be, for example, any one or more of an HTT gene, FMR1 gene, FMR2 gene, FRDA gene, DMPK/SIX gene, ZNF9 gene, SCA8 gene, ATXN10 gene, PPP2R2B gene, CSTB gene, TCF4 gene, C9orf72 gene, AIB1 gene, KCNN3 gene, CBFA1 gene, COMP gene, AR gene, JPH3 gene, DRPLA gene, ATXN1 gene, ATXN2 gene, ATXN3 gene, CACNA1A gene, ATXN7 gene, TBP gene, PABPN1 gene, HOXD13 gene, RUNX2 gene, HOXA13 gene, ZIC2 gene, FOXL2 gene, PHOX2B gene, SOX3 gene, and ARX gene, but is not limited thereto.

The one region of the gene may be, for example, any one or more of an exon, an intron, a 3'-UTR, a 5'-UTR, and a polyadenylation signal sequence, but is not limited thereto.

The over-amplified repeated sequence includes:
a first repeated part including duplication of the repeated sequence; and
a second repeated part including over-duplication of the repeated sequence,
the first repeated part is included in a diseased subject and a normal subject, and
the second repeated part is not included in a normal subject.

The first repeated part and the second repeated part may be nucleic acid sequences encoding the repetition of a certain amino acid sequence.

A polypeptide having the repetition of the certain amino acid sequence may be translated from the first repeated part and the second repeated part.

It is possible to provide an effect in which the gene which regulates the expression of the over-amplified repeated sequence is artificially manipulated by the composition for gene manipulation.

The gene which regulates the expression of the over-amplified repeated sequence may be one or more selected from the group consisting of an SPT4 gene, SPT5 gene, SUPT4H gene, and SUPT5H gene.

The composition for gene manipulation may provide an effect of reducing the expression of the gene which regulates the expression of the over-amplified repeated sequence.

For example, the composition for gene manipulation may provide an effect of reducing the expression of any one or more of the SPT4 gene, SPT5 gene, SUPT4H gene, and SUPT5H gene.

The reduction in expression of the gene which regulates the expression of the over-amplified repeated sequence may be a reduction in expression of a transcription elongation factor for the over-amplified repeated sequence. For example, the reduction in expression of any one or more of the SPT4 gene, SPT5 gene, SUPT4H gene, and SUPT5H gene may be a reduction in expression of a transcription elongation factor for the over-amplified repeated sequence.

The reduction in expression of the gene which regulates the expression of the over-amplified repeated sequence may reduce the expression of the over-amplified repeated sequence. The reduction in expression of the gene which regulates the expression of the over-amplified repeated sequence may reduce the expression of the second repeated part in the over-amplified repeated sequence.

For example, the reduction in expression of any one or more of the SPT4 gene, SPT5 gene, SUPT4H gene, and SUPT5H gene may reduce the expression of the over-amplified repeated sequence. The reduction in expression of any one or more of the SPT4 gene, SPT5 gene, SUPT4H gene, and SUPT5H gene may reduce the expression of the second repeated part in the over-amplified repeated sequence.

The reduction in expression of the second repeated part in the over-amplified repeated sequence may reduce the transcription of an mRNA from a nucleic acid sequence.

Still another embodiment disclosed by the present specification is a method for treating a disease from a patient, the method including administering the composition for gene manipulation described above to a patient in need thereof.

The disease may be a disease caused by an over-amplified repeated sequence.

Specific examples of the disease caused by the over-amplified repeated sequence include Huntington's Disease (HD), Huntington's Disease-like 2, dentatorubropallidoluysian atrophy (DRPLA), spinal and bulbar muscular atrophy (SBMA), spinocerebellar ataxia (SCA), fragile X syndrome (FXS), fragile X-associated tremor/ataxia syndrome (FXTAS), fragile XE mental retardation, X-linked mental retardation (XLMR) caused by ARX mutations; Fuchs' corneal dystrophy, Friedreich's ataxia (FRDA), myotonic dystrophy, amyotrophic lateral sclerosis (C9orf72 mutation), cleidocranial dysplasia, oculopharyngeal muscular dystrophy, synpolydactyly type 2, hand-foot-genital syndrome (HFGS), holoprosencephaly, blepharophimosis ptosis epicanthus inversus syndrome, congenital central hypoventilation syndrome, mental retardation with GH deficiency; and the like, but are not limited thereto.

The disease to be treated may include the following repeated sequence amplification units.

As a disease caused by an over-amplification of a 3-nucleotide repeated sequence,
the 3-nucleotide repeated sequence may include a repetition of a CAG-, CCG-, CTG-, CGG-, GAA-, GAC-, GCG-, GCA-, GCC- or GCT-nucleotide sequence;
as a disease caused by an over-amplification of a 4-nucleotide repeated sequence,
the 4-nucleotide repeated sequence may include a repetition of a CCTG-nucleotide sequence;
as a disease caused by an over-amplification of a 5-nucleotide repeated sequence,
the 5-nucleotide repeated sequence may include a repetition of a ATTCT- or TGGAA-nucleotide sequence;
as a disease caused by an over-amplification of a 6-nucleotide repeated sequence,
the 6-nucleotide repeated sequence may include a repetition of a GGCCTG- or GGGGCC-nucleotide sequence; and
as a disease caused by an over-amplification of a 12-nucleotide repeated sequence,
the 12-nucleotide repeated sequence may include a repetition of a CCCCGCCCCGCG-nucleotide sequence,
but the nucleotide repeated sequences are not limited thereto.

When an expression regulatory gene of an over-amplified repeated sequence is artificially manipulated and regulated by the composition for gene manipulation disclosed by the present specification, the survival, proliferation and/or persistency, cytotoxicity and the like of animal cells may be improved. Further, through a pharmaceutical composition disclosed by the present specification and a treatment method using the pharmaceutical composition, it is possible to alleviate or treat a genetic disease caused by a specific gene including an over-amplified repeated sequence.

Hereinafter, the present invention will be described in further detail with reference to examples.

The examples are merely provided to describe the present invention in further detail, and it might be obvious to those of ordinary skill in the art that the scope of the present invention is not limited to the following examples.

### Example 1: Design and synthesis of gRNA

### 1-1. Design of sgRNA

CRISPR/Cas9 target regions of human SUPT4H1 gene (SUPT4H1; NCBI Accession No. NM_003168), and mouse SUPT4A gene (SUPT4a: NCBI Accession No. NM_009296) were selected using CRISPR RGEN Tools (Institute for Basic Science, Korea). As CRISPR/Cas9 target region, DNA sequence without 0-, 1-, or 2bp mismatches were selected as sgRNA target region, except for on-target region in the human genome.

### 1-2. Synthesis of sgRNA

Templates for sgRNA synthesis were PCR-amplified by annealing and extending two complementary oligonucleotides.

The target region sequence used, the primers used to amplify the target region sequence, and the target DNA sequence which was targeted by sgRNA produced were summarized in Table 3 to Table 8.

For the template DNA(except 'NGG' of 3' end of the target sequence), *In vitro* transcription using T7 RNA polymerase (New England Biolabs) was carried out, RNA was synthesized according to the manufacturer's instructions, then the template DNA was removed by using DNAse(Ambion). The transcribed RNA was purified by Expin Combo kit (GeneAll) and isopropanol precipitation.

### 1-3 Construction of AAV vector encoding CjCas9 and sgRNA

Human codon-optimized CjCas9 and sgRNA derived from Campylobacter jejuni Cas9 (CjCas9) which incurred a minor modification having HA-tag epitopes at a nuclear localization signal (NLS) and a C-terminal thereof were delivered with an AAV inverted terminal repeat-based vector plasmid.

The sgRNA transcription was induced by a U6 promoter, and the expression of CjCas9 and a red fluorescent protein (RFP) was regulated by an EFS promoter. The AAV vector was used for screening each sgRNA.

### Example 2: Culturing of cell lines

### 2-1. Cell lines and culture media

A human HEK 293 (ATCC, CRL-1573) cell line and a mouse NIH-3T3 fibroblast cell line were cultured in a Dulbecco's Modified Eagle Medium (DMEM) along with high concentration glucose supplemented with a 10% fetal bovine serum (FBS, Welgene), IX penicillin and streptomycin.

### 2-2. Construction of forebrain-derived neural stem cells of E12.5 YAC128 mouse

YAC128 HD transgenic mice which are Huntington's disease model mice were purchased from Jackson Laboratory (Bar Harbor, ME, USA). The YAC128 HD transgenic mice (FVBN/NJ background strain) were reproduced and bred. Forebrain-derived neural stem cells (NSCs) were obtained from embryonic brains of the YAC128 HD transgenic mice and wild-type mice on day 12.5 (embryonic day 12.5; E12.5). A minor modification was performed to construct neural stem cells.

In short, the forebrain tissues of the mice were removed, minced for 1 to 2 minutes until very small pieces remained using a scalpel blade, and then treated with 0.5% trypsin-EDTA at 37°C for 5 minutes. Thereafter, cells were dissociated in a DMEM base medium containing a 10% fetal bovine serum (FBS, Welgene), IX penicillin and streptomycin. Thereafter, the cells were smeared onto a tissue culture petri dish coated with 15 µg/ml of poly-L-ornithine (PLO, Sigma, St Louis, MO) and 1 µg/ml of fibronectin (FN, Sigma). One day later, the culture medium was exchanged with an NSC complete medium including a DMEM/F12 medium containing a 100X N₂ supplement, 300 mM D-glucose, 200 mM L-glutamine, 100 U/mL penicillin, 100 µg/ml streptomycin, 0.1 mM non-essential amino acids, 0.1 mM β-mercaptoethanol, 10 ng/mL epidermal growth factor (EGF), and 10 ng/ml basic fibroblast growth factor (bFGF).

Thereafter, when the growth of the cells was stabilized, magnetic sorting was performed using PSA-NCAM⁺ microbeads, and then purified neural progenitor cells were obtained. The NSCs were maintained at 37°C in a 5% CO₂ humidified incubator and repeatedly subcultured using a IX trypLE Select (Gibco). Prior to the present experiment, cells were subcultured in individually coated wells of a 24-well plate (1 x 10⁴ cells/well) or a 60 mm dish (3 x 10⁵ cells/dish).

### 2-3. Differentiation of neural stem cells of E12.5 YAC128 mouse into nerve cells

For the differentiation of NSCs into neurons, a PLO/Laminin (5 µg/ml, Sigma)-coated dish or plate was prepared. In the culture of 2-2 described above, the culture conditions were changed into conditions in which the epidermal growth factor was removed, and the basic fibroblast growth factor (bFGF) was gradually decreased.

In short, the differentiation proceeded through the following procedure:
A EUROMED-N medium was replaced with a medium of DMEM/F12: Neuron cell culture medium = 1: 1, and the exchanged medium was used as a differentiation medium. The differentiation medium contains a 200X N₂ supplement, 100X B27, 100U/mL penicillin, 100 µg/ml streptomycin, 0.1 mM non-essential amino acids, 0.1 mM β-mercaptoethanol, 3 mM D-glucose, 2 mM L-glutamine. The growth factor NGF was used for neural induction instead of BDNF. In the differentiation step, neural differentiation was performed in the differentiation medium supplemented with NGF (200 ng/mL) and bFGF-2 (10 ng/mL) for the first three days. For the next three days, neural differentiation was performed in the differentiation medium supplemented with NGF (30 ng/mL) and bFGF-2 (6.7 ng/mL). On day 7 after differentiation, cells were used for immunofluorescence and western blot.

### Example 3: Introduction of CRISPR/Cas9

### 3-1. SpCas9-gRNA transfection

To transfect the above-described cells with a CRISPR/SpCas component, a RNP complex was formed by culturing 4 µg of a Cas9 protein (ToolGen) along with 1 g of sgRNA at room temperature for 15 minutes. Thereafter, the RNP complex was electroporated into 2 x 10⁵ cells using a neon electroporator (ThermoFisher) with a 10 uL electroporation tip. For targeted deep sequencing, genomic DNAs (gDNAs) were collected from the cells using a DNeasy Blood & Tissue kit (Qiagen) 48 hours after transfection.

### 3-2. CjCas9-gRNA transfection

To transfect the above-described cells with a CRISPR/CjCas9 component, AAV plasmids delivering each of sgRNA and CjCas9 were electroporated using the neon electroporator (ThermoFisher) with a 10 uL electroporation tip. For targeted deep sequencing, genome DNAs (gDNAs) were collected from the cells using a DNeasy Blood & Tissue kit (Qiagen) 48 hours after transfection.

### Example 4: Screening of gene scissors targeting SPT4 gene

### 4-1. Targeted deep sequencing

The gDNA was exracted from the transfected cells, and an on-target region was PCR-amplified with a specific primer (SEQ ID Nos. 25 to 42) using Phusion polymerase (New England BioLabs) (Table 3).

**[Table 3]**

| ON-target region specific primer sequences of Human SUPT4H1 gene, C57B1/6 MOUSE Supt4a gene, and FVB/NJ MOUSE Supt4a gene | | |
|---|---|---|
| Target site | Primer-F(5' to 3') | Primer-R(5' to 3') |
| Human SUPT4H1 | ACGAGCTATTTACTTCCT GC (SEQ ID 25) | CCACCTCTGAGTTC TGAGAC (SEQ ID 26) |
| | TCTCTTGGTCTTGTTCAT GC (SEQ ID 27) | CAACTAGGATTAAC ACTTGC (SEQ ID 28) |
| | TTCTCTTGGAGTTGCATC AC (SEQ ID 29) | GCTGAACCTCATAG AATGTG (SEQ ID 30) |
| Mouse Supt4a (C57B1/6) | AGCTCCTGTGTGTGCAG ATG (SEQ ID 31) | ACAGATGAGAGCG ACTCCAC (SEQ ID 32) |
| | ATGCATCCTACCCCTCTT CC (SEQ ID 33) | CAGTCCCATGAGAC AGTCAC (SEQ ID 34) |
| Mouse Supt4a (FVB/NJ) | TGGAGGAACCTGTGTTG TCG (SEQ ID 35) | AGCCTTCCTTTCCTG TGTCT (SEQ ID 36) |
| | AGGAGAGGGTGTTGGGT TGT (SEQ ID 37) | AGCTGGTGCAGTCA TAAACC (SEQ ID 38) |
| | TCGAATATGATGGGTGT GAC (SEQ ID 39) | ACTAGCTCTCAGAA CTGCTG (SEQ ID 40) |
| Mouse Rosa26 | AGACTCCCGCCCATCTTC TAGAAA (SEQ ID 41) | AAGTCGCTCTGAGT TGTTATCAGT (SEQ ID 42) |

Thereafter, a PCR amplicon produced as a result of the PCR was subjected to paired-end deep sequencing using Mi-Seq (Illumina). Data from the deep sequencing was analyzed using an online Cas-Analyzer tool (www.rgenome.net). The Indel in the 3bp upstream region from the PAM sequence was considered as a mutation caused by Cas9.

### 4-2. Targeted deep sequencing for Human SUPT4H1 gene

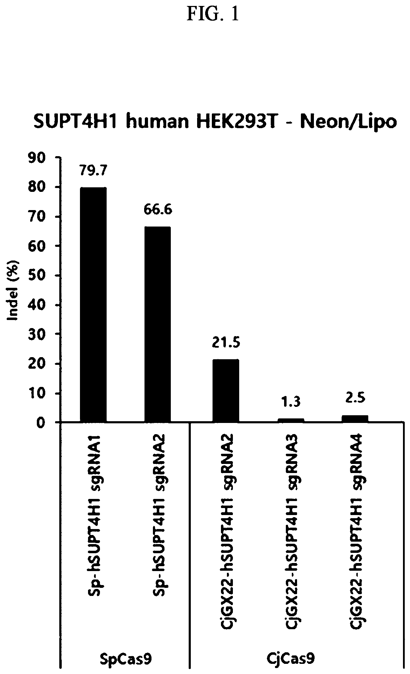

An sgRNA was synthesized by selecting and designing gene scissors for SpCas9 and CjCas9 targeting a target sequence of a human SUPT4H1 gene with sequences in which the off-target was minimized by in silico-base (Tables 4 and 5). Thereafter, indel efficiency (%) caused by the gene scissors in the human HEK293T cell line was screened (FIG. 1).

Each of the following Table 4 and 5 discloses examples of the sgRNA sequence of SpCas9 and the sgRNA sequence of the human SUPT4H1 gene.

**[Table 4]**

| Examples of sgRNA sequence of SpCas9 targeting Human SUPT4H1 gene | | |
|---|---|---|
| | #RGEN Target (5' to 3') | SEQ ID No. |
| Sp-hSUPT4H1 sgRNA1 | CGCAGATGCCGCAGGTCCT TCGG | SEQ ID 1 |
| Sp-hSUPT4H1 sgRNA2 | AATGAAGGGTAACCGAGA GATGG | SEQ ID 2 |

**[Table 5]**

| Examples of sgRNA sequence of CjCas9 targeting Human SUPT4H1 gene | | |
|---|---|---|
| | #RGEN Target (5' to 3') | SEQ ID No. |
| CjGX22-hSUPT4H1 sgRNA1 | AACTGGTCTATAGTCTTG ACCAGCGAACAC | SEQ ID 3 |
| CjGX22-hSUPT4H1 sgRNA2 | CAGGCGACCAGTGACTG ACACCGCATATAC | SEQ ID 4 |
| CjGX22-hSUPT4H1 sgRNA3 | GGCCCGCAGATGCCGCA GGTCCTTCGGCAC | SEQ ID 5 |
| CjGX22-hSUPT4H1 sgRNA4 | GGGTAACCGAGAGATGG TATATGACTGCAC | SEQ ID 6 |
| CjGX22-hSUPT4H1 sgRNA5 | GGCGACCAGTGACTGAC ACCGCATATACAC | SEQ ID 7 |

### 4-3. Targeted deep sequencing for C57B1/6 MOUSE Supt4a gene

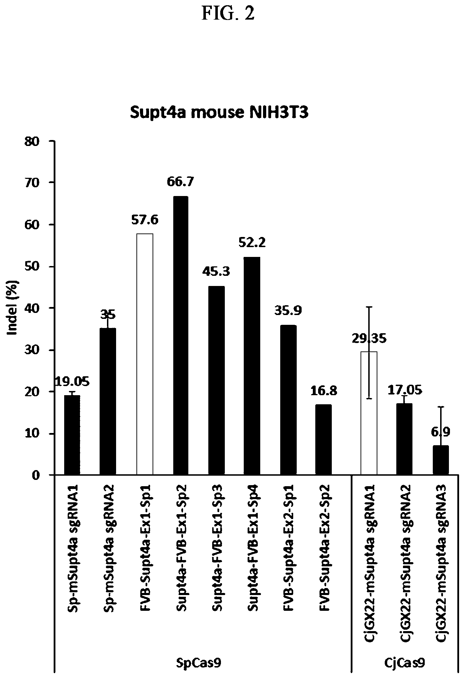

An sgRNA for SpCas9 and CjCas9, targeting a target sequence of a mouse Supt4a gene was synthesized by selecting and designing with sequences in which the off-target was minimized by in silico-base (Tables 6 and 7). Thereafter, indel efficiency (%) caused by the gene scissors in the mouse C57B1/6 cell line was screened (FIG. 2).

Each of the following Tables 6 and 7 discloses examples of the sgRNA sequence of SpCas9 and the sgRNA sequence of CjCas9 for targeting the target sequence of the mouse Supt4a gene.

**[Table 6]**

| Examples of sgRNA sequence of SpCas9 targeting C57B1/6 MOUSE Supt4a gene | | |
|---|---|---|
| | #RGEN Target (5' to 3') | SEQ ID No. |
| Sp-mSupt4a sgRNA1 | CTAGCTCTCAAGCTGAGCA GGGG | SEQ ID 8 |
| Sp-mSupt4a sgRNA2 | GAGCAGGGACACAAGTG TCGGG | SEQ ID 9 |
| Sp-mSupt4a sgRNA3 | CGAGTCAGTAACTTTAAGC CAGG | SEQ ID 10 |

**[Table 7]**

| Examples of sgRNA sequence of CjCas9 targeting C57B1/6 MOUSE Supt4a gene | | |
|---|---|---|
| | #RGEN Target (5' to 3') | SEQ ID No. |
| CjGX22-mSupt4a sgRNA1 | GAGAGCTAGGACTGAAGGC TGCATCAACAC | SEQ ID 11 |
| Cj GX22-mSupt4a sgRNA2 | CTCATGATGCAGCAGAAGG CTACCCGACAC | SEQ ID 12 |
| CjGX22-mSupt4a sgRNA3 | GTCCTAGCTCTCAAGCTGA GCAGGGGACAC | SEQ ID 13 |

### 4-4. Targeted deep sequencing for FVB/NJ MOUSE Supt4a gene

An sgRNA for SpCas9 and CjCas9, targeting a target sequence of a mouse Supt4a gene was synthesized by selecting and designing with sequences in which the off-target was minimized by in silico-base (Tables 8 and 9). Thereafter, indel efficiency (%) caused by the gene scissors in the mouse FVB/NJ cell line was screened (Table 3).

Each of the following Tables 8 and 9 discloses examples of the sgRNA sequence of SpCas9 and the sgRNA sequence of CjCas9 for targeting the target sequence of the mouse Supt4a gene.

**[Table 8]**

| Examples of sgRNA sequence of SpCas9 targeting FVB/NJ MOUSE Supt4a gene | | |
|---|---|---|
| | #RGEN Target (5' to 3') | SEQ ID No. |
| Supt4a-FVB-Ex1-Sp1 | TTTGCTGTGCTCGTTAGTCA AGG | SEQ ID 14 |
| Supt4a-FVB-Ex1-Sp2 | TCGTTAGTCAAGGTGTCCG TTGG | SEQ ID 15 |
| Supt4a-FVB-Ex1-Sp3 | CGTTAGTCAAGGTGTCCGT TGGG | SEQ ID 16 |
| Supt4a-FVB-Ex1-Sp4 | GTTAGTCAAGGTGTCCGTT GGGG | SEQ ID 17 |
| Supt4a-FVB-Ex2-Sp 1 | ATTCGAACTGGTCTATAGT CTGG | SEQ ID 18 |
| Supt4a-FVB-Ex2-Sp2 | GCACCAGCTCTTCATTTGA TGGG | SEQ ID 19 |
| Supt4a-FVB-Ex2-Sp3 | TTACCCATCAAATGAAGAG CTGG | SEQ ID 20 |

**[Table 9]**

| Examples of sgRNA sequence of CjCas9 targeting FVB/NJ MOUSE Supt4a gene | | |
|---|---|---|
| | #RGEN Target (5' to 3') | SEQ ID No. |
| FVB-Supt4a-Ex1-Cj 1-F | TACCGTCTCCAGGGCCAT CCTCACCGATAC | SEQ ID 21 |
| FVB-Supt4a-Ex1-Cj2-F | GCCCCAACGGACACCTTG ACTAACGAGCAC | SEQ ID 22 |
| FVB-Supt4a-Ex1-Cj3-F | GTACCGTCTCCAGGGCCA TCCTCACCGATA | SEQ ID 23 |
| SUPT4a-FVB-Ex3-Cj1F | CTCCCCAAAGCAACAGCC CGAGCAAGGCAC | SEQ ID 24 |

### Example 5: Cytotoxicity assay against FVB/NJ MOUSE (YAC128) cell

It was confirmed whether an alteration in reading frame of mRosa26 had a selective effect on survival during three passages using the SpCas9 gene scissors targeting the mRosa26 gene (control) in primary neural stem cells of a Huntington's disease mouse model (YAC128) in a mouse FVB/NJ.

It was confirmed whether an alteration in reading frame of Supt4a had a selective effect on survival during three passages using the SpCas9 gene scissors targeting the Supt4a gene in primary neural stem cells of a Huntington's disease mouse model (YAC128) in a mouse FVB/NJ.

After the SpCas9 gene scissors targeting the mRosa26 gene (control) or the SpCas9 gene scissors targeting the Supt4a gene were applied, cells were subcultured three times. Thereafter, targeted deep sequencing was performed by collecting genomic DNA from each cell subcultured three times. In sequencing readings, the numbers of in-frame indels and out-frame indels were determined, and the percentage was calculated. During the three passages, the case where the ratio of the out-frame indel and the in-frame indel was significantly changed was considered cytotoxicity caused by gene knockout.

### Example 6: Immunostaining for FVB/NJ MOUSE (YAC128) cell

### 6-1. Western blot

NSCs and differentiated neurons were lysed in ice-cold RIPA buffer. Thereafter, the lysate was centrifuged (20 minutes, 14,000 g, 4°C), and the supernatant thereof was transferred to a new tube. Proteins were analyzed using the BCA Assay Kit (Thermo Fisher Scientific, Waltham, MA). Equal amounts of proteins (20 to 50 µg) were loaded on an 8 to 12% SDS polyacrylamide gel. The isolated proteins were transferred to a polyvinylidene difluoride membrane (Millipore, Bedford, MA, USA) by electrophoresis. The membrane was washed with a Tris-buffered saline solution containing 2.5 mM EDTA (TNE), and blocked in TNE containing 5% skim milk for 1 hour. The membrane was cultured along with primary antibodies; 1C₂ (1 : 500, Millipore), EM48 (1 : 500, Millipore) and Spt4 (1 : 1000, Biorbyt) at 4°C overnight. Subsequently, the membrane was cultured along with secondary antibodies (1 : 1000, GeneTex, Irvine, CA, USA) at room temperature for 1 hour, and washed again. Blotting was performed using an ECL western blotting detection reagent (Millipore, Bedford, MA, USA). For quantitative analysis, the density of an immunoblot band was measured using Image J software.

### 6-2. Immunofluorescence

NSCs and differentiated neurons were prepared with a 12 mm circular cover slip coated with PLO/Laminin (5 µg/ml, Sigma) in a 4-well plate. Thereafter, the cells were washed with iced PBS, and fixed in 4% paraformaldehyde at room temperature for 20 minutes. Thereafter, the cells were washed twice with PBS, and cultured under room temperature conditions in IX PBS containing 1% bovine serum albumin (BSA) containing 0.2% Triton X-100 for 30 minutes. Thereafter, the cells were washed three times with IX PBS containing 0.5% bovine serum albumin (BSA). After washing, the cells were cultured along with primary antibodies ; 1C₂ (1 : 100, Millipore), EM48 (1 : 100, Millipore), and Spt4 (1 : 100, Biorbyt) at 4°C overnight. Next, the cells were washed with IX PBS containing 0.5% bovine serum albumin (BSA), and cultured along with appropriate secondary antibodies including goat anti-mouse IgG (Alexa Fluor-555, Molecular Probes, Wyman, MA, USA) and goat anti-rabbit IgG (Alexa Fluor-488, Molecular Probes, Wyman, MA, USA). The stained cells were observed using Carl Zeiss Microscopy LSM 880 confocal laser scanning microscope (Carl Zeiss Microscopy, Jena, Germany).

### [Sequence Listing Free Text]

Described is a target sequence of an expression regulatory gene of an over-amplified repeated sequence.

Described are on-target site specific primer sequences of the human SUPT4H1 gene, C57B1/6 mouse Supt4a gene and FVB/NJ mouse Supt4a gene.

## Claims

1. A system for regulating an expression of repeat expansion, including an artificially engineered gene to reduce an expression of repeat expansion,
wherein the artificially engineered gene includes one or more genes selected from the group consisting of SPT4, SPT5, SUPT4H, and SUPT5H,
wherein the artificially engineered gene includes an artificial mutation in its genome,
wherein the artificially engineered gene includes the gene which is dysfunctional or is reduced on expression,
wherein the reducing an expression of the gene is **characterized in that** an amount of expression product thereof is reduced or suppressed compared to that of a gene which is not artificially engineered,

2. The system for regulating an expression of repeat expansion mutaion of claim 1,
wherein the SUPT4H includes SUPT4H1 gene.

3. The system for regulating an expression of repeat expansion mutaion of claim 1,
wherein the artificial mutation of the gene includes at least one of a deletion, substitution, or insertion of one or more nucleotides.

4. The system for regulating an expression of repeat expansion mutaion of claim 1,
wherein the repeat expansion comprises:
a first repeated part including duplication of the repeated sequence; and
a second repeated part including over-duplication of the repeated sequence;
wherein the first repeated part is included to disordered subject and normal subj ect,
wherein the second repeated part is not included to normal subject,
the regulation of an expression of repeat expansion includes reduced expression of the second repeated part.

5. The system for regulating an expression of repeat expansion mutaion of claim 1,
wherein the expression of repeat expansion is at least one selected from the group consisting of transcription, post-transcriptional processing, translation, and post-translational modification.

6. The system for regulating an expression of repeat expansion mutaion of claim 1,
wherein the repeat expansion comprising:
a first repeated part including duplication of the repeated sequence; and
a second repeated part including over-duplication of the repeated sequence;
wherein the first and second repeated part include one or more repeat expansions selected from the followings:
3-nucleotide repeat expansion;
4-nucleotide repeat expansion;
5-nucleotide repeat expansion;
6-nucleotide repeat expansion; and
12-nucleotide repeat expansion.

7. The system for regulating an expression of repeat expansion mutaion of claim 6,
wherein the 3-nucleotide repeat expansion is CAG-, CCG-, CTG-, CGG-, GAA-, GAC-, GCG-, GCA-, GCC- and GCT- repeat expansion,
wherein the 4-nucleotide repeat expansion is CCTG- repeat expansion; wherein the 5-nucleotide repeat expansion is one or more repeat expansions selected from ATTCT- and TGGAA- repeat expansion;
wherein the 6-nucleotide repeat expansion is one or more repeat expansions selected from GGCCTG- and GGGGCC- repeat expansion;
wherein the 12-nucleotide repeat expansion is CCCCGCCCCGCG- repeat expansion.

8. The system for regulating an expression of repeat expansion mutaion of claim 1,
wherein the expression of repeat expansion is the cause of one or more disorder selected from the followings:
Huntington's Disease(HD);
Hungtington's Disease-like 2;
Dentatorubropallidoluysian atrophy(DRPLA);
Spinal and bulbar muscular atrophy(SBMA);
Spinocerebellar ataxia(SCA);
Fragile X syndrome(FXS);
Fragile X-associated tremor/ataxia syndrome(FXTAS);
Fragile XE mental retardation;
XLMR;
Fuchs corneal dystrophy;
Friedreich's ataxia(FRDA);
Myotonic dystrophy;
Amyotrophic lateral sclerosis(C9orf72mutation);
Cleidocranial dysplasia;
Oculopharyngeal muscular dystrophy;
Synpolydactyly type 2;
hand-foot-genital syndrome;
holoprosencephaly;
Blepharophimosis ptosis epicanthus inversus syndrome;
Congenital central hypoventilation syndrome; and
Mental retardation with GH deficiency.

9. A composition for gene manipulation to reduce an expression of repeat expansion, the composition includes Clustered regularly interspaced short palindromic repeats(CRISPR)- CRISPR associated protein(Cas) system, Zinc finger nuclease(ZFN), Transcription activator-like effector nuclease(TALEN), FokI, or endonuclease,
wherein the gene is one or more genes selected from the group consisting of SPT4, SPT5, and SUPT5H.

10. The composition for gene manipulation of claim 9,
wherein the gene manipulation includes one or more modifications of nucleotides which is deletion, substitution, or insertion.

11. The composition for gene manipulation of claim 9,
wherein the composition for gene manipulation is a composition for treating any one or more disorders caused by the followings:
3-nucleotide repeat expansion;
4-nucleotide repeat expansion;
5-nucleotide repeat expansion;
6-nucleotide repeat expansion; and
12-nucleotide repeat expansion;

12. The composition for gene manipulation of claim 11,
wherein the 3-nucleotide repeat expansion is CAG-, CCG-, CTG-, CGG-, GAA-, GAC-, GCG-, GCA-, GCC- and GCT- repeat expansion;
wherein the 4-nucleotide repeat expansion is CCTG- repeat expansion; wherein the 5-nucleotide repeat expansion is one or more repeat expansions selected from ATTCT- and TGGAA- repeat expansion;
wherein the 6-nucleotide repeat expansion is one or more repeat expansions selected from GGCCTG- and GGGGCC- repeat expansion;
wherein the 12-nucleotide repeat expansion is CCCCGCCCCGCG- repeat expansion;

13. The composition for gene manipulation of claim 9,
the composition for gene manipulation is a composition for treating one or more disorders selected from the followings:
Huntington's Disease(HD);
Hungtington's Disease-like 2;
Dentatorubropallidoluysian atrophy(DRPLA);
Spinal and bulbar muscular atrophy(SBMA);
Spinocerebellar ataxia(SCA);
Fragile X syndrome(FXS);
Fragile X-associated tremor/ataxia syndrome(FXTAS);
Fragile XE mental retardation;
XLMR;
Fuchs corneal dystrophy;
Friedreich's ataxia(FRDA);
Myotonic dystrophy;
Amyotrophic lateral sclerosis(C9orf72mutation);
Cleidocranial dysplasia;
Oculopharyngeal muscular dystrophy;
Synpolydactyly type 2;
hand-foot-genital syndrome;
holoprosencephaly;
Blepharophimosis ptosis epicanthus inversus syndrome;
Congenital central hypoventilation syndrome; and
Mental retardation with GH deficiency.

14. A composition for gene manipulation which includes guide nucleic acids for target sequence of one or more genes selected from the group consisting of SPT4, SPT5, SUPT4H, and SUPT5H.

15. The composition for gene manipulation of claim 14,
wherein the composition for gene manipulation includes the editor protein or nucleic acid encoding the smae,
wherein the editor protein comprises one or more proteins selected form the group consisting of a Streptococcus pyogenes-derived Cas9 protein, a Campylobacter jejuni-derived Cas9 protein, a Streptococcus thermophilus-derived Cas9 protein, a Streptocuccus aureus-derived Cas9 protein, a Neisseria meningitidis-derived Cas9 protein, and a Cpfl protein.

16. The composition for gene manipulation of claim 14,
wherein the target sequence exists in one ore more regions of the gene selected from the followings:
a promoter region;
an intron region;
an exon region;
an enhancer region;
3'-UTR(Untranslated region); and
5'-UTR.

17. The composition for gene manipulation of claim 14,
wherein the target sequence exists in an exon region of the gene.

18. The composition for gene manipulation of claim 14,
wherein the target sequence is a nucleotide sequence which exists in a continuous 10 bp to 25 bp nucleotide sequence region adjacent to the 5' end and/or 3' end of a proto-spacer-adjacent motif(PAM) sequence in a nucleic acid sequence constituting the gene.

19. The composition for gene manipulation of claim 14,
wherein the PAM sequence comprises at least one selected from the group consisting of the following sequences (described in the 5' to 3' direction):
NGG (wherein N is A, T, C or G);
NNNNRYAC (wherein N is each independently A, T, C or G, R is A or G, and Y is C or T);
NNAGAAW (wherein N is each independently A, T, C or G and W is A or T);
NNNNGATT (wherein N is each independently A, T, C or G);
NNGRR(T) (wherein N is each independently A, T, C or G, R is A or G, and Y is C or T); and
TTN (wherein N is A, T, C or G).

20. The composition for gene manipulation of claim 14,
wherein the target sequence is one or more sequences selected from the group consisting SEQ ID NOs: 1 to 24.

21. The composition for gene manipulation of claim 14,
wherein the target sequence is one or more sequences selected from the group consisting SEQ ID NOs: 1, 2, 14, 15, 16, and 17.

22. The composition for gene manipulation of claim 15,
wherein the composition comprises:
guide nucleic acids for one or more sequences selected from SEQ ID Nos: 1, 2, 8 to 10 and 14 to 20; and
a Streptococcus pyogenes-derived Cas9 protein or a nucleic acid encoding the same.

23. The composition for gene manipulation of claim 15,
wherein the composition comprises:
guide nucleic acids for one or more sequences selected from SEQ ID Nos: 3 to 7, 11 to 13 and 21 to 24; and
a Campylobacter jejuni-derived Cas9 protein or a nucleic acid encoding the same.

24. The composition for gene manipulation of claim 14,
wherein the guide nucleic acids includes a guide domain capable of forming complementary bonds with respect to a target sequence of the gene,
wherein the complementary bonds include 0 to 5 mismatching bonds.

25. The composition for gene manipulation of claim 24,
wherein the guide domain includes a complementary nucleotide sequence with respect to the target sequence of the gene,
wherein the complementary nucleotide sequence includes 0 to 5 mismatching.

26. The composition for gene manipulation of claim 14,
the guide nucleic acids comprises at least one domain selected from the group consisting of:
a first complementary domain;
a linker domain;
a second complementary domain;
a proximal domain; and
a tail domain.

27. The composition for gene manipulation of claim 14 or claim 15,
wherein the guide nucleic acids and the editor protein are in the form of a DNA encoding thereof, respectively.

28. The composition for gene manipulation of claim 27, the DNA is included in the form of plasmid or viral vector.

29. The composition for gene manipulation of claim 28,
wherein the viral vector is at least one selected from the group consisting of a retrovirus, a lentivirus, an adenovirus, an adeno-associated virus (AAV), a vaccinia virus, a poxvirus, and a herpes simplex virus.

30. The composition for gene manipulation of claim 15,
wherein the nucleic acid encoding the editor protein is provided in a form of an mRNA.

31. The composition for gene manipulation of claim 15,
wherein the editor protein is in a form of polypeptides or protein.

32. The composition for gene manipulation of claim 15,
wherein the composition is in a form of a guide nucleic acid-editor protein complex.

33. The composition for gene manipulation of claim 14 or claim 15,
wherein the composition is a composition for treating any one or more disdorders caused by:
3-nucleotide repeat expansion;
4-nucleotide repeat expansion;
5-nucleotide repeat expansion;
6-nucleotide repeat expansion; and
12-nucleotide repeat expansion;

34. The composition for gene manipulation of claim 33,
wherein the 3-nucleotide repeat expansion is CAG-, CCG-, CTG-, CGG-, GAA-, GAC-, GCG-, GCA-, GCC- and GCT- repeat expansion,
wherein the 4-nucleotide repeat expansion is CCTG- repeat expansion;
wherein the 5-nucleotide repeat expansion is one or more repeat expansions selected from ATTCT- and TGGAA- repeat expansion;
wherein the 6-nucleotide repeat expansion is one or more repeat expansions selected from GGCCTG- and GGGGCC- repeat expansion;
wherein the 12-nucleotide repeat expansion is CCCCGCCCCGCG- repeat expansion;

35. The composition for gene manipulation of claim 14 or claim 15,
wherein the composition is a composition for treating one or more of the following disease:
Huntington's Disease(HD);
Hungtington's Disease-like 2;
Dentatorubropallidoluysian atrophy(DRPLA);
Spinal and bulbar muscular atrophy(SBMA);
Spinocerebellar ataxia(SCA);
Fragile X syndrome(FXS);
Fragile X-associated tremor/ataxia syndrome(FXTAS);
Fragile XE mental retardation;
XLMR;
Fuchs corneal dystrophy;
Friedreich's ataxia(FRDA);
Myotonic dystrophy;
Amyotrophic lateral sclerosis(C9orf72mutation);
Cleidocranial dysplasia;
Oculopharyngeal muscular dystrophy;
Synpolydactyly type 2;
hand-foot-genital syndrome;
holoprosencephaly;
Blepharophimosis ptosis epicanthus inversus syndrome;
Congenital central hypoventilation syndrome; and
Mental retardation with GH deficiency.

36. A method for treating repeat expansion disorder comprising an administrating a composition to a subject,
wherein the composition comprises a gene manipulation composition as an active ingredient,
wherein the gene manipulation composition is to reduce an expression of repeat expansion,
wherein the gene manipulation composition comprises Clustered regularly interspaced short palindromic repeats(CRISPR)- CRISPR associated protein(Cas) system, Zinc finger nuclease(ZFN), Transcription activator-like effector nuclease(TALEN), FokI, or endonuclease,
wherein the gene is at least one gene selected from the group consisting of SPT4, SPT5, SUPT4H, and SUPT5H.

37. A method for treating repeat expansion disorder comprising an administrating a composition to a subject,
wherein the composition comprises a composition for gene manipulation as an active ingredient,
wherein the composition for gene manipulation comprises:
a guide nucleic acids for target sequences of at least one gene selected from the group consisting of SPT4, SPT5, SUPT4H, and SUPT5H; and
an editor protein or nucleic acid encoding the smae,
wherein the editor protein comprises at least one protein selected form the group consisting of a Streptococcus pyogenes-derived Cas9 protein, a Campylobacter jejuni-derived Cas9 protein, a Streptococcus thermophilus-derived Cas9 protein, a Streptocuccus aureus-derived Cas9 protein, a Neisseria meningitidis-derived Cas9 protein, and a Cpfl protein.

38. A method for treating repeat expansion disorder of claim 37,
wherein the target sequences are at least one target sequence selected from the group consisting of SEQ ID NOs: 1 to 24.

39. A method for treating repeat expansion disorder of claim 37,
the composition for gene manipulation comprises:
a guide nucleic acids for at leaset one sequence selected from SEQ ID NOs: 1, 2, 8 to 10, and 14 to 20; and
a Streptococcus pyogenes-derived Cas9 protein.

40. A method for treating repeat expansion disorder of claim 37
the composition for gene manipulation comprises:
a guide nucleic acids for at least one sequence selected from SEQ ID NOs: 3, 7, 11 to 13, and 21 to 24; and
a Campylobacter jejuni-derived Cas9 protein.

41. A method for treating repeat expansion disorder of claim 36 or claim 37,
wherein the composition is administered by one or more of ribonucleoprotein(RNP), liposomes, plasmids, viral vectors, nanoparticles, and Protein translocation domain(PTD) method.

42. A method for treating repeat expansion disorder of claim 41,
wherein the viral vector is at least one selected from the group consisting of a retrovirus, a lentivirus, an adenovirus, an adeno-associated virus (AAV), a vaccinia virus, a poxvirus, and a herpes simplex virus.

43. A method for treating repeat expansion disorder of claim 37,
wherein the guide nucleic acids are delivered to cells in a form of DNA or RNA; and
wherein the editor protein is delivered to cells in a form of polypeptides or the nucleic acid encoding the editor protein is delivered to cells in a form of DNA.

44. A method for treating repeat expansion disorder of claim 36 or claim 37,
wherein the administrating method to subject is one or more methods selected from electroporation, injection, transfusion, implantation, and transplantation.

45. A method for treating repeat expansion disorder of claim 36 or claim 37,
wherein the administration is conducted locally or topically.

46. A method for treating repeat expansion disorder of claim 36 or claim 37,
wherein the administering is conducted by subcutaneous, intradermal, intranodal, intramedullary, intramuscular, intravenous, intralymphatical, or intraperitoneal method.

47. A method for treating repeat expansion disorder of claim 46,
wherein the local or topical administration to the subject is conducted to at least one of the following organs of the subject:
kidney;
digestive system including stomach, pancreas, duodenum, ileum and/or colon;
heart;
lung;
brain, in particular, neurons, and/or CNS in general;
eyes including retinal tissue;
ears including the inner ear;
skin;
muscle;
bone; and
liver.

48. A method for treating repeat expansion disorder of claim 36 or claim 37,
wherein the subject is mammals including humans, monkeys, mice, and rats.

49. A method for treating repeat expansion disorder of claim 36 or claim 37,
wherein the repeat expansion comprises:
a first repeated part including duplication of the repeated sequence; and
a second repeated part including over-duplication of the repeated sequence;
wherein the first repeated part is included to disordered subject and normal subj ect,
wherein the second repeated part is not included to normal subject,
wherein the treatment of repeat expansion comprises reduced expression of the second repeated part.

50. A method for treating repeat expansion disorder of claim 36 or claim 37,
wherein the repeat expansion disorder is one or more disease caused by:
3-nucleotide repeat expansion;
4-nucleotide repeat expansion;
5-nucleotide repeat expansion;
6-nucleotide repeat expansion; and
12-nucleotide repeat expansion;

51. A method for treating repeat expansion disorder of claim 50,
wherein the 3-nucleotide repeat expansion is CAG-, CCG-, CTG-, CGG-, GAA-, GAC-, GCG-, GCA-, GCC- and GCT- repeat expansion,
wherein the 4-nucleotide repeat expansion is CCTG- repeat expansion;
wherein the 5-nucleotide repeat expansion is one or more repeat expansions selected from ATTCT- and TGGAA- repeat expansion;
wherein the 6-nucleotide repeat expansion is one or more repeat expansions selected from GGCCTG- and GGGGCC- repeat expansion;
wherein the 12-nucleotide repeat expansion is CCCCGCCCCGCG- repeat expansion;

52. A method for treating repeat expansion disorder of claim 36 or claim 37,
wherein the repeat expansion disorder is at least one of the followings:
Huntington's Disease(HD);
Hungtington's Disease-like 2;
Dentatorubropallidoluysian atrophy(DRPLA);
Spinal and bulbar muscular atrophy(SBMA);
Spinocerebellar ataxia(SCA);
Fragile X syndrome(FXS);
Fragile X-associated tremor/ataxia syndrome(FXTAS);
Fragile XE mental retardation;
XLMR;
Fuchs corneal dystrophy;
Friedreich's ataxia(FRDA);
Myotonic dystrophy;
Amyotrophic lateral sclerosis(C9orf72mutation);
Cleidocranial dysplasia;
Oculopharyngeal muscular dystrophy;
Synpolydactyly type 2;
hand-foot-genital syndrome;
holoprosencephaly;
Blepharophimosis ptosis epicanthus inversus syndrome;
Congenital central hypoventilation syndrome; and
Mental retardation with GH deficiency.


<110>    COLLEGE OF MEDICINE POCHON CHA UNIVERSITY INDUSTRY-ACADEMIC
COOPERATION FOUNDATION
         TOOLGEN

<120>    A SYSTEM FOR GENOME EDITING TOWARD REPEAT EXPANSION MUTATION

<130>    OPP17-034-NP-PCT

<150>    US 62/520,098
<151>    2017-06-15

<160>    42

<170>    KoPatentIn 3.0

<210>    1
<211>    23
<212>    DNA
<213>    Homo sapiens

<400>    1
cgcagatgcc gcaggtcctt cgg                                               23


<210>    2
<211>    23
<212>    DNA
<213>    Homo sapiens

<400>    2
aatgaagggt aaccgagaga tgg                                               23


<210>    3
<211>    30
<212>    DNA
<213>    Homo sapiens

<400>    3
aactggtcta tagtcttgac cagcgaacac                                        30


<210>    4
<211>    30
<212>    DNA
<213>    Homo sapiens

<400>    4
caggcgacca gtgactgaca ccgcatatac                                        30


<210>    5
<211>    30
<212>    DNA
<213>    Homo sapiens

<400>    5
ggcccgcaga tgccgcaggt ccttcggcac                                        30


<210>    6
<211>    30
<212>    DNA

<213>    Homo sapiens

<400>    6
gggtaaccga gagatggtat atgactgcac                                        30


<210>    7
<211>    30
<212>    DNA
<213>    Homo sapiens

<400>    7
ggcgaccagt gactgacacc gcatatacac                                        30


<210>    8
<211>    23
<212>    DNA
<213>    Mus musculus

<400>    8
ctagctctca agctgagcag ggg                                               23


<210>    9
<211>    23
<212>    DNA
<213>    Mus musculus

<400>    9
gagcagggga cacaagtgtc ggg                                               23


<210>    10
<211>    23
<212>    DNA
<213>    Mus musculus

<400>    10
cgagtcagta actttaagcc agg                                               23


<210>    11
<211>    30
<212>    DNA
<213>    Mus musculus

<400>    11
gagagctagg actgaaggct gcatcaacac                                        30


<210>    12
<211>    30
<212>    DNA
<213>    Mus musculus

<400>    12
ctcatgatgc agcagaaggc tacccgacac                                        30


<210>    13
<211>    30
<212>    DNA

<213>    Mus musculus

<400>    13
gtcctagctc tcaagctgag caggggacac                                        30


<210>    14
<211>    23
<212>    DNA
<213>    Mus musculus

<400>    14
tttgctgtgc tcgttagtca agg                                               23


<210>    15
<211>    23
<212>    DNA
<213>    Mus musculus

<400>    15
tcgttagtca aggtgtccgt tgg                                               23


<210>    16
<211>    23
<212>    DNA
<213>    Mus musculus

<400>    16
cgttagtcaa ggtgtccgtt ggg                                               23


<210>    17
<211>    23
<212>    DNA
<213>    Mus musculus

<400>    17
gttagtcaag gtgtccgttg ggg                                               23


<210>    18
<211>    23
<212>    DNA
<213>    Mus musculus

<400>    18
attcgaactg gtctatagtc tgg                                               23


<210>    19
<211>    23
<212>    DNA
<213>    Mus musculus

<400>    19
gcaccagctc ttcatttgat ggg                                               23


<210>    20
<211>    23
<212>    DNA

<213>    Mus musculus

<400>    20
ttacccatca aatgaagagc tgg                                              23


<210>    21
<211>    30
<212>    DNA
<213>    Mus musculus

<400>    21
taccgtctcc agggccatcc tcaccgatac                                       30


<210>    22
<211>    30
<212>    DNA
<213>    Mus musculus

<400>    22
gccccaacgg acaccttgac taacgagcac                                       30


<210>    23
<211>    30
<212>    DNA
<213>    Mus musculus

<400>    23
gtaccgtctc cagggccatc ctcaccgata                                       30


<210>    24
<211>    30
<212>    DNA
<213>    Mus musculus

<400>    24
ctccccaaag caacagcccg agcaaggcac                                       30


<210>    25
<211>    20
<212>    DNA
<213>    Artificial Sequence

<220>
<223>    primer


<400>    25
acgagctatt tacttcctgc                                                  20


<210>    26
<211>    20
<212>    DNA
<213>    Artificial Sequence

<220>
<223>    primer

<400>    26
ccacctctga gttctgagac                                                    20


<210>    27
<211>    20
<212>    DNA
<213>    Artificial Sequence

<220>
<223>    primer


<400>    27
tctcttggtc ttgttcatgc                                                    20


<210>    28
<211>    20
<212>    DNA
<213>    Artificial Sequence

<220>
<223>    primer


<400>    28
caactaggat taacacttgc                                                    20


<210>    29
<211>    20
<212>    DNA
<213>    Artificial Sequence

<220>
<223>    primer


<400>    29
ttctcttgga gttgcatcac                                                    20


<210>    30
<211>    20
<212>    DNA
<213>    Artificial Sequence

<220>
<223>    primer


<400>    30
gctgaacctc atagaatgtg                                                    20


<210>    31
<211>    20
<212>    DNA
<213>    Artificial Sequence

<220>

<223>    primer

<400>    31
agctcctgtg tgtgcagatg                                                    20

<210>    32
<211>    20
<212>    DNA
<213>    Artificial Sequence

<220>
<223>    primer

<400>    32
acagatgaga gcgactccac                                                    20

<210>    33
<211>    20
<212>    DNA
<213>    Artificial Sequence

<220>
<223>    primer

<400>    33
atgcatccta cccctcttcc                                                    20

<210>    34
<211>    20
<212>    DNA
<213>    Artificial Sequence

<220>
<223>    primer

<400>    34
cagtcccatg agacagtcac                                                    20

<210>    35
<211>    20
<212>    DNA
<213>    Artificial Sequence

<220>
<223>    primer

<400>    35
tggaggaacc tgtgttgtcg                                                    20

<210>    36
<211>    20
<212>    DNA
<213>    Artificial Sequence

<220>
<223>    primer


<400>    36
agccttcctt tcctgtgtct                                                    20


<210>    37
<211>    20
<212>    DNA
<213>    Artificial Sequence

<220>
<223>    primer


<400>    37
aggagagggt gttgggttgt                                                    20


<210>    38
<211>    20
<212>    DNA
<213>    Artificial Sequence

<220>
<223>    primer


<400>    38
agctggtgca gtcataaacc                                                    20


<210>    39
<211>    20
<212>    DNA
<213>    Artificial Sequence

<220>
<223>    primer


<400>    39
tcgaatatga tgggtgtgac                                                    20


<210>    40
<211>    20
<212>    DNA
<213>    Artificial Sequence

<220>
<223>    primer


<400>    40
actagctctc agaactgctg                                                    20


<210>    41
<211>    24

<212>    DNA
<213>    Artificial Sequence

<220>
<223>    primer


<400>    41
agactcccgc ccatcttcta gaaa                                              24


<210>    42
<211>    24
<212>    DNA
<213>    Artificial Sequence

<220>
<223>    primer


<400>    42
aagtcgctct gagttgttat cagt                                              24